# EUROPEAN PATENT SPECIFICATION

(11) **EP 0 074 708 B1**
(45) Date of publication and mention of the grant of the patent: **19.02.1986**
(21) Application number: 82303942.5
(22) Date of filing: 26.07.1982
(51) Int. Cl.: C09J 3/14, C08F 220/00, C08F 230/00, A61L 15/06, A61K 6/00, A61L 15/07

(54) **Adhesive composition**
Klebstoffzusammensetzung
Composition adhésive

(30) Priority: 29.07.1981 JP 119536/81; 29.07.1981 JP 119537/81; 30.07.1981 JP 120371/81; 28.01.1982 JP 13038/82; 04.05.1982 JP 74830/82
(43) Date of publication of application: 23.03.1983
(73) Proprietor: KURARAY CO., LTD., Kurashiki-City Okayama Prefecture 710 (JP)
(72) Inventor: Omura, Ikuo, Kurashiki-city (JP); Yamauchi, Junichi, Kurashiki-city (JP); Nagase, Yoshinori, Kurashiki-city (JP); Uemura, Fumiko, Kurashiki-city (JP)
(74) Representative: Crampton, Keith John Allen

## Description

This invention relates to adhesive compositions for adhering strongly to hard tissues in a living body, such as teeth and bones, as well as metals, organic polymers and ceramics. The term "adhesive composition" as herein used means not only a composition used for bonding two or more adherends to one another, but also means a composition used for forming a highly adhesive coating on the surface of an adherend such as a metal or organic polymer, and a composition used for forming a highly adhesive filling material for repairing hard tissues in a living body. In other words, this invention covers any and all compositions that are adhesively applicable to various kinds of substances, including hard tissues in a living body, metals, organic polymers and ceramics.

Various kinds of metals, organic polymers and ceramics are used for the restoration of teeth. When these restorative materials are mounted in the mouth, it is necessary to ensure that they are securely bonded to teeth, or to each other. Since they are placed in the mouth, it is particularly necessary that satisfactory adhesion be obtained in a wet condition. A variety of attempts have hitherto been made to explore a practically useful adhesive for dentistry as will hereinafter be set forth. Although some adhesives have been put to practical use, they are used only for a limited scope of application, since they do not work satisfactorily. An adhesive that can strongly bond a metal or organic polymer, in a wet condition, exhibiting an adhesive strength not easily affected by water, would contribute greatly to improving the art of dental restoration. An adhesive that provides a high adhesive strength in a wet condition will also be useful for many other applications.

U.S. Patents Nos. US―A―4,259,075 and US―A―4,259,117 show that a polymerizable composition containing a vinyl compound having a group of the formula 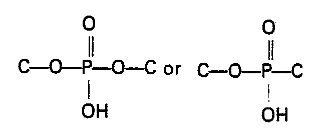 is effective as a dental adhesive. U.S. Patent No. US-A-4,222,780 shows that a polymerizable composition containing a vinyl compound having a group of the formula 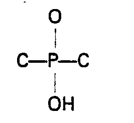 is also an effective dental adhesive.

In fact, some of the compositions defined by these patents are widely used as primers for application to the wall of a tooth cavity before the cavity is filled. They, however, require acid etching of the cavity wall in order to provide a satisfactory adhesive strength for the tooth. Moreover, they do not provide any satisfactory adhesive strength for a Ni-Cr alloy comprising 93% Ni and 6% Cr, which is used commonly in dentistry.

There have also been proposed dental adhesives prepared from polymerizable phosphate compounds as will hereinafter be described. None of them has, however, been put to practical use because of their unsatisfactory adhesive strength.
(i) U.S. Patent No. 3,882,600 describes phosphoryl monofluoride.
(ii) Journal of Dental Research, vol. 53, pages 878 to 888 and vol. 56, pages 943 and 952, Chemical Abstract, vol. 77, page 290 (66175 g) and Japanese Laid-Open Patent Specification No. 44152/1976 describe CH₂=CH-PO(OH)₂ and CH₂=CHC₆H_{Q}CH_{Z}PO(OH)₂.
(iii) Japanese Laid-Open Patent Specification No. 113843/1978 shows by a general formula the compounds obtained by neutralizing one of the two hydroxyl groups in the compounds of the formula 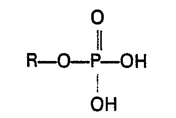 in which R stands for an organic residue having at least one vinyl group. More specifically, it shows the following compounds. In the following formulas, M stands for an alkali metal: 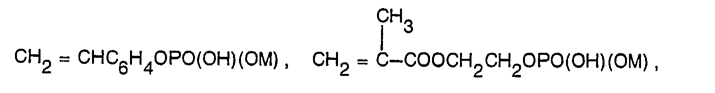 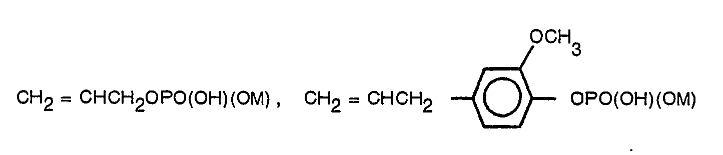 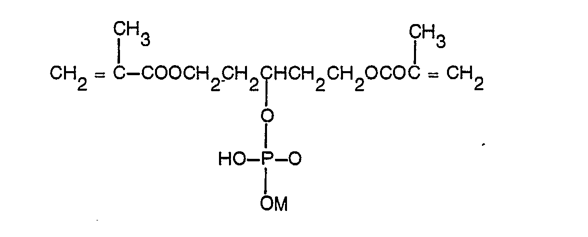 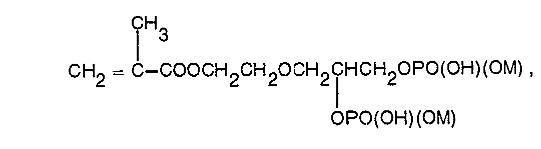 and

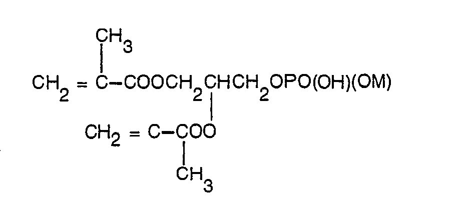

None of the compounds set forth at (i) to (iii) above provides a satisfactorily high adhesive strength in a wet condition.

There have also been made a number of other proposals to provide adhesives which are useful for both teeth and metals, as will hereinafter be summarized.

(i) U.S. Patent No. 4,148,988 shows 4-methacryloxy ethyl trimellitate as an adhesive monomer. This monomer, however, cannot maintain a strong bond between a tooth and a metal or organic polymer for a long time in the mouth in which a wet condition and repeated occlusal pressure prevail.

U.S. Patent No. 3,872,047 and Japanese Laid-Open Patent Specification No. 98878/1974 disclose a polymer having both a hydrophilic group and a hydrophobic group as an adhesive constituent. They show methacryloxyethyl phosphate as a monomer constituting the polymer. This polymer does, however, not appear to provide any satisfactory adhesive strength.

(iii) It is known that a polymer obtained by polymerizing a vinyl monomer on the tooth surface employing a ternary curing agent composed of a peroxide, an amine and a sulfinate has an improved strength on the tooth (U.S. Patent No. 4,182,035). No satisfactory adhesive strength can, however, be obtained from any combination employing a known vinyl monomer, but is is believed necessary to explore a new adhesive vinyl monomer.

It is known that if a polymerizable composition containing 2-(meth)acryloyloxyethyl dihydrogen phosphate is cured on an iron or stainless steel surface, the cured product adheres to the metal surface, as described in the various references which will hereinafter be cited. No attempt has, however, been hitherto made to obtain phosphate esters having a higher adhesive strength than 2-(meth)acryloyloxyethyl dihydrogen phosphate, particularly in a wet condition.
A. U.S. Patent No. 3,754,972 discloses the formation of an adhesive coating on a metal surface by applying a polymerizable composition containing a phosphate ester thereto. While it shows the applicable phosphate esters by way of a general formula, the examples set forth therein provide a specific support for the adhesive property of only the compounds of the following formula obtained by reacting hydroxyethyl acrylate with P₂0₅: 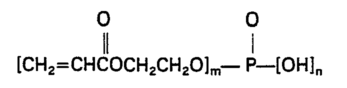 in which m is 1 or 2, n is 1 or 2, an m+n=3.
B. U.S. Patent No. 3,884,864 discloses the manufacture of a phosphorus cured material which is useful as a fire retardant material, and says that it shows a high adhesive strength on a metal. It-is, however, manufactured from phosphate esters obtained by reacting hydroxyethyl methacrylate or β-hydroxychloropropyl methacrylate with P₂0₅. They are the phosphate esters represented by the general formula I which will hereinafter appear, and in which R₂ has a maximum of three carbon atoms.
C. U.S. Patent No. 3,987,127 discloses a radiation polymerizable coating composition containing a polymerizable phosphate ester. It shows a general formula for the applicable phosphate esters which includes those represented by the formula I when R₂ stands for an alkylene group having a maximum of six carbon atoms. The compounds listed specifically therein, and employed in the examples are, however, only those obtained when R₂ is a group of the formula ―CH₂CH₂―. It does not give any example of the compounds containing an alkylene group having three or more carbon atoms.
D. U.S. Patent No. 4,001,150 discloses a composition containing a polymerizable phosphate ester, and used for preparing an electroconductive resin. The applicable phosphate esters shown by way of example therein are those represented by the formula I when R₂ is an alkylene group having a maximum of four carbon atoms. The patent says that it is not advisable to use any compound containing an alkylene group having more than four carbon atoms.
E. U.S. Patent No. 4,044,044 dicloses an anaerobic adhesive composition containing a polymerizable phosphate ester of the general formula: 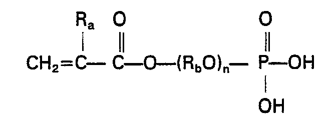 in which R₄ stands for H, CH₃ or C₂H₅, R_{b} stands for ―CH₂₇―, (̵CH₂)̵₂, (̵CH₂)̵₃, or t1m19 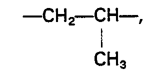 and n is an integer of 1 to 10.

It does, however, not show any phosphate ester specifically except 2-methacryloyloxyethyl dihydrogen phosphate.

F. Japanese Laid-Open Patent Specification No. 20238/1974 discloses an anaerobic adhesive containing a compound of the general formula 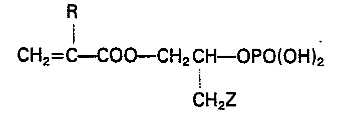 in which R stands for H or CH₃, and Z stands for a halogen. It does not show any attempt to use a phosphate ester having a larger number of carbon atoms.

Japanese Laid-Open Patent Specification No. 100596/1975 describes the usefulness of an organic compound having a P-OH group as one of the constituents of an electroconductive composition, and specifically shows by way of example a compound which corresponds to that of the formula I when R₂ stands for ―CH₂CH₂― or ―CH₂CH₂OCH₂CH₂―. It, however, fails to show any other compound having a larger number of carbon atoms.

Japanese Laid-Open Patent Specification No. 125182/1976 discloses a curable resin composition suitable for use in coating a metal surface, but which contains only a compound corresponding to that represented by the formula I when R₂ stands for -CH₂CH₂- or

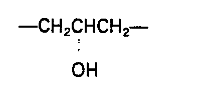

Japanese Laid-Open Specification No. 12995/1978 discloses a low temperature curable resin composition containing a phosphate ester which corresponds to that represented by the formula I when R₂ stands for

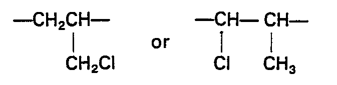

Japanese Laid-Open Patent Specification No. 11920/1981 discloses a polymerizable composition containing an epoxy diacrylate and a phosphate ester as a diluent therefor, and having a high adhesive strength on a metal. Although it shows a general formula for the compounds corresponding to those represented by the formula I when R₂ stands for -[CH₂CH_{2]1}- in which I is from 1 to 10, it specifically shows only a compound obtained when I is 1, and 2-methacryloyloxyethyl dihydrogen phosphate is the only phosphate ester employed in the examples set forth in the specification.

It will be noted from the foregoing that, though there are a lot of prior patents and patent applications disclosing compositions containing polymerizable phosphate esters as hereinabove mentioned at A to F, it is only the compositions containing 2-(meth)acryloyloxyethyl dihydrogen phosphate corresponding to the compounds represented by the formula I when R₂ stands for a group having two carbon atoms that have specifically been found effective as an adhesive, and that all the other similar compounds shown by way of example therein correspond merely to those represented by the formula I when R₂ stands for a group having two to four carbon atoms. Although these patents and patent applications state that the compositions containing those compounds exhibit a high adhesive strength on metals, none of them speaks of the adhesiveness of the compositions in a wet condition, nor do they contemplate the use of those compositions for dental applications.

The present invention provides an adhesive composition comprising 100 parts by weight of a polymerizable monomer comprising (a) 0.5 to 50 parts by weight of a compound represented by one of formulae I and II: 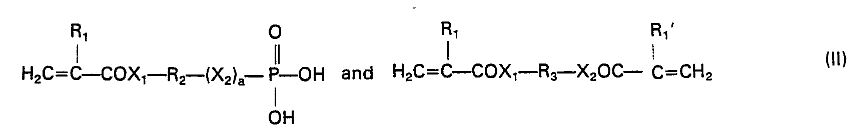 where each of R, and R,' is hydrogen or methyl;
R₂ is (i) a bivalent hydrocarbon group having 5 to 30 carbon atoms optinally having halogen, hydroxy, amino and/or carboxy substituent(s), or (ii) a bivalent organic group having 5 to 30 carbon atoms composed of two to seven hydrocarbon residues bonded to one another by one or more ether, thioether, ester, thioester, thiocarbonyl, amide, urethane, carbonyl and/or sulfonyl linkages, each of the hydrocarbon residues having 1 to 29 carbon atoms and at least one of them having four or more carbon atoms, and optionally having halogen, hydroxy, amino or carboxy substituents;
each of X, and X₂ is -0-, -S-, or -NH-;
a is 0 or 1, and
R₃ is a group of the formula 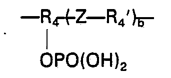 having 6 to 40 carbon atoms, where each of R₄ and R₄' is a hydrocarbon group having 1 to 29 carbon atoms optionally having as substituent(s) halogen, hydoxy, amino or carboxy, b is 0, 1, 2 or 3, the R₄'s, if more than one are present, being the same or different, and at least one of R₄ and R₄' having at least three carbon atoms, and Z is -0-, -COO- or -NH-; and (b) 50 to 99.5 parts by weight of a vinyl monomer copolymerizable with said compound; and
0.01 to 20 parts by weight of a curing agent.

As will appear from the experimental data below, compositions can be made in accordance with the present invention that adhere strongly to any of the hard tissues in a living body, such as teeth and bones, and to metals, organic polymers and ceramics, and that exhibit an excellent adhesive strength even in the presence of water. Such adhesive compositions can be used for bonding, with a high adhesive strength, a hard tissue in a living body to another tissue or to a material for the restoration of the tissue, such as a metal, organic polymer or ceramics, or for filling a hard tissue in a living body to restore it. In dentistry, they can be applied to the surface of a cavity in a tooth to form a strong bond between the tooth and the material with which the cavity is filled, as dental filling composition, for securing a restorative material, such as an inlay, onlay, crown, bridgework or orthodontic appliance, to a tooth tissue or abutment tooth, in every case having a high adhesion to the tooth. They can also be used industrially or in the home to bond one metal to another, a metal to an organic polymer or ceramics, one ceramic to another, or ceramics to an organic polymer, or as a coating agent or paint to form a highly adhesive film on a metal or ceramic surface.

The term "organic residue" as herein used means:
(i) a hydrocarbon group optionally having an OH, COOH or NH₂ group or a halogen atom (Cl, Br, F or I) as a substituent; or
(ii) a residue consisting of 2 to 20 hydrocarbon groups of the type defined above at (i) bonded to one another by ether, thioether, ester, thioester, thiocarbonyl, amide, carbonyl, sulfonyl or urethane linkages. Furthermore, the residue may have a principal chain composed of two or more hydrocarbon groups, possibly with at least one side chain formed at least in part by the hydrocarbon groups.

The following formulae exemplify the applicable organic residues. In the formulae,
A stands for a hydrocarbon group,
B stands for a linkage 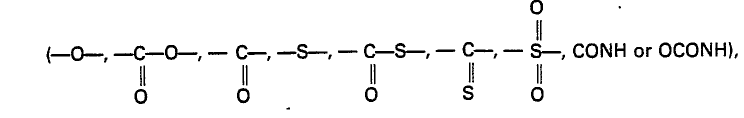 [C=C] stands for a double bond, and [PO(OH)₂] stands for phosphoric or phosphonic acid.

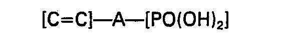

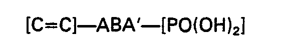

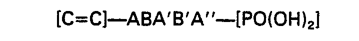

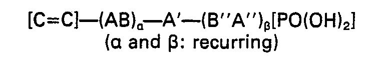

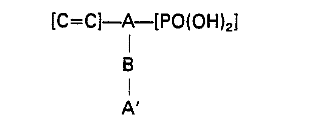

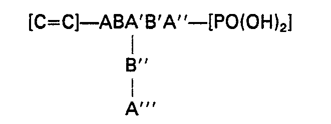

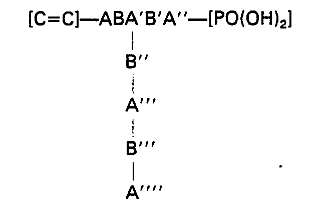

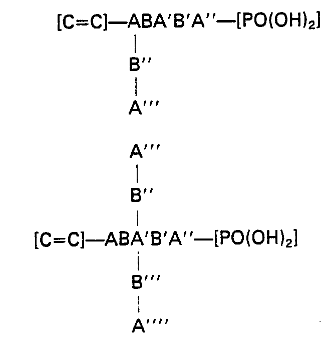

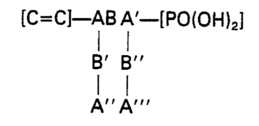

The term "hydrocarbon group" as herein used covers halogenated hydrocarbons even if not specifically mentioned.

The organic residue R₂ defined above at (b) is more particularly of the following formulae:
(a) ―R₅(̵Y―R₅')̵_{c}, in which each of R₅ and R₆' stands for a hydrocarbon group having 1 to 29 carbon atoms, and optionally having as substituent(s) halogen, hydroxy, amino or carboxy; Y stands for-0-, ―S―, ―COO―, ―COS―, 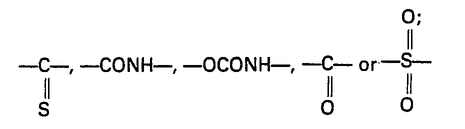 and c is an integer of from 1 to 6, and if there are two or more R₅'s, they are the same or different, at least one hydrocarbon group having at least four carbon atoms; or 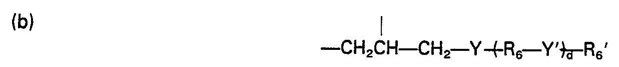 in which Y is as defined above, Y' is the same as Y, d is 0, 1,2 or 3, and if d is 0, R₆' stands for a hydrocarbon group having 4 to 27 carbon atoms, and optionally having as substituent(s) halogen, hydroxy, amino or carboxy, while if d is 1, 2, or 3, each of R₆ and R₆' stands for a hydrocarbon group having 4 to 27 carbon atoms, and optionally having as substituent(s) halogen, hydoxy, amino or carboxy, at least one of hydrocarbon groups R₆ and Rₛ' having at least four carbon atoms.

The compounds of the formula I in which X₁ and X₂ both stand for ―O― and a is 1 are preferably for dental application from the standpoints of biological safety, storage stability and adhesive strength. In these commpounds, R, is usually a methyl group and R₂ preferably stands for any of the following: 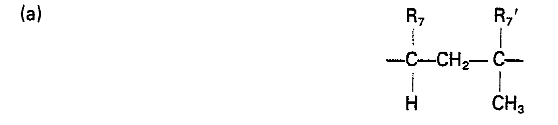 in which each of R₇a nd R₇' stands for a hydrogen atom or a hydrocarbon group having 1 to 4 carbon atoms, optionally having halogen substituent(s); 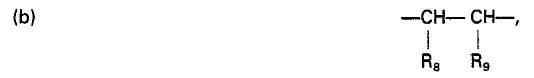 in which R₈ stands for hydrogen or methyl and R₉ stands for a hydrocarbon group having 3 to 28 carbon atoms, optionally having halogen substituent(s), particularly 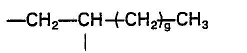 in which g is an integer of 2 to 20, or 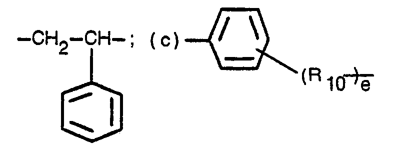 in which R₁₀ stands for a hydrocarbon group having 1 to 12 carbon atoms, optionally halogen-substituted, andeis0or1;
(d) (̵CH₂)̵_{f}, in which f is an integer of from 5 to 20, preferably 5 to 12, particularly 8 to 12; 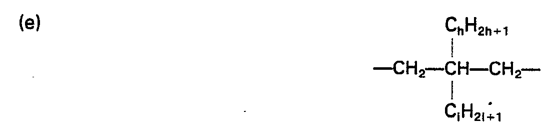 in which h is an integer of from 1 to 5, i is 0, 1, 2 or 3, and h + i is at least 2; 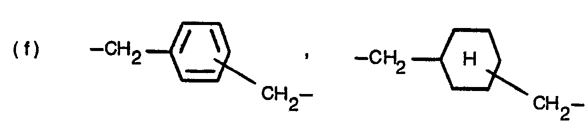 or 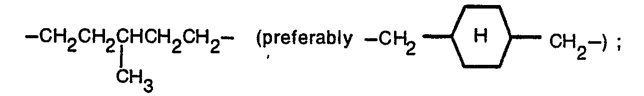 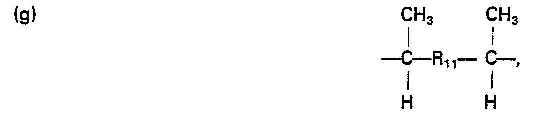 in which R₁₁ stands for a hydrocarbon group having 2 to 6 carbon atoms, and optionally halogen-substituted; 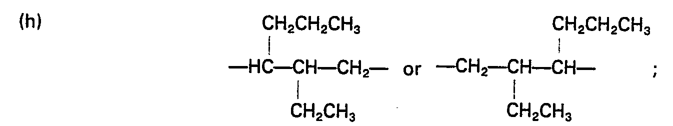 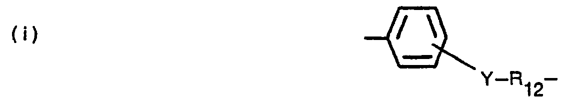 in which R₁₂ stands for a hydrocarbon group having 1 to 12 carbon atoms, optionally halogen-substituted, and Y preferably stands for ―O― or ―COO―; 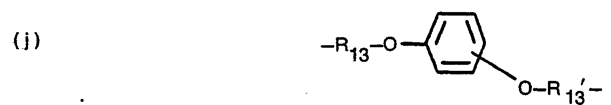 in which each of R₁₃ and R₁₃' stands for a hydrocarbon group having 1 to 6 carbon atoms, optionally halogen-substituted; preferably, 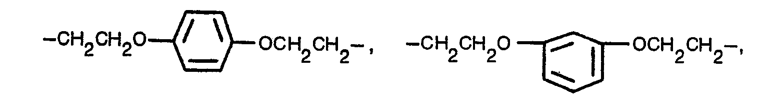 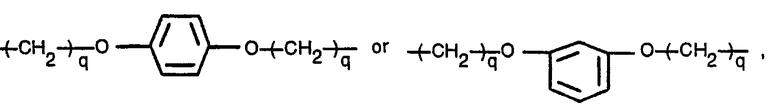 in which q is 3 or 4 ; 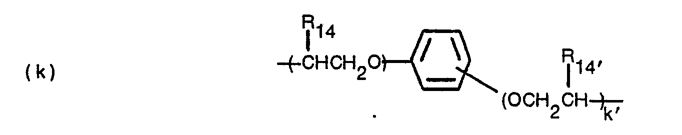 in which each end of R₁₄ and R₁₄. stands for hydrogen or methyl, and each of k and k' is 2 or 3; 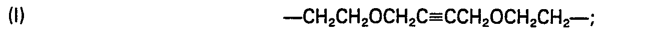 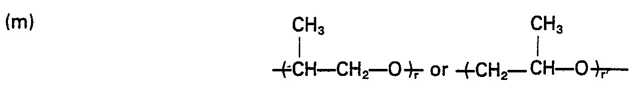 in which each of r and r' is an integer from 2 to 6; or 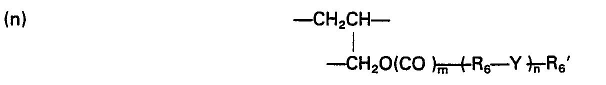 in which R₆ and R₆' are as defined above, m is 0 or 1, and n is 0, 1, 2 or 3. At least one of R₆ and R₆' preferably stands for an aromatic hydrocarbon. R₆ may have one or two branches of the formula (̵Y―R₆")ₛ in which Y is as defined above, R₆' is equal to R₆, and s is 1 or 2. R₆ and R₆' may be bonded to each other by two Ys in the form of 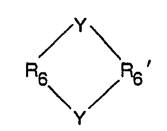 when n is 1.

The phosphate ester of the formula II preferably contains a group in which R₄ stands for ―CH₂CHCH₂―. The adhesive monomerssuitable for use in the preparation of the adhesive composition of this invention will hereunder be shown by way of example:

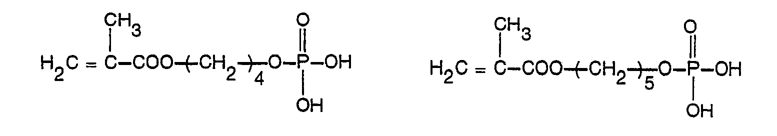

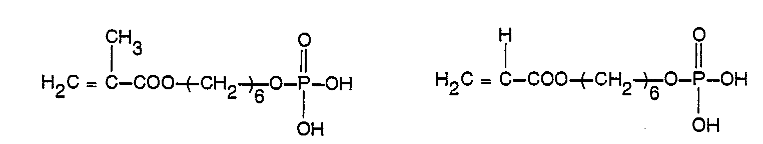

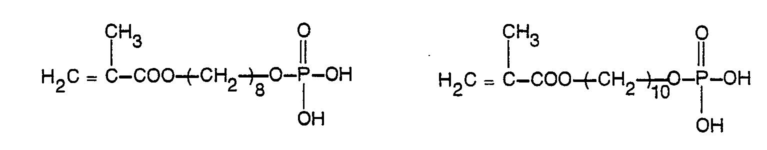

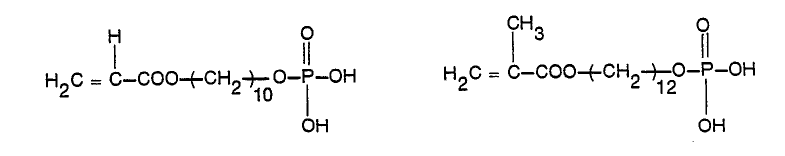

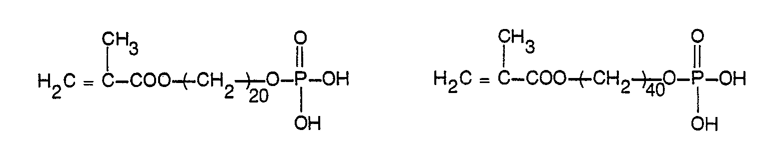

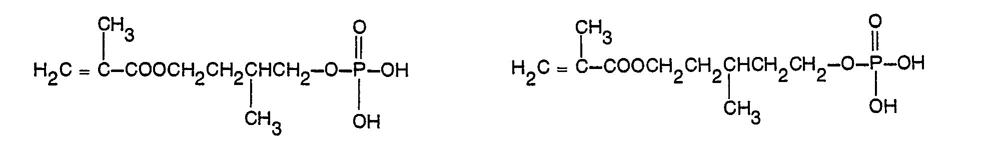

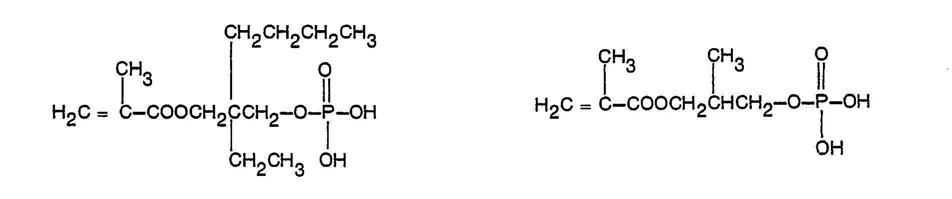

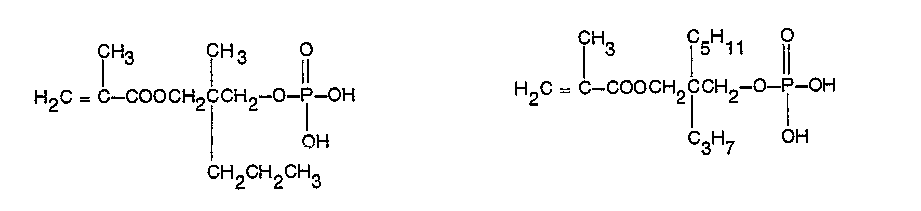

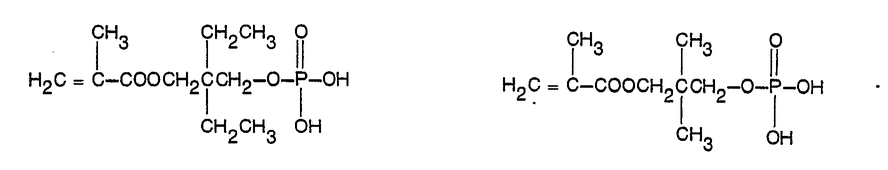

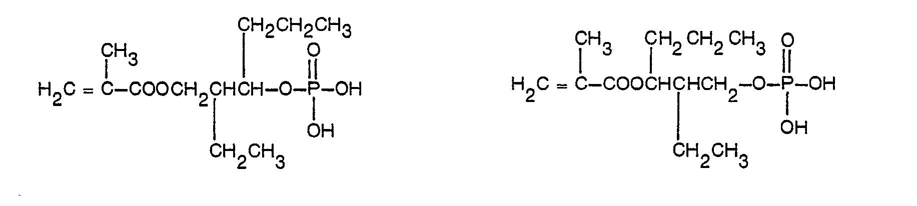

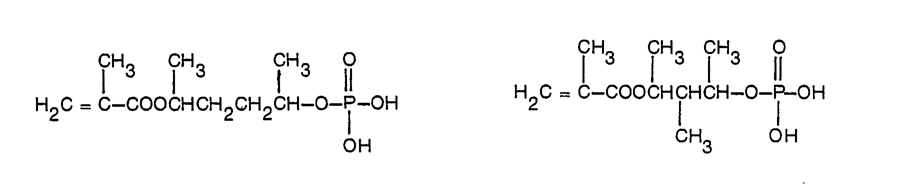

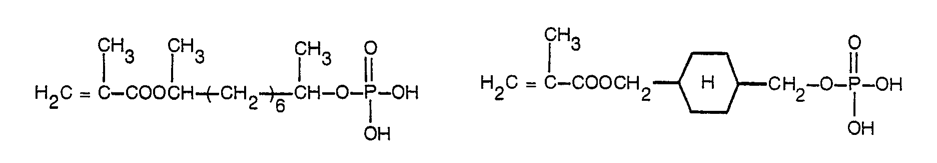

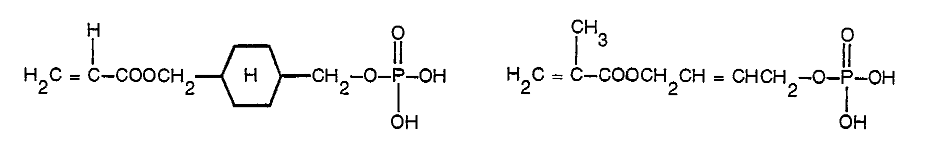

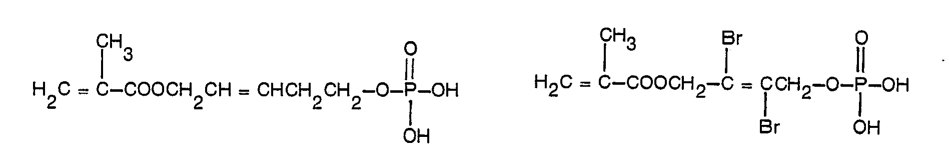

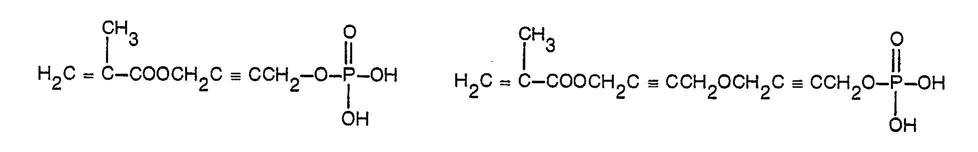

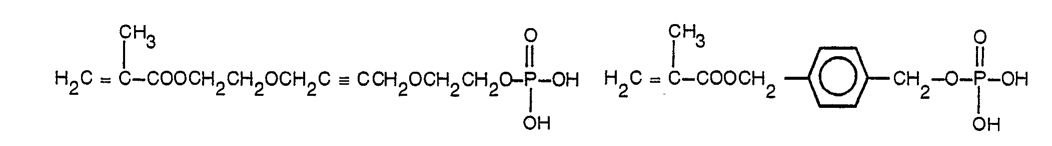

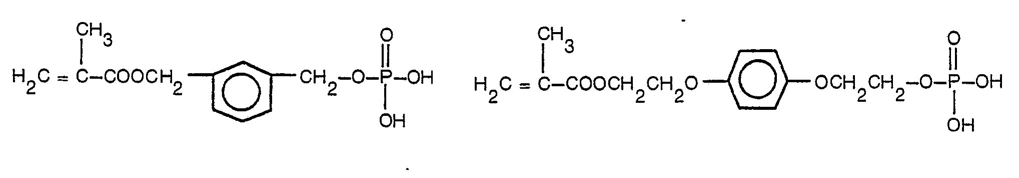

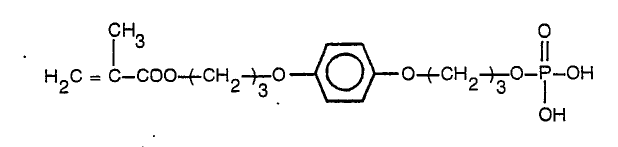

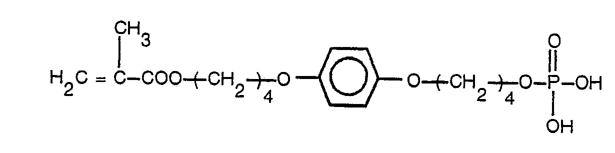

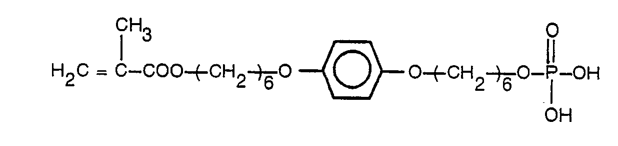

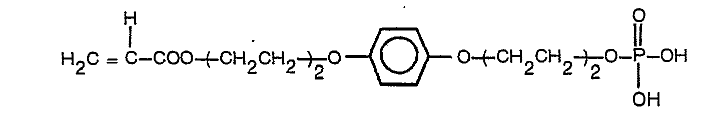

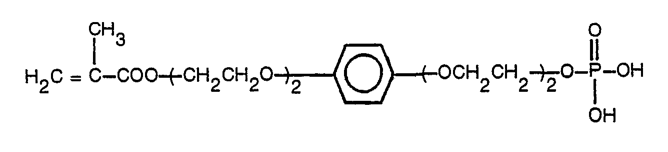

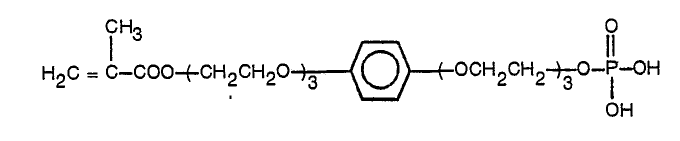

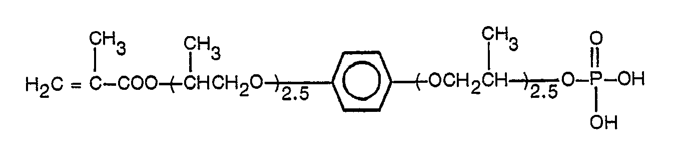

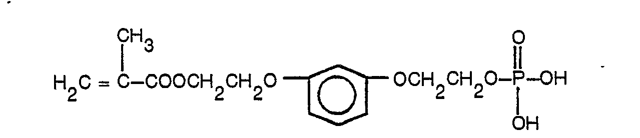

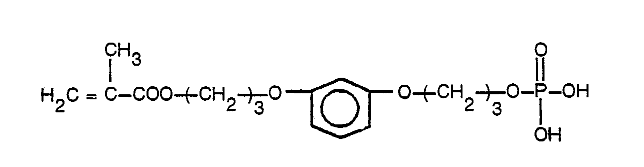

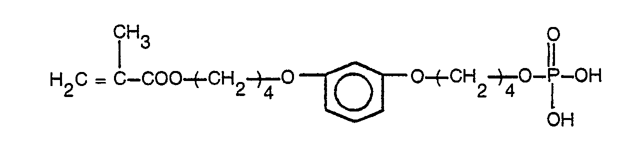

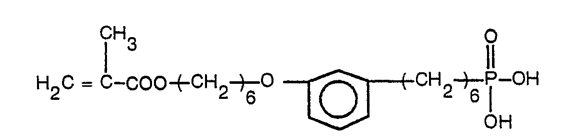

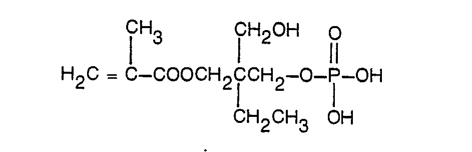

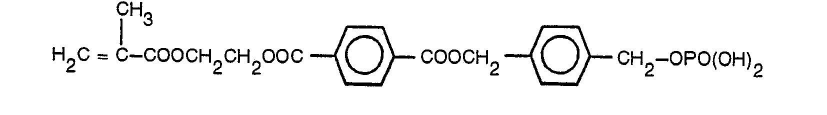

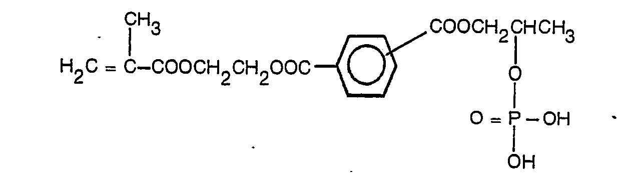

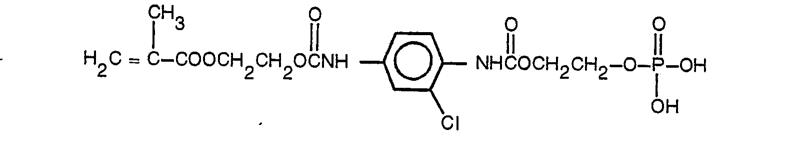

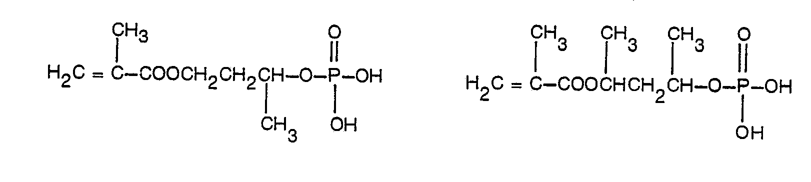

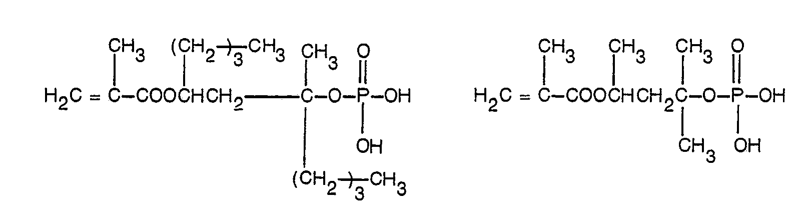

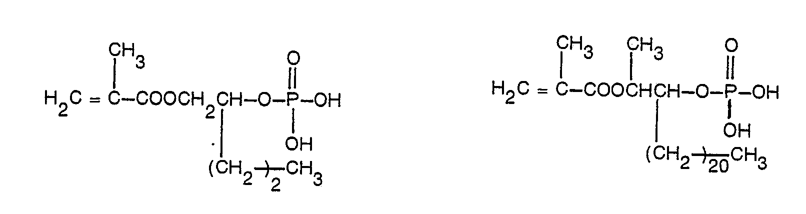

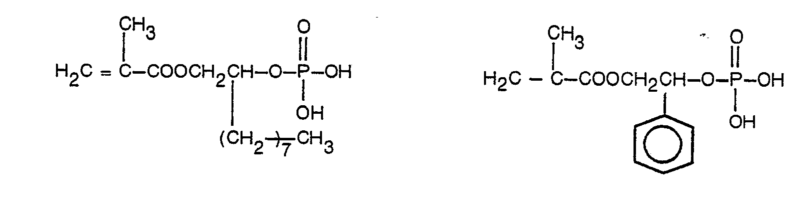

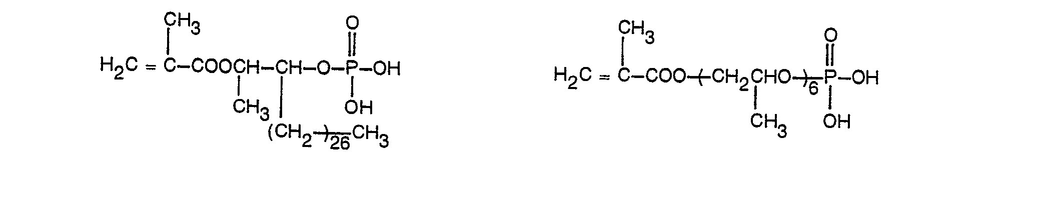

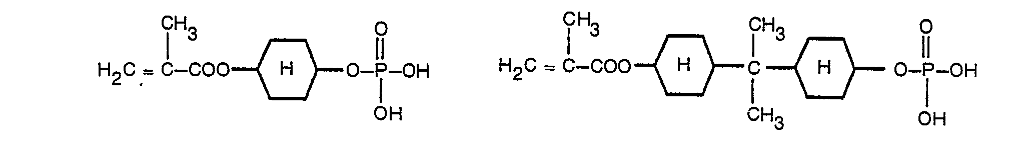

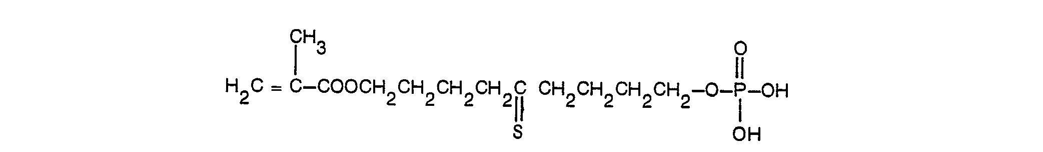

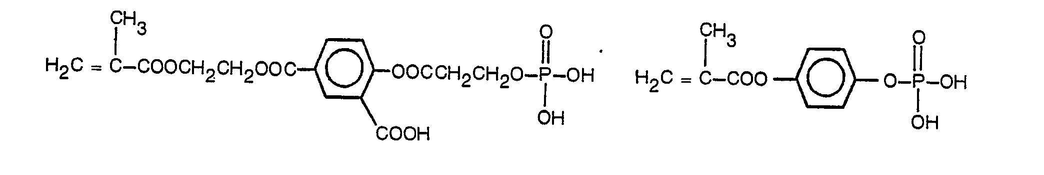

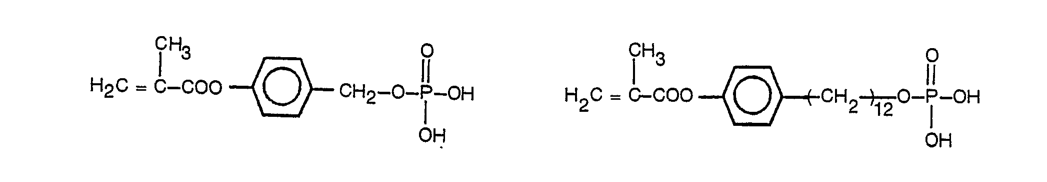

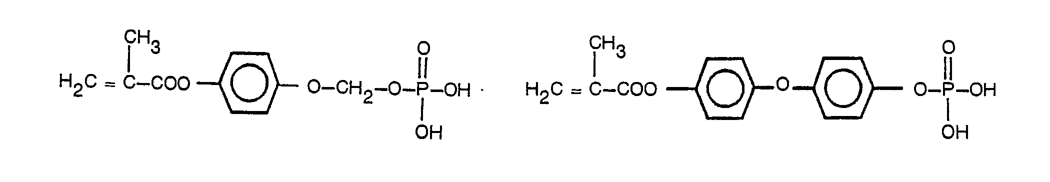

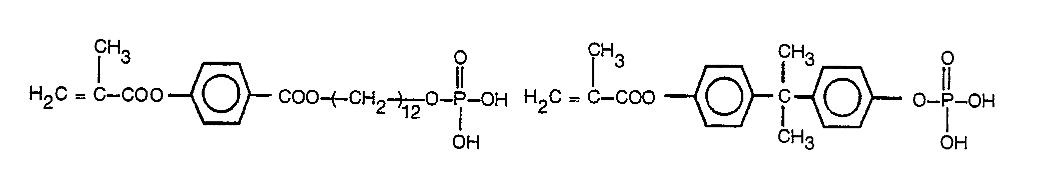

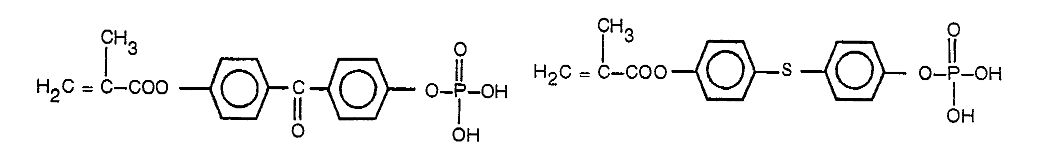

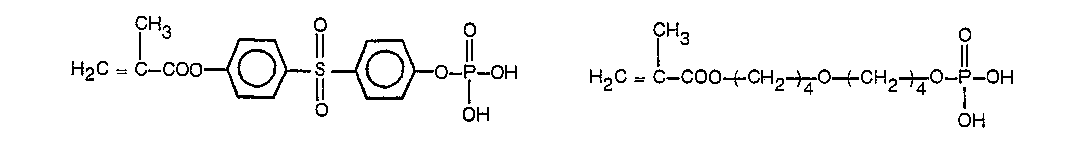

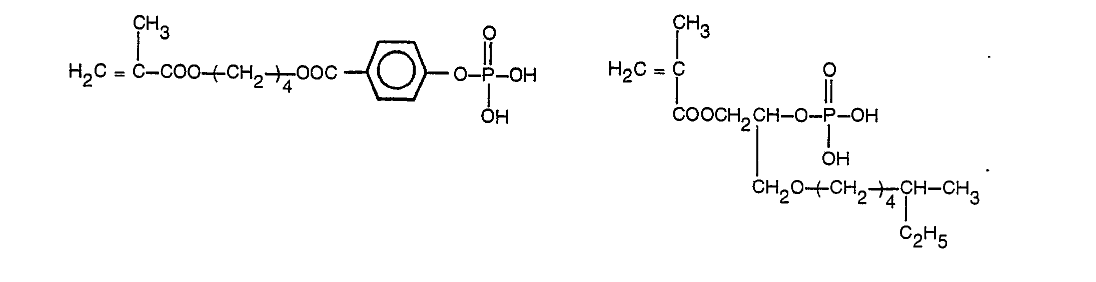

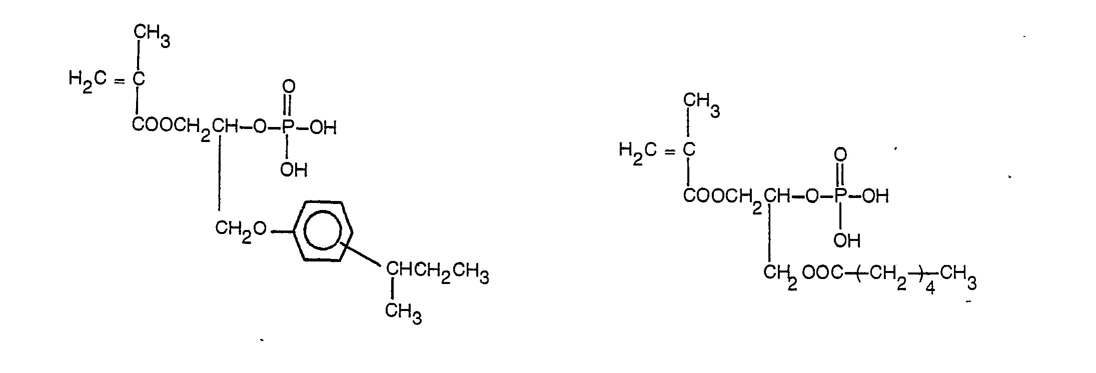

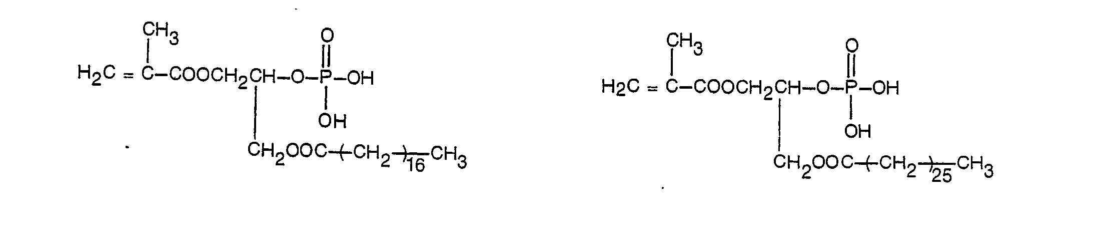

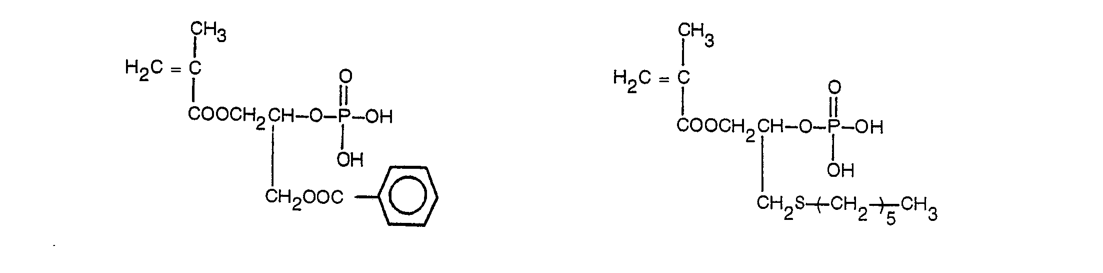

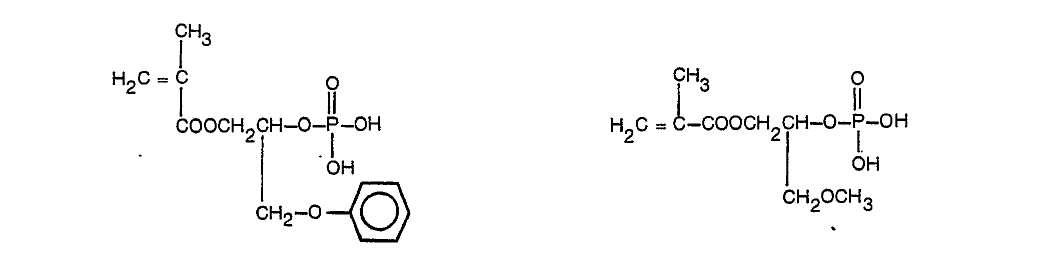

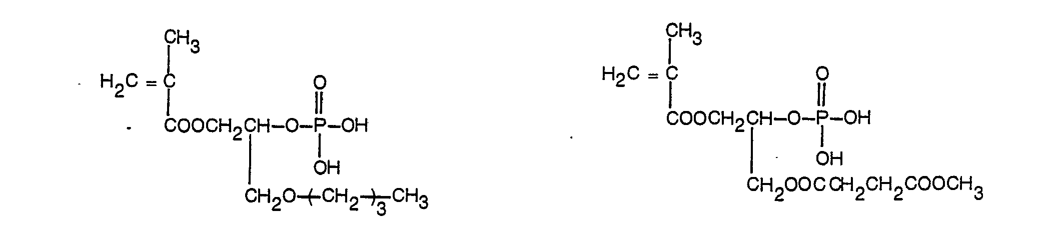

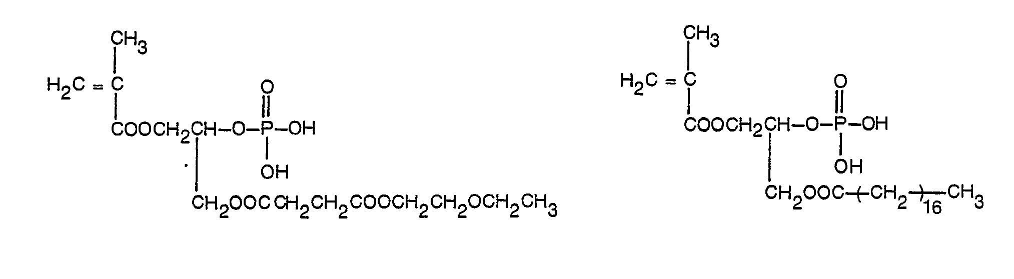

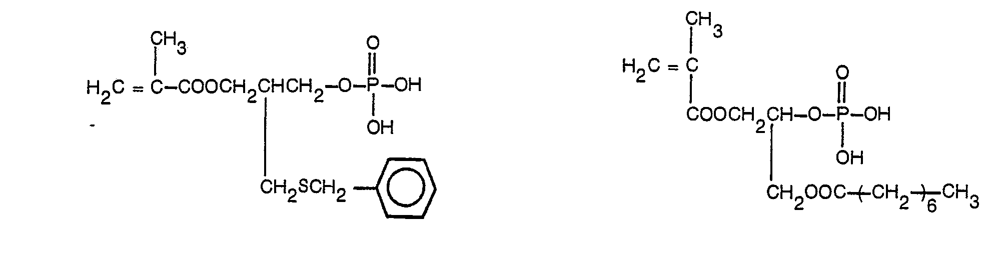

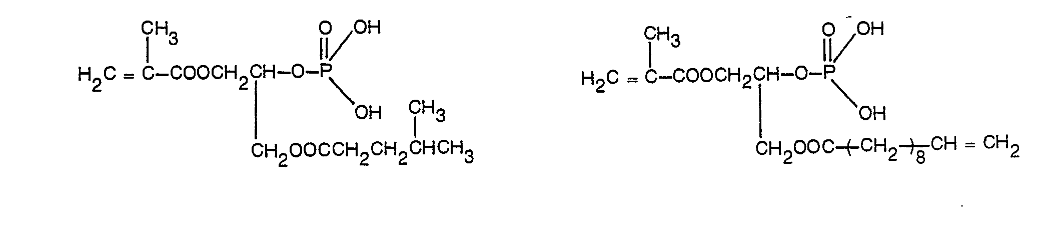

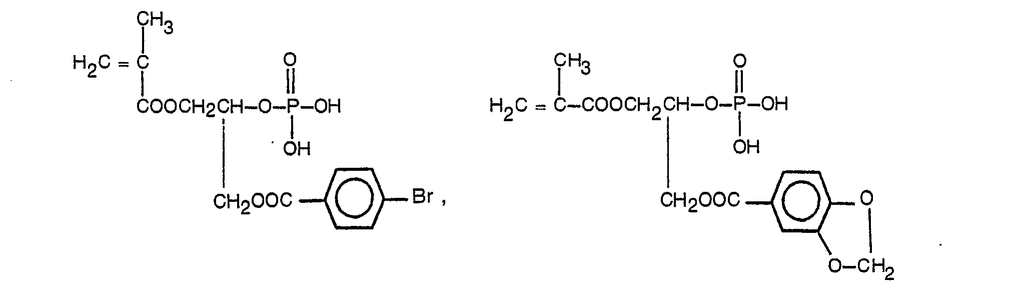

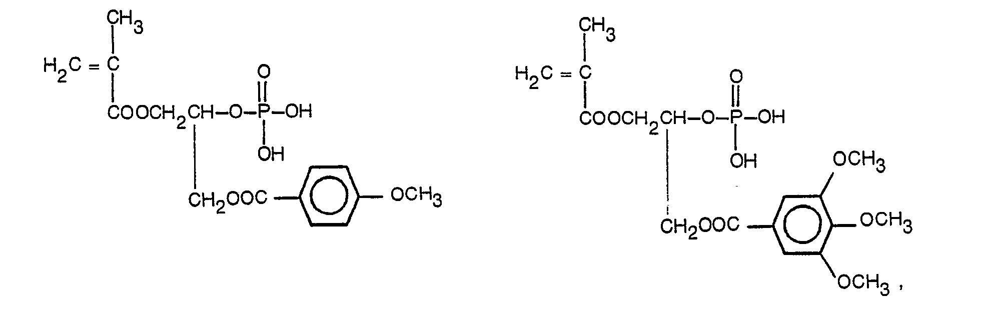

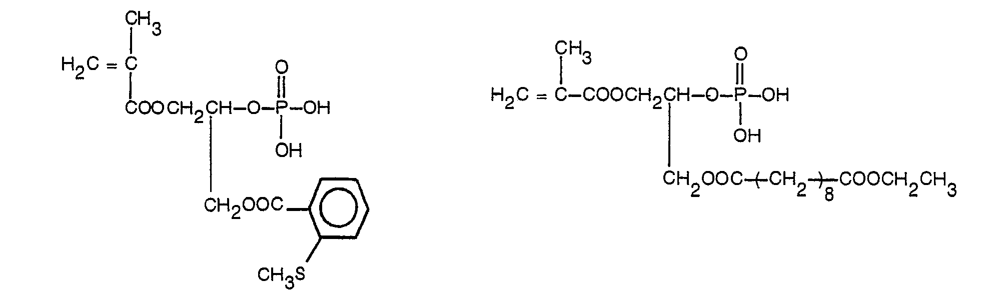

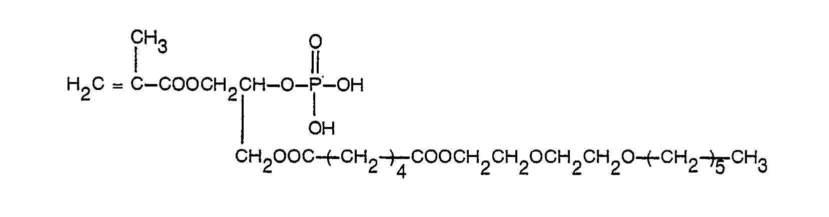

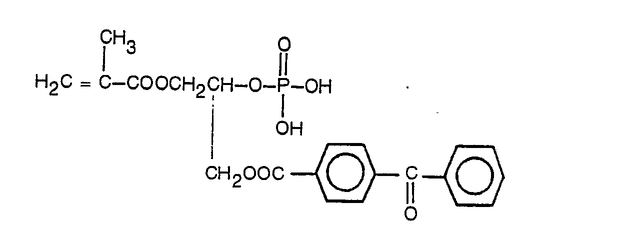

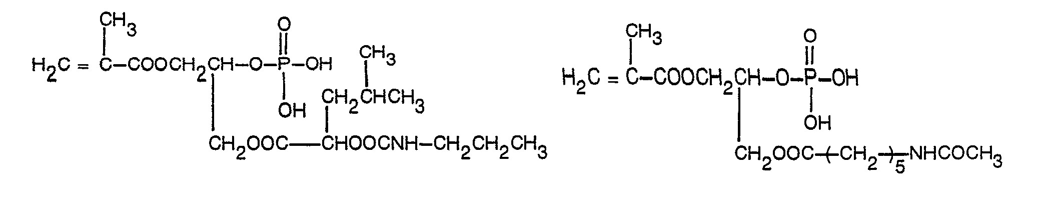

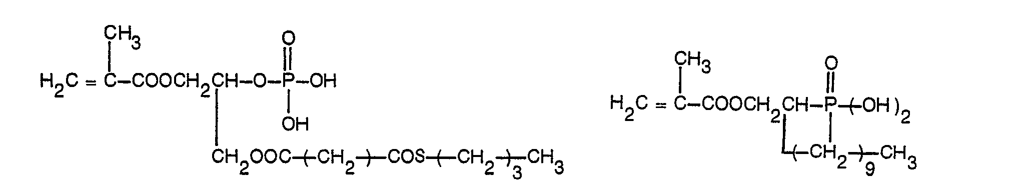

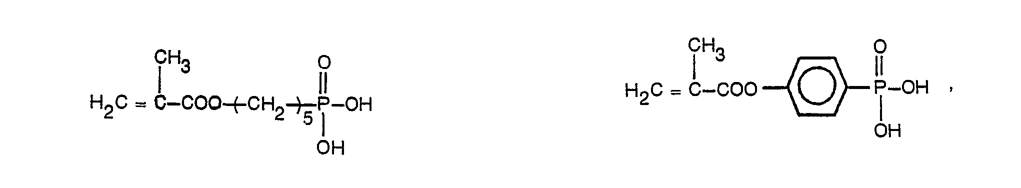

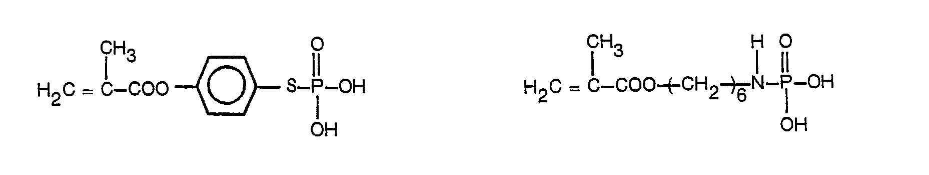

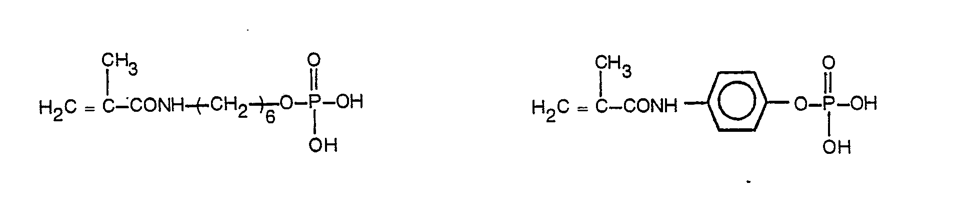

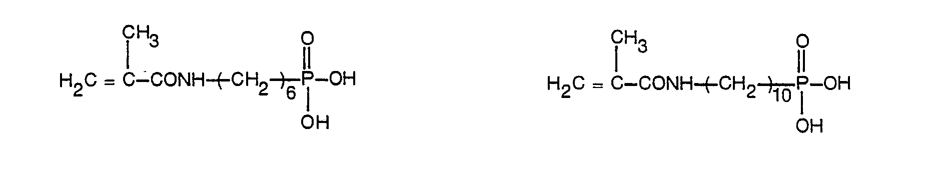

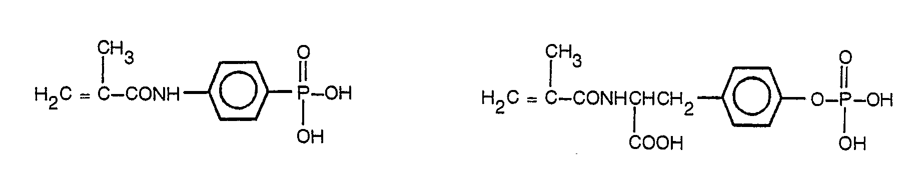

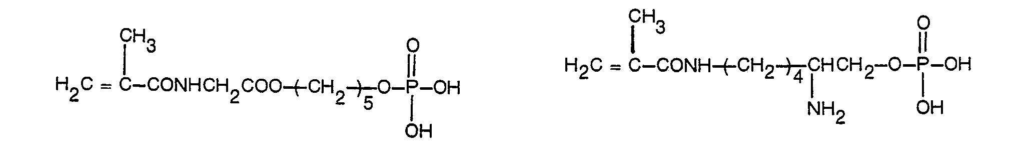

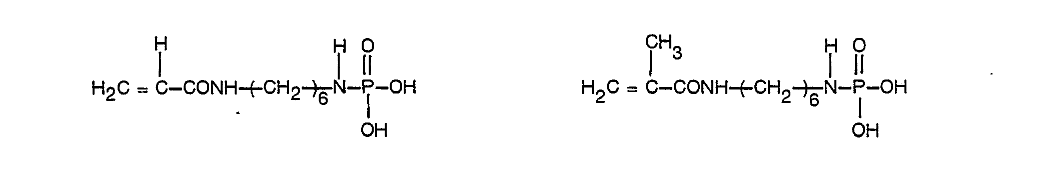

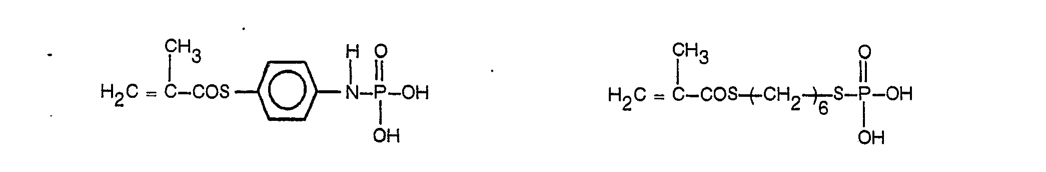

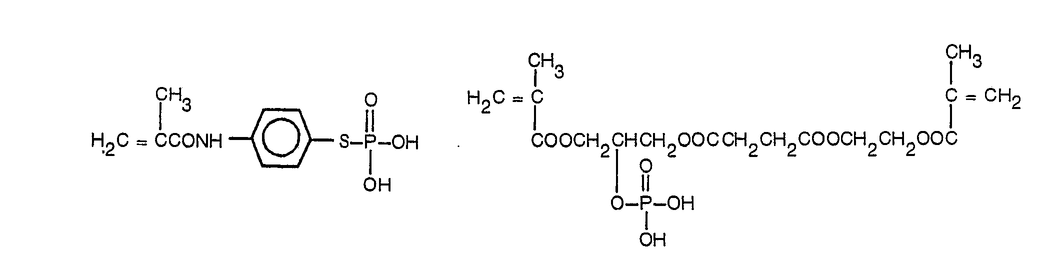

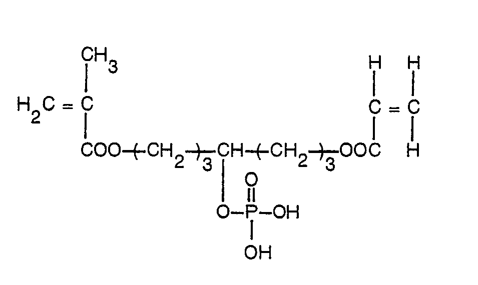

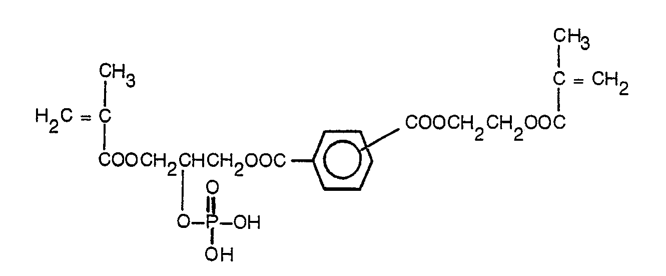

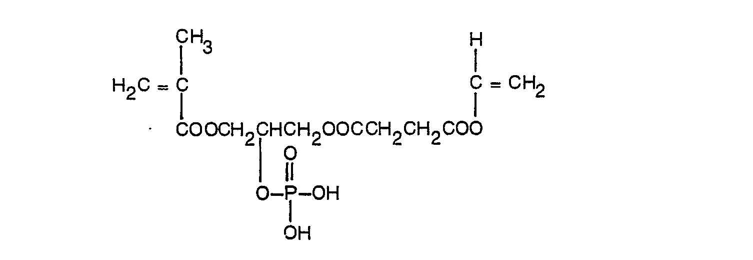

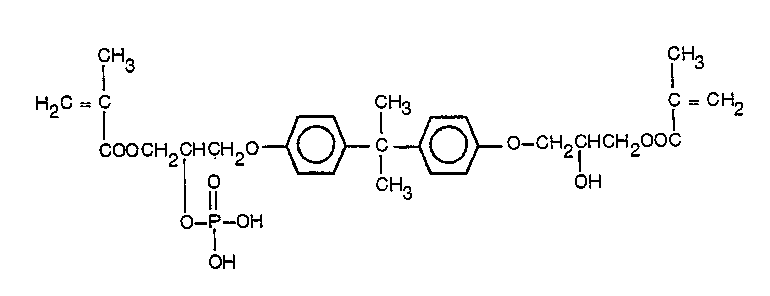

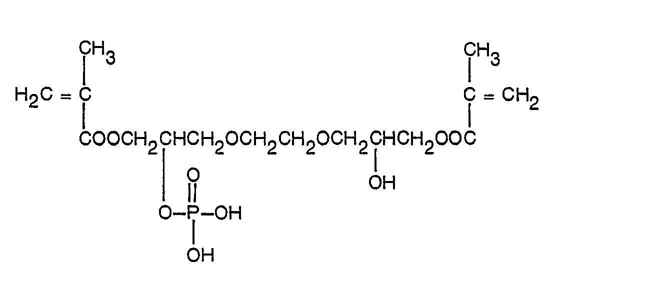

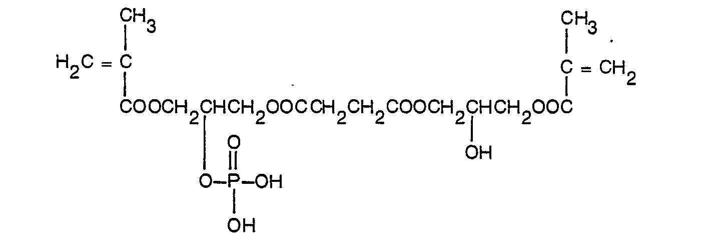

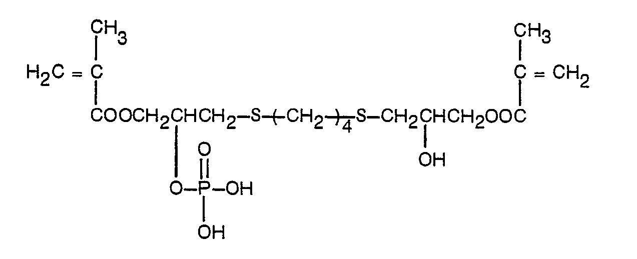

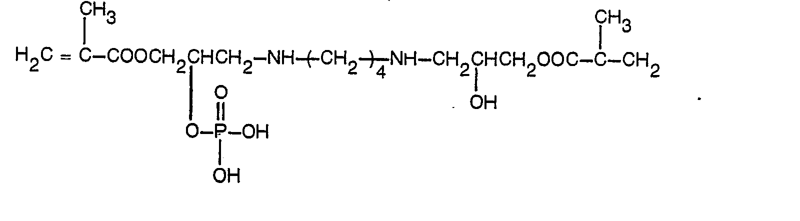

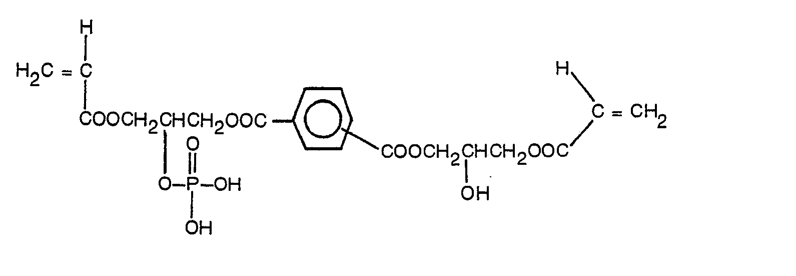

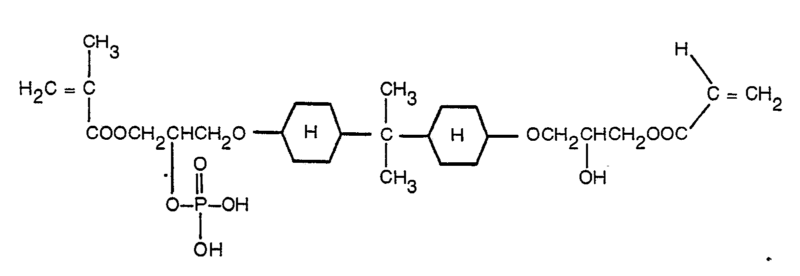

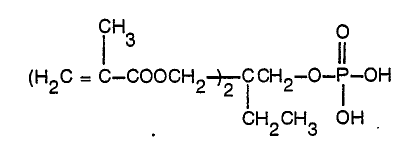

It is preferable to use a phosphate monoester of the general formula: 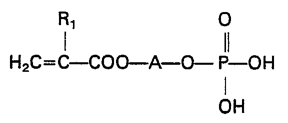 in which R₁ stands for hydrogen or a methyl group, and A stands for any of the following formulas 3 to 6:

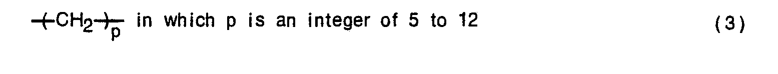

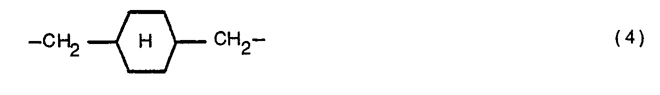

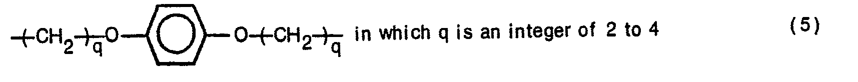

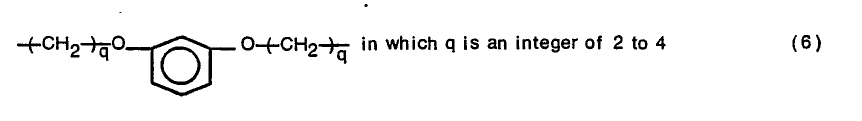

The formulas 3 to 6 specifically represent the following compounds:

### Formula 3:

5-(Meth')acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphatge, 9-(meth)acryloyloxynonyl dihydrogen phosphate, 10-(meth)acryloyloxydecyl dihydrogen phosphate, 11-(meth)acryloyloxyundecyl dihydrogen phosphate, and 12-(meth)acryloyloxydodecyl dihydrogen phosphate;

### Formula 4:

4-(Methacryloyloxymethyl)cyclohexylmethyl dihydrogen phosphate, and 4-(acryloxyoxymethyl)cyclohexylmethyl dihydrogen phosphate;

### Formula 5:

2-[P-{2-(methacryloyloxy)ethoxy}phenoxy]ethyl dihydrogen phosphate, 3-[P-{3-(methacryloyloxy)-propoxy}phenoxy]propyl dihydrogen phosphate, 4-[P-{4-(methacryloyloxy)butoxy}phenoxy]butyl dihydrogen phosphate, 2-[P-{2-acryloyloxy)ethoxy}phenoxy]ethyl dihydrogen phosphate, 3-[P-{3-(acryloyl- oxy)propoxy}phenoxy]propyl dihydrogen phosphate, and 4-[P-{4-(acryloyloxy)butoxy}phenoxy]butyl dihydrogen phosphate;

### Formula 6:

2-[m-{2-(methacryloyloxylethoxy}phenoxy]ethyl dihydrogen phosphate, 3-[m-{3-(methacryloyloxy)-propoxy}phenoxy]propyl dihydrogen phosphate, 4-[m-{4-(methacryloxy)butoxy}phenoxy]butyl dihydrogen phosphate, 2-[m-{2-(acryloyloxy)ethoxy}phenoxy]ethyl dihydrogen phosphate, 3-[m-{3-(acryloyloxy)propoxy}phenoxy]propoyl dihydrogen phosphate, and 4-[m-{4-(acryloyloxy)butoxy}- phenoxy]butyl dihydrogen phosphate.

The compounds of the formula I in which R₂ stands for a group having not more than three carbon atoms are extremely inferior to the compounds employed in accordance with this invention in the adhesive strength on a tooth, metal or the like, and the water resistance of the adhesive bond. These compounds generally show an increased adhesive strength with an increase in the number of the carbon atoms which R₂ has, and provide the highest adhesive strength when R₂ has 6 to 20 carbon atoms. Their adhesive strength begins to drop if the number of the carbon atoms in R₂ exceeds 30.

The compounds of the formulas I and II can generally be synthesized in accordance with the processes for the synthesis of phosphorus compounds as described in Organophosphorus Compounds (G. M. Kosolapoff, Wiley, 1950), Organophosphorus Monomers and Polymers (Ye. L. Gefter, Pergamon Press, 1962), Modern Organic Synthesis Series 5, Organophosphorus Compounds (The Society of Synthertic Organic Chemistry, Japan, Gihodo, 1971) and Beilstein (Springer-Verlag). For example, the compounds of the formula 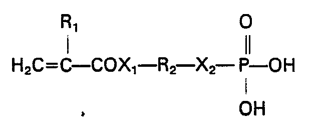 can be easily obtained by reacting 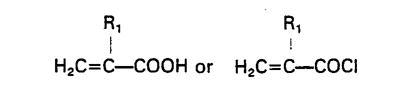 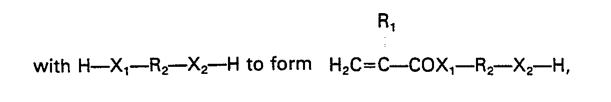 reacting it with at least an equimolar amount of phosphorus oxychloride in the presence of a tertiary amine to form a compound of the formula 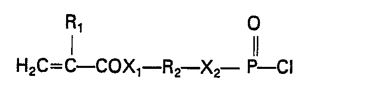 and hydrolyzing the P-Cl bonds therein. The compounds of the formula 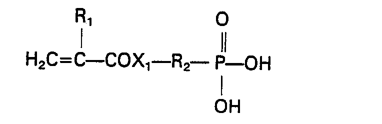 can be obtained by synthesizing H―X₁―R₂―PO₃₂²⁻.N2a⁺ or H―X₁―R₂―PO(OR)₂, in which R stands for a hydrocarbon group having 1 to 10 carbon atoms, and reacting it with 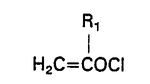 under the Shotten-Baumann's reaction conditions. If R₂ stands for 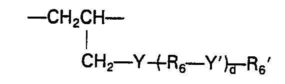 the phosphate ester for use in the preparation of the adhesive composition according to this invention may be obtained as will hereunder be described. Equimolar amounts of 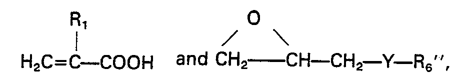 in which R₆" stands for (R₆―Y')_{d}R₆', are reacted with each other in the presence of triethylbenzylammonium chloride to form 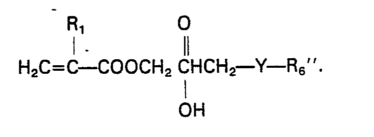 Similarly, 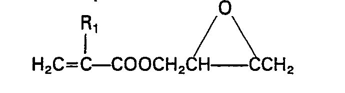 and HOOC-R₆" are reacted with each other to form 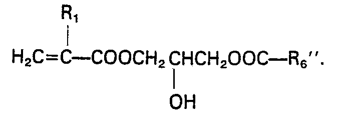 hydroxyl compounds are reacted with at least an equimolar amount of phosphorus oxychloride in the presence of a tertiary amine to form 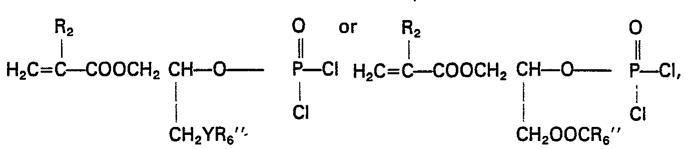 followed by the hydrolysis of the P-Cl bonds therein.

The adhesive composition of this invention also contains a vinyl monomer which is copolymerizable with the compound of the formula I or II. The viscosity, wettability, curability and mechanical properties of the adhesive depend on the copolymerizable vinyl monomer to be employed. While various types of vinyl monomers can be used in accordance with the requirements for the adhesive to be prepared, it is usually advisable to employ a (meth)acrylate type monomer, a styrene type monomer or vinyl acetate. It is, however, possible to employ other compounds, for example, acrylamides such as (meth)acrylamide, N-n-butoxymethyl(meth)acrylamide and N-(hydroxymethyl)acrylamide, (meth)acrylic acid, isobutylvinyl ether, diethyl fumarate, diethyl maleate, methyl vinyl ketone, allyl chloride, vinylnaphthalene and vinylpyridine. Suitable examples of the styrene type monomers include compounds of the formula 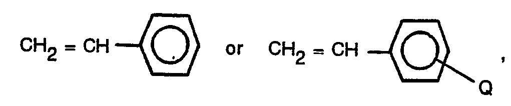 in which Q stands for a halogen atom or a hydrocarbon group having 1 to 6 carbon atoms, or more specifically, divinylbenzene and p-chlorostyrene.

The (meth)acrylate type monomers often employed in the preparation of conventional anaerobic or dental adhesives can advantageously be used for preparing the adhesive composition of this invention. These monomers are represented by the formula 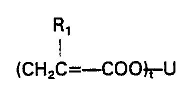 H or CH₃, U stands for an organic residue (as hereinabove defined) having 1 to 50 carbon atoms, and t is an integer of 1 to 4. Specific examples of these monomers are as follows:

### (1) Monofunctional (meth)acrylates:

Methyl (meth)acrylate, ethyl (meth)acrylate, n-butyl (meth)acrylate, isobutyl (meth)acrylate, n-hexyl (meth)acrylate, 2-ethylhexyl (meth)acrylate, benzyl (meth)acrylate, decyl (meth)acrylate, lauryl (meth)acrylate, stearyl (meth)acrylate, 2-hydroxyethyl (meth)acrylate (HEMA), 2-hydroxypropyl (meth)acrylate, dimethylaminoethyl (meth)acrylate, diethylaminoethyl (meth)acrylate, 3-chloro-2-hydroxypropyl methacrylate, and 2,3-dibromopropyl (meth)acrylate;

### (2) Bifunctional (meth)acrylates:

(a) Compounds of the above formula in which Q stands for -CH2CH2(OCH2CH2)s- or 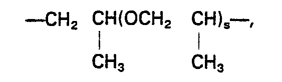 Ethylene glycol di(meth)acrylate, diethylene glycol di(meth)acrylate, triethylene glycol di(meth)acrylate, tetraethylene glycol di(meth)acrylate, polyethylene glycol di(meth)acrylate, propylene glycol di(meth)acrylate, dipropylene glycol di(meth)acrylate, and tripropylene glycol di(meth)acrylate;
(b) Compounds of the above formula in which Q stands for an alkylene normally having 3 to 12 carbon atoms:
   Propanediol di(meth)acrylate, glycerol di(meth)acrylate, 1,3-butanediol di(meth)acrylate, 1,4-butanediol di(meth)acrylate, 1,5-pentanediol di(meth)acrylate, 1,6-hexanediol di(meth)acrylate, neopentyl glycol di(meth)acrylate, 1,10-decanediol di(meth)acrylate, and 2,3-dibromoneopene glycol dimethacrylate;
(c) Compounds of the above formula in which Q contains a bisphenol A derivative residue:
   Bisphenol A di(meth)acrylate, 2,2-bis[(meth)acryloyloxypolyethoxyphenyl]propane 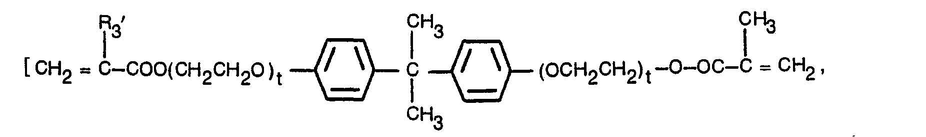 in .which t is an . integer of 1 to 9 and R'₃ is hydrogen or methyl], 2,2'-bis(4-acryloyloxypropoxyphenyl)propane and 2,2'-bis[4-(3-methacryloyloxy-2-hydroxypropoxy)phenyl]propane (Bis-GMA), among which the compounds in which Q has 15 to 30 carbon atoms are particularly preferred;
(d) Compounds of the above formula in which Q stands for 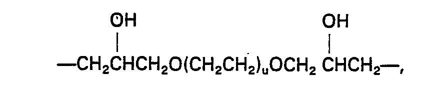 in which u is 1 or 2:
   1,2-Bis[3(meth)acryloyloxy₋2-hydroxypropoxy]ethane, and 1,4-bis[3-(meth)acryloyloxy-2-hydroxypropoxy] butane;
(e) Urethane di(meth)acrylates of the above formula in which Q stands for JOCONHTNHCOOJ, in which J stands for an alkylene group normally having 2 to 10 carbon atoms, and T stands for an organic diisocyanate residue having 1 to 50 carbon atoms, as disclosed, for example,.in Japanese Laid-Open Patent Specification No. 687/1975;

### (3) Trifunctional and tetrafunctional methacrylates:

Trimethylolpropane tri(meth)acrylate, trimethylolethane tri(meth)acrylate, tetramethylolmethane tri(meth)acrylate, tetramethylolmethane tetra(meth)acrylate, and N,N'-(2,2,4-trimethylhexamethylene)-bis[2-(aminocarboxy)propane-1,3-diol]tetramethacrylate.

One or more of these copolymerizable monomers are employed. For preparing a dental adhesive composition, it is advisable to use a methacrylate or methacrylates in the quantity of at least 50% by weight of all the copolymerizable monomers employed. Preferred examples of the methacrylates include methyl methacrylate, ethyl methacrylate, HEMA, n-hexyl methacrylate, benzyl methacrylate, lauryl methacrylate, Bis-GMA, bisphenol A dimethacrylate, 2,2-bis[(meth)acryloyloxypolyethoxyphenyl]propane, ethylene glycol dimethacrylate, diethylene glycol dimethacrylate, triethylene glycol dimethacrylate, 1,6-hexanediol dimethacrylate, 1,10-decanediol dimethacrylate, neopentyl glycol dimethacrylate, and trimethylolethane trimethacrylate.

For preparing the adhesive composition of this invention, it is necessary to employ the compound of the formula I or II in the quantity of 0.5 to 50%, or preferably 1.5 to 40%, by weight of all the polymerizable monomers employed. If it is less than 0.5% by weight, no satisfactorily high adhesive strength is obtained, while the use of more than 50% by weight is likely to result in an adhesive bond of lower water resistance. The water resistance of an adhesive bond is also affected by the type of the copolymerizable monomer employed. If a water-soluble monomer having a hydrophilic functional group, such as (meth)acrylic acid, 2-hydroxyethyl (meth)acrylate or methacrylamide, is employed, therefore, it is highly advisable to ensure that it not occupy more than 50% by weight of all the polymerizable monomers employed.

The adhesive composition of this invention is applied to adherend surfaces, and cured either physically by heating or irradiation with X-rays or ultraviolet or visible light, or chemically by a polymerization initiator. It is usually advisable to polymerize by irradiating light in the presence of a photosensitizer, or using a polymerization initiator. The term "curing agent" as herein used covers both of the polymerization initiator and the photosensitizer together. It is possible to use various curing agents, for example, organic peroxides, azo compounds, organic metal compounds, redox type initiators, and photosensitizers for ultraviolet or visible rays. More specifically, it is possible to use, for example, benzoyl peroxide, di-t-butyl peroxide, cumene hyhdroperoxide, t-butyl hydroperoxide, methyl ethyl ketone peroxide, azobisisobutyronitrile, tributylborane, organic sulfinic acids or the salts thereof, hydrogen peroxide/Fe²- salts, cumene hydroperoxide/Fez+ salts, benzoyl peroxide/N,N-dialkylaniline derivatives, ascorbic acid/ Cu2+ salts, organic sulfinic acid (or the salt thereof)/amine (or the salt thereof)/peroxide, a-diketone/ allylthiourea (for visible rays), benzoin methyl ether, benzoin ethyl ether, benzyl, diacetyl or diphenyl disulfide, and di-β-naphthyl sulfide. For a dental adhesive composition, it is particularly preferable to use benzoyl peroxide, azobisisobutyronitrile, tributylborane, organic sulfinic acids or the salts thereof, or aromatic sulfinic acid (or the salt thereof)/diacyl peroxide/aromatic secondary or tertiary amine (or the salt thereof). Suitable examples of the aromatic sulfinic acids include benzenesulfinic acid, p-toluenesulfinic acid, (3-naphthalenesulfinic acid and styrenesulfinic acid. While alkali metal, alkali earth metal or ammonium ions may, for example, be used as the cation to form a salt with a sulfinic acid, it is preferable to use alkali or alkali earth metal ions in order to produce a high degree of storage stability and adhesive strength. Examples of such ions are Li⁺, Na⁺, K⁺, Mg²⁺, Ca²⁺ and Sr²⁺. Preferred examples of the aromatic amines include N,N-dimethylaniline, N,N-dimethyl-p-toluidine, N,N-diethanolaniline, N,N-diethanol-p-toluidine, N-methylaniline, and N-methyl-p-toluidine. It is also possible to use a salt of any such amine with an acid, e.g. hydrochloric, acetic or phosphoric acid. Preferred examples of the diacyl peroxides include benzoyl peroxide, m-toluoyl peroxide, 2,4-dichlorobenzoyl peroxide, octanoyl peroxide, lauroyl peroxide and succinic acid peroxide. It is particularly preferable to use benzoyl peroxide or m-tuluoyl peroxide. The curing agent may be used in the quantity of 0.01 to 20, or preferably 0.1 to 15, parts by weight for 100 parts by weight of the polymerizable monomer employed.

It is sometimes preferable for the adhesive composition of this invention to contain a volatile organic solvent having a boiling point not higher than 150°C at 760 Torr (101 kPa). The addition of any such solvent is particularly useful for an adhesive composition used as a primer for the tooth cavity to be filled with a dental filling material. After the composition has been applied, air or nitrogen is blown thereagainst to volatilize the solvent to form a vinyl compound film on the adherend surfaces. Examples of the suitable solvents include methanol, ethanol, acetone, methyl ethyl ketone, methyl acetate, ethyl acetate, dichloromethane, chloroform, ethyl ether, isopropyl ether and toluene. The quantity of the solvent should not exceed 300, or preferably 100, times as much by weight as that of all the polymerizable monomers employed. The use of the solvent in any larger quantity can result, upon volatilization, in the formation of too thin a film of the polymerizable monomer on the adherend surface, or a great reductgion in its adhesive strength.

The adhesive composition of this invention may further contain a conventionally known filler of the inorganic, organic or composite type to form a dental cement for luting or filling, a dental composite resin or a bone cement. The adhesive composition may contain up to 1,000 parts, or preferably 20 to 500 parts, by weight of the filler for 100 parts by weight of the polymerizable monomers. The filler improves the rheological properties of the composition, and the mechanical properties, adhesive strength and water resistance of the cured composition. Examples of the applicable inorganic fillers include natural minerals such as quartz, feldspar, pottery stone, wollastonite, mica, clay, kaolin and marble, ceramics such as silica, alumina, silicon nitride, boron carbide, boron nitride, and glass-ceramics containing lanthanum, glass such as soda glass, barium glass, strontium glass or borosilicate glass, and water-insoluble inorganic salts such as barium sulfate and calcium carbonate. The inorganic filler is usually treated with a silane coupling agent such as 6-methacryloyloxypropyltrimethoxysilane, vinyltrimethoxysilane, vinyltriethoxysilane, vinyltrichlorosilane, vinyltris(2-methoxyethoxy)silane, vinyltriacetoxysilane or 6-mercaptopropyltrimethoxysilane.

If an organic filler is preferred, it is possible to use any of various types of polymers, for example, polymethyl methacrylate, polyamide, polyester, polypeptide, polysulfone, polycarbonate, polystyrene, chloroprene rubber, nitrile rubber, styrene-butadiene rubber or polyvinyl acetate. It is also possible to use a composite filler comprising a silanated inorganic filler coated with a polymer.

One or more substances may be used as the filler. The filler may usually have a particle size of 100 microns or less, and its particles may be formless, spherical, lamellar or fibrous. If a polymer is used, it may be dissolved in the polymerizable monomer and the volatile organic solvent. The adhesive composition of this invention preferably contains an inorganic or composite filler to form a dental cement or composite resin, and an organic filler to form a bone cement.

In addition to the various constituents as hereinabove described, the adhesive composition of this invention may contain a polymerization inhibitor such as hydroquinone monomethyl ether (MEHQ) or an antioxidant such as 2,6-di-tert-butyl-p-cresol(BHT), an ultraviolet absorbing agent, a pigment or dye, phthalic acid diester or silicone oil if required to satisfy the various practical requirements for performance. These additives are employed in a small quantity up to a maximum of 10 parts, and usually up to five parts, by weight for 100 parts by weight of the polymerizable monomers.

A room temperature curing redox type initiator may often be used when the adhesive composition of this invention is used for dental, orthopedic or other medical purposes. In such a case, it is necessary to ensure the storage stability of the composition, and therefore, to choose a proper package form which keeps the oxidant and the reducing agent away from each other. It is, for example, possible to employ separate packages for (a) the vinyl compound and the oxidant and (b) the vinyl compound and the reducing agent, (a) the vinyl compound and the oxidant (or reducing agent) and (b) the volatile organic solvent and the reducing agent (or oxidant), (a) the vinyl compound and the oxidant (or reducing agent) and (b) the filler and the reducing agent (or oxidant), or (a) the vinyl compound, the filler and the oxidant and (b) the vinyl compound, the filler and the reducing agent. In an organic sulfinic acid (or salt thereof)/amine (or salt thereof)/peroxide system, which is a particularly suitable redox polymerization initiator for the adhesive composition of this invention, the sulfinic acid and the amine serve as a reducing agent, and the peroxide as an oxidant. In this case, it is possible to employ three separate packages by packing the sulfinic acid and the amine separately. When a photosensitizer is employed, it is necessary to keep the vinyl compound and the photosensitizer in a container shielded against light. If tributylborane or any other polymerization initiator initiating polymerization within a short time after contacting the vinyl compound is employed, it is necessary to pack the vinyl compound and the initiator separately from each other. The various constituents of the adhesive composition stored in separate packages as hereinabove described may be mixed together immediately prior to application.

The adhesive composition of this invention adheres excellently to any of the following materials, and maintains a high adhesive strength for a long time even in a wet condition:
(1) Hard tissues in a living body, such as teeth and bones;
(2) Metals, including not only base metals such as iron, nickel, chromium, cobalt, aluminum, copper, zinc and tin, or stainless steel, brass or other alloys thereof, but also noble metal alloys containing 50 to 90% of gold or platinum, which it has hitherto been difficult to bond by any known adhesive;
(3) ceramics, such as glass, porcelain, silica and alumina; and
(4) organic polymers, such as polymethyl methacrylate, polyester, polyamide, polyurethane, polycarbonate, polyvinyl chloride and polyethylene.

The adhesive composition of this invention can be used for various fields of application because of its high adhesive strength on various kinds of materials. The preferred examples of its applications will hereunder be set forth:

### (1) Dental Applications:

The composition can be used as a composite resin adhering to a tooth, as an adhesive for application to the cavity surface prior to the placement of the composite resin in the cavity, as an adehesive for bonding an inlay, onlay, crown or the like to a cavity or abutment tooth, as an adhesive for orthodontics, as an adhesive for holding a bridgework, post or the like, or as a pit and fissure sealant. The composite resin usually comprises a polymerizable monomer, a filler and a curing agent. The composite resin and the adhesive used for the placement thereof are usually supplied together in a combination to the dentist. The specific constituents of a particular adhesive composition are selected to suit its application as hereinbefore described. If the composition is used, for example, as the adhesive to be coated on the tooth prior to the placement of the composite resin, it may contain 2 to 40% by weight of the adhesive vinyl compound diluted with any other polymerizable monomer, such as Bis-GMA, HEMA or an aliphatic dimethacrylate, or an organic solvent such as ethanol, and further contain a room-temperature curing agent in accordance with the recipe shown in U.S. Patent No. 4,259,075 or 4,259,117. The adhesive composition which is used as a dental composite resin may comprise a customary filling material consisting of 20 to 40% by weight of Bis-GMA or other polymerizable monomer and 60 to 80% by weight of filler, and 2 to 40% by weight of an adhesive vinyl compound based on the total weight of the polymerizable monomer in accordance with the recipe shown in the U.S. patents hereinabove cited. The composition may be applied to a tooth by a customary method. As opposed to the compositions disclosed in U.S. Patents Nos. 4,259,075 and 4,259,117, however, the composition of this invention does not call for the acid etching of the tooth surface to provide a satisfactory adhesive strength.

The acid etching of the tooth should be avoided as far as possible, since it is likely to have an injurious effect on the dentin. When the composite resin is cured, it strongly adheres to the tooth, and does not require any mechanical retention such as an undercut. The adhesive composition used for bonding an inlay, onlay, crown, or the like to a tooth cavity or abutment tooth may, for example, comprise 60 to 98 parts by weight of methyl methacrylate, 2 to 40 parts by weight of adhesive vinyl monomer, and 50 to 150 parts by weight of polymethyl methacrylate. A slight excess of the adhesive may be applied to the adherend surface, and an inlay or the like brought into intimate contact therewith, whereby it is possible to effect the bonding of any such dental restorative appliance to the tooth which no conventional luting cement has hitherto been able to achieve.

### (2) Orthopedic Applications:

The adhesive composition of this invention can be used as a bone cement.

### (3) Industrial and Domestic Applications in General:

The adhesive composition of this invention is useful as an adhesive for transportation machinery, electrical apparatus, building materials, cans or ceramics, or for home use, since it provides a superior adhesive strength on metals, ceramics and organic polymers. It is also useful as a paint, a primer for a paint or a coating composition. It adheres surprisingly well to an adherend surface carrying oil, or even water. Its adhesive strength is markedly higher than that of any conventional polymerization curing type adhesive, such as cyanoacrylate, epoxy resin or second generation acrylic adhesives.

The invention will now be described more specifically with reference to a variety of examples which are merely illustrative. They consist of the following:
(1) Examples 1 to 20 - Examples of the preparation of the phosphate esters represented by formula 3, and examples of the adhesive compositions containing those compounds;
(2) Examples 21 to 26 - Examples of the preparation of the phosphate esters represented by formula 4, and examples of the adhesive compositions containing those compounds;
(3) Examples 27 to 36- Examples of the preparation of the phosphate esters represented by formula 5, and examples of the adhesive compositions containing those compounds;
(4) Examples 37 to 46- Examples of the preparation of the phosphate esters represented by formula 6, and examples of the adhesive compositions containing those compounds;
(5) Examples 47 to 53 - Examples of the adhesive composition of this invention used for dental application; and
(6) Examples 54 to 61 - Adhesive strength of the adhesive composition of this invention on metals, ceramics and organic polymers.

### Example 1

A 500 ccthree-neck flask was charged with 140 g (1.6 mols) of methacrytic acid, 190 g (1.6 mols) of 1,6-hexanediol, 15 g of p-toluenesulfonic acid and 0.6 g of 2,2'-methylene-bis(4-ethyl-6-tert-butylphenol). They were heated to 90°C at a pressure of 100 to 150 mm Hg (13 to 20 kPa). The reaction was continued for several hours, while oxygen was blown into the flask, until water disappeared. When water had disappeared, the reactant was cooled to ordinary room temperature, transferred into a separatory funnel, and washed with a 5% aqueous solution of sodium carbonate until the aqueous solution showed an alkaline reaction. The reactant was then washed with 300 cc of water five times. After the reactant had been dehydrated with anhydrous sodium sulfate, it was heated with 30 mg of MEHQ to 80°C, whereby the remaining water was removed by distillation under reduced pressure and 198 g of a monoester and diester mixture were obtained. The analysis of the mixture by HLC indicated that it contained 75 mol% of monoester, and not more than 0.5% by weight of diol.

A solution containing 55 g (0.36 mol) of phosphorus oxychloride in 100 cc of ethyl etherwas placed in a one-liter reactor, and cooled to -40°C. A solution containing 81 g of a previously synthesized methacrylate mixture containing 0.3 mol of monoester, and 37 g (0.36 mol) of triethylamine was placed in a 300 cc dropping funnel connected to the reactor. The latter solution was dropped slowly, while the phosphorus oxychloride solution was strongly, and dry N₂ gas was blown into the reactor. The reactant was kept at a temperature of -30°C for three hours after the dropping of the solution from the dropping funnel had been finished, and then, its temperature was raised to 0°C. 30 g of water were placed in the dropping funnel, and dropped under stirring. A solution containing 72.9 g (0.72 mol) of triethylamine in 100 cc of ethyl ether was, then, dropped. The reactant was held at 0°C for 10 hours thereafter under stirring. After the precipitated triethylamine hydrochloride had been separated by a glass filter, 10 mg of MEHQ were added into the filtrate, and the ethyl ether was removed by vacuum distillation at 40°C, whereby a nonvolatile liquid residue was obtained. The liquid was dispersed in 200 cc of water, and while the dispersion was cooled with ice, and stirred strongly, 65 g (0.6 mol) of sodium carbonate were added little by little into the dispersion to neutralize it. When the dispersion had ceased to bubble, it was transferred into a separatory funnel, and extracted twice with 100 cc of ethyl ether and four times with 100 cc of chloroform. The aqueous solution was cooled with ice, and 6N HCI was added thereinto to acidify it, and the separated oily matter was extracted with ethyl ether three times. All the extracts were combined, and dried with anhydrous sodium sulfate. 10 mg of MEHQ were added, and the solvent was removed by distillation at a temperature not exceeding 40°C, whereby 67 g of a colorless, transparent liquid were obtained.

The NMR of a 10% CDCI₃ solution of the liquid was examined at 90 MHz at ordinary room temperature. There were observed an ethylenic proton signal at 6 = 6.05 and 5.5, a methyl proton signal at δ = 1.9, a multifold hexamethylenic proton signal at 6 = 3.8 to 4.2 and 1.2 to 1.8, and an acid proton signal of phosphoric acid in the vicinity of δ = 9.7. The elementary analysis of the liquid indicated that it contained 44.5% C, 7.8% H, 36.5% O and 11.2% P, while they were theoretically 45.1%, 7.2%, 36.1% and 11.6%, respectively, and showed that it was a compound known as 6-methacryloyloxyhexyl dihydrogen phosphate. The analysis of the compound by high-speed liquid chromatography (HCL) employing a Unisil Q C₁₈ column indicated that it had a purity of 97 to 98%.

### Example 2

A diol monoester and diester mixture was prepared by repeating the procedures of Example 1, except for the use of 170 g (1.6 mols) of 1,5-pentanediol instead of 1,6-hexanediol. The quantity of the monoester in the mixture was determined by HLC, and that quantity of the mixture which contained 0.3 mol of monoester was subjected to phosphate esterification. The procedures of Example 1 were repeated for the separation and purification of the reaction product to yield 61 g of an acidic nonvolatile liquid. The NMR of the compound thus obtained, and its elementary analysis [C: 42.6% (theoretically 42.9%), H: 7.1% (6.8%), 0: 38.5% (38.1%), and P: 11.8% (12.3%)] indicated that it was 5-methacryloyloxypentyl dihydrogen phosphate. The analysis of the compound by HLC indicated that it had a purity of 96 to 98%.

### Example 3

A 500 cc flask was charged with 140 g (1.9 mols) of acrylic acid, 190 g (1.6 mols) of 1,6-hexanediol,15 g of p-toluenesulfonic acid, and 0.6 g of 2,2'-methylenebis(4-ethyl-6-tert-butylphenol). They were heated to 80°C at a pressure of 100 to 200 mm Hg, while oxygen was being blown into the flask through a capillary, whereby water was removed by distillation. The reaction product was washed with alkaline water and water, and dehydrated to yield 175 g of an acrylic acid monoester and diester mixture. The analysis of the product by HLC indicated that it contained 68 mol% of monoester. The procedures of Example 1 were repeated for reacting 83.6 g of the mixture with 55 g (0.36 mol) of phosphorus oxychloride, and separating phosphate monoester to yield 62 g of a colorless transparent nonvolatile liquid. The NMR of the compound thus obtained, and its elementary analysis [C: 42.7% (theoretically 42.9%), H: 7.1% (6.8%), 0: 38.3% (38.1%) and P: 11.9% (12.3%)] indicated that it was 6-acryloyloxyhexyl dihydrogen phosphate. The analysis of the compound by HLC indicated that it had a purity of 95 to 97%.

### Example 4

A diol monoester and diester mixture was prepared by repeating the procedures of Example 3, except for the use of 170 g (1.6 mols) of 1,5-pentanediol instead of 1,6-hexanediol. After the monoester content of the mixture had been examined by HLC, that quantity of the mixture which contained 0.3 mol of monoester was subjected to phosphate esterification. The procedures of Example 1 were repeated for the separation and purification of the reaction product to yield 48 g of an acidic nonvolatile liquid. The NMR of the compound thus obtained, and its elementary analysis [C: 40.5% (theoretically 40.3%), H: 6.5% (6.4%), 0: 40.0% (40.3%) and P: 13.0% (13.0%)] indicated that it was 5-acryloyloxypentyl dihydrogen phosphate. It showed a purity of 95 to 97% upon analysis by HLC.

### Example 5

A 500 cc three-neck flask was charged with 140 g (1.6 mols) of methacrylic acid, 230 g (1.3 mols) of 1,10- decanediol, 15 g of p-toluenesulfonic acid, and 0.6 g of 2,2'-methylenebis(4-ethyl-6-tert-butylphenol). They were heated to 80°C to form a uniform solution. The flask pressure was reduced to a level of 100 to 150 mm Hg, and while oxygen was being blown into the flask to stir the solution, it was subjected to phosphate esterification at 90°C, and water was removed by distillation. When water had ceased to appear, the reaction was discontinued, the reaction product was cooled to ordinary room temperature, and 300 cc of n-hexane were added thereinto to dilute it. The solid precipitate was removed by filtration, and the filtrate was washed with an aqueous solution of sodium carbonate until the aqueous solution showed an alkaline reaction. After the reactant had been further washed with water, it was diluted with one liter of n-hexane, and left at 5°C with anhydrous sodium sulfate. After one day, the unreacted diol precipitate was removed by filtration, 40 mg of MEHQ were added into the filtrate, and n-hexane was removed by vacuum distillation at a temperature not exceeding 80°C, whereby 268 g of a diol monoester and diester mixture were obtained. The analysis of the mixture by HLC indicated a monoester content of 65 mol%, and only a trace of unreacted diol.

A solution containing 55 g (0.36 mol) of phosphorus oxychloride in 100 cc of ethyl ether was placed in a one-liter reactor, and cooled to -40°C. A solution containing 123 g of a separately synthesized ester mixture containing 0.3 mol of monoester, and 37 g (0.36 mol) of triethylamine in 120 cc of ethyl ether was placed in a 300 cc dropping funnel, and the funnel was connected to the reactor. The latter solution was dropped slowly, while the phosphorus oxychloride soluton was being stirred strongly, and dry N₂ gas was being blown into the reactor. The reactant was held at a temperature of -30°C for three hours after the dropping of the solution from the dropping funnel, and its temperature was raised to 0°C. 30 g of water were placed in the dropping funnel, and dropped under stirring. A solution containing 72.9 g (0.72 mol) of triethylamine in 100 cc of ethyl ether was, then, dropped. The reactant was held at 0°C for 10 hours thereafter, while it was being stirred slowly. After the precipitated triethylamine hydrochloride had been removed by a glass filter, the filtrate was washed with water repeatedly, dehydrated and dried. After 20 mg of MEHQ had been added, the ethyl ether was removed by vacuum distillation at 40°C, whereby a liquid residue was obtained. The liquid was washed with n-hexane repeatedly for the removal of the diol and phosphate diesters by extraction, and the n-hexane was removed from the phosphate monoester phase by vacuum distillation, whereby 73 g of a liquid compound were obtained.

The NMR at 90 MHz of a 10% CDCI₃ solution of the compound was examined at ordinary room temperature. There were found an ethylenic proton signal at 6 = 6.05 and 5.5, a methyl proton signal at 6 = 1.9, a multifold decamethylenic proton signal at 6 = 3.8 to 4.2 and 1.2 to 1.8, and an acidic proton signal of phosphoric acid in the vicinity of 6 = 9.7. The elementary analysis of the compound indicated that it contained 52.9% C, 8.7% H, 29.2% 0 and 9.2% P, while they were theoretically 52.2%, 8.4%, 29.8% and 9.6%, respectively, and showed that it was 10-methacryloyloxydecyl dihydrogen phosphate. The analysis of the compound by HLC employing a Uhisit Q C₁₈ column indicated a purity of 92 to 95%.

### Example 6

A diol monoester and diester mixture was prepared by repeating the procedures of Example 5, except for the use of 260 g (1.3 mols) of 1,12-dodecanediol instead of 1,10-decanediol. After the monoester content of the mixture had been examined by HLC, that quantity of the mixture which contained 0.3 mol of monoester was subjected to phosphate esterification. The procedures of Example 1 were repeated for the separation and purification of the reaction product to yield 75 g of a liquid compound. The NMR of the compound, and its elementary analysis [C: 55.3% (theoretically 54.9%), H: 9.2% (8.9%), O: 27.0% (27.4%) and P: 8.5% (8.8%)] indicated that it was 12-methacryloyoxydodecyl dihydrogen phosphate. It showed a purity of 92 to 95% upon analysis by HLC.

### Example 7

A 500 cc flask was charged with 115 g (1.6 mols) of acrylic acid, 230 g (1.3 mols) of 1,10-decanediol, 15 g of p-toluenesulfonic acid, and 0.6 g of 2,2'-methylenebis-(4-ethyl-6-tert-butylphenol). They were heated to 80°C at a pressure of 100 to 200 mm Hg, (13 to 27 kPa) while oxygen was blown into the flask through a capillary, and water was removed by distillation. The procedures of Example 5 were, then, repeated for dilution with n-hexane, filtration, washing with an alkali solution and water, dehydration, dilution with n-hexane, filtration and solvent removal by distillation to yield 223 g of a diol monoester and diester mixture. The analysis of the mixture by HLC indicated a monoester content of 66 mol%. The procedures of Example 5 were further repeated for reacting 114 g of the mixture with 55 g (0.36 mol) of phosphorus oxychloride, and separating phosphate monoestger to yield 62 g of a colorless transparent nonvolatile liquid. The NMR of the ocmpound thus obtained, and its elemental analysis [C: 51.0% (theoretically 50.6%), H: 8.4% (8.2%), O: 30.8% (31.1%) and P: 9.0% (9.6%)] indicated that it was 10-acryloyloxydecyl dihydrogen phosphate. It showed a purity of 91 to 95% upon analysis by HLC.

### Example 8

A diol monoester and diester mixture was prepared by repeating the procedures of Example 7, except for the use of 260 g (1.3 mols) of 1,12-dodecanedio) instead of 1,1 O-decanediol. After the monoester content of the mixture had been examined by HLC, that quantity of the mixture which contained 0.3 mol of monoester was subjected to phosphate esterification. The procedures of Example 5 were repeated for the separation and purification of the reaction product to yield 79 g of a liquid compound. The NMR of the compound, and its elementary analysis [C: 53.9% (theoretically 53.6%), H: 9.0% (8.7%), O: 28.1% (28.5%) and P: 9.0% (9.2%)] indicated that it was 12-acryloyloxydodecyl dihydrogen phosphate. It showed a purity of 92 to 96% upon analysis by HLC.

### Examples 9 to 18, and

### Comparative Examples 1 and 2

The adhesive compositions containing the adhesive monomers obtained in Examples 1 to 8 were prepared in accordance with the recipe hereinafter shown, and tested for the water resistance of their adhesive strength on metal.

Components and Proportions:

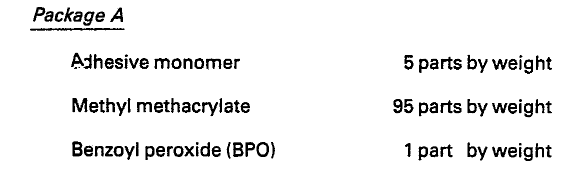

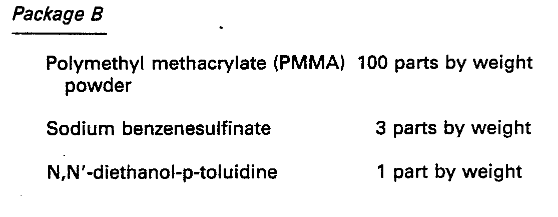

The package A consisted of a uniform solution, and the package B was a uniform dispersion of the powders of sodium benzenesulfinate and amine in the PMMA powder.

A 10 mm square by 3 mm thick plate of a Ni-Cr alloy containing 92.7% of nickel was polished with #1000 abrasive paper, and washed ultrasonically with water and methyl ethyl ketone. An adhesive cellophane tape having a hole with a diameter of 5 mm was bonded to the plate so that the hole might be aligned with the center of the polished plate surface. An equal number of stainless steel bars having a diameter of 7 mm and a length of 35 mm were prepared, and one end surface of each bar was polished and washed as each alloy plate had been. The components in Package A were mixed with an equal weight of the components in Package B to prepare an adhesive composition. The adhesive was applied by a brush to the alloy plate exposed through the hole of the cellophane tape, and the polished end of the bar, and the bar and the plate were bonded to each other to form a butt joint. Twenty samples, or bar and plate combinations were prepared by employing one adhesive composition, and immersed in water at 37°C an hour after they had been bonded. Ten samples were removed from water after 24 hours, and the other 10 samples after 10 days. The adhesive bond on each sample was tested for tensile strength by an Instron tensile tester. Table 1 shows an average adhesive strength obtained by each adhesive composition in each lot of 10 samples. The adhesives developed cohesive failure at a tensile strength of 400 kg/cm² (39 MPa) or above, and adhesive failure mainly on the Ni-Cr alloy surface at a lower tensile strength.

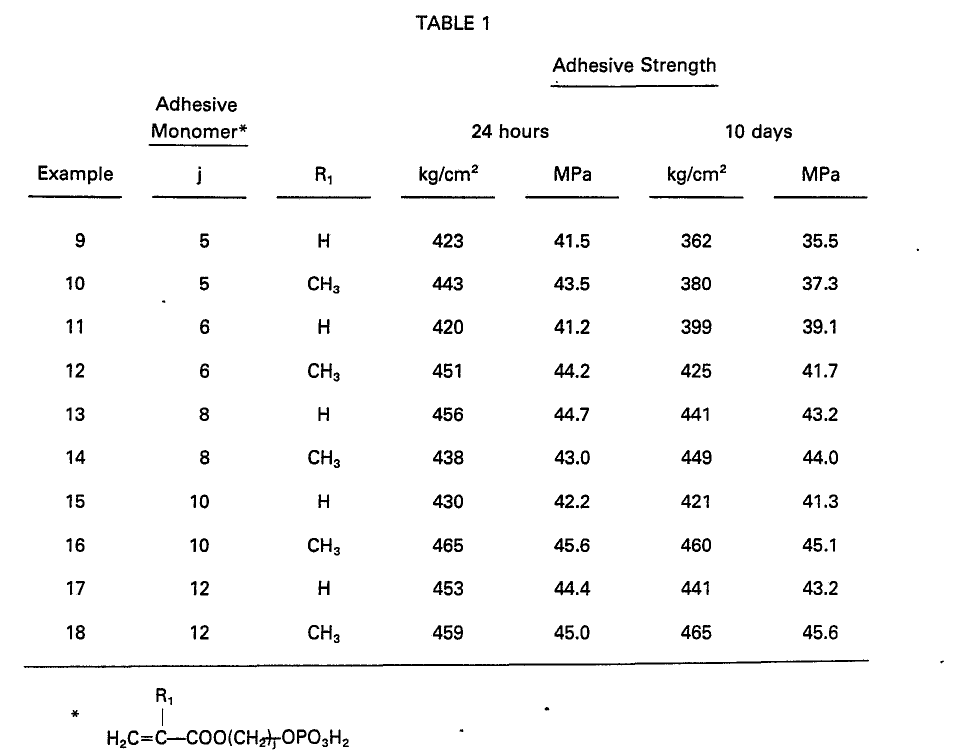

The adhesive compositions of this invention showed an adhesive strength higher than 400 kg/cm² (39 MPa) after 24 hours of immersion in water, and did not undergo any great reduction in adhesive strength after 10 days, either.

For comparison purposes, similar tests were conducted on a composition containing a conventionally known phosphate ester monomer, for example, methacryloyloxyethyl dihydrogen phosphate (Comparative Example 1), and on a composition not containing any adhesive monomer (Comparative Example 2). The results are shown in Table 2. Immersion in water brought about.a heavy reduction in adhesive strength.

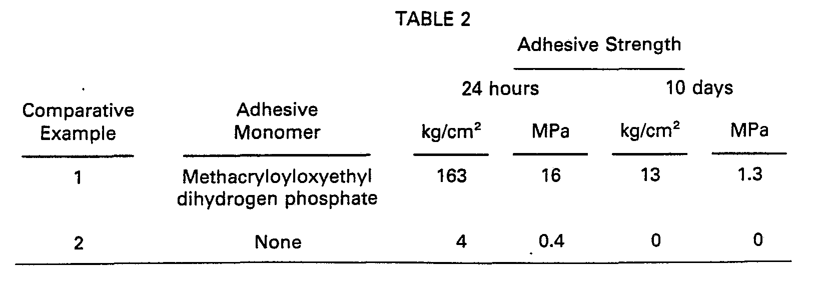

### Example 19

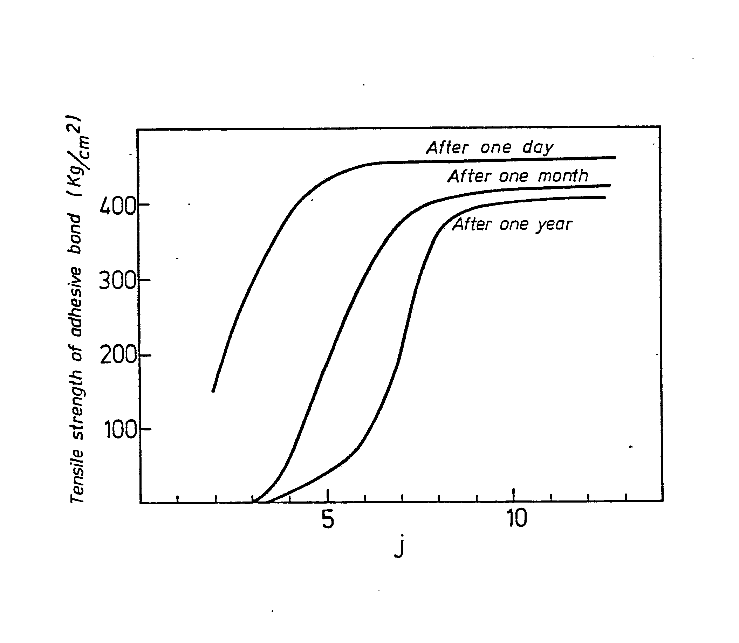

Nine adhesive compositions each containing a phosphate ester of the general formula 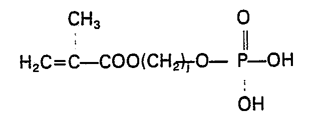 in which is 2, 3, 4, 5, 6, 7, 8, 10 or 12, were prepared, and tested for adhesive strength in water. Each of these thermosetting adhesive compositions contained four parts by weight of a phosphate ester of the above formula, one part by weight of BPO, 95 parts by weight of methyl methacrylate and 100 parts by weight of PMMA powder. Two round nickel bars having a diameter of 7 mm were joined end-to-end by using each adhesive composition. The bars were heated at 80°C for three hours, whereby the adhesive composition was cured. Each sample was immersed in water at 25°C, and tested for adhesive strength after one day, month or year. The relationship between and the wet adhesive strength of the composition is shown in the single figure of the drawing, which indicates that an improved adhesive strength in water was obtained with an increase in the value of j, particularly when it was 5 or more, and more particularly when it was 8 or more.

### Example 20 and Comparative Example 3

Two-pack adhesive compositions were prepared in accordance with the following recipe by employing the adhesive monomers shown in Table 3, and tested for adhesive strength by bonding a commercially available dental composite resin to the dentin of a human tooth:

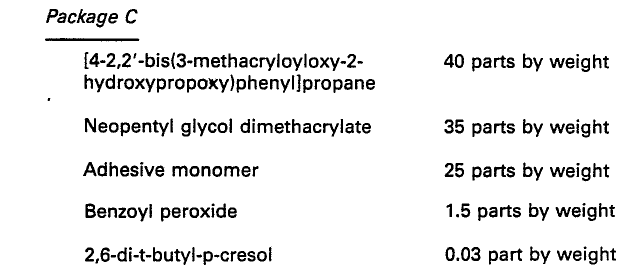

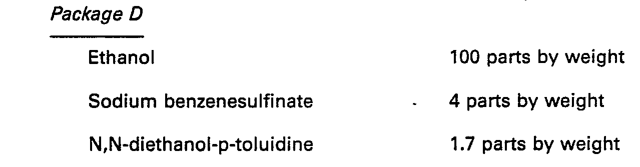

Human molars were cut in the shape of a square pillar to have the dentin exposed on an adherend surface, and square ivory bars measuring 10 mm square by 30 mm long were also prepared. They were kept in cold water until immediately prior to use. Immediately prior to use, water was wiped off the adherend dentin surface of each molar, and the dentin surface was etched for a minute with a 40% aqueous solution of orthophosphoric acid. The molar was washed carefully with flowing water, and after clean air or nitrogen had been blown against the molar to volatilize water, a strip of aluminum was wound about the molar. Water was also wiped off the adherend surface of each ivory bar. A mixed solution prepared by mixing equal volumes of the liquids C and D was coated on the molars and the ivory bars, and clean air or nitrogen was blown against them to volatilize ethanol. A commercially available dental composite resin Clearfil F (trademark of KURARAY CO., LTD,, Japan) was kneaded and it was sandwiched between the adherend surfaces of the molars and the ivory bars to form test samples. They were immersed in water at 37°C after 30 minutes, and examined after one day for adhesive strength by an Instron tensile tester at a crosshead speed of 2 mm/min. The results are shown in Table 3. Seven human teeth were used for testing one adhesive composition. Table 3 shows an average adhesive strength.

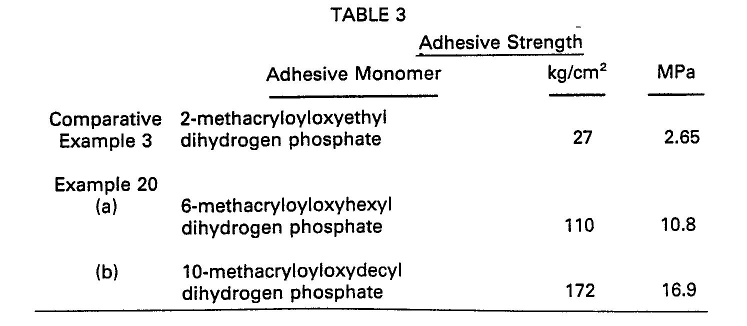

### Example 21

A 500 cc three-neck flask was charged with 129 g (1.5 mols) of methacrylic acid, 216 g (1.5 mols) of 1,4-cyclohexane dimethanol, 17 g of p-toluenesulfonic acid, and 0.7 g of 2,2'-methylenebis(4-ethyl-6-tertbutylphenol). They were dissolved in a bath of water at 70°C to form a uniform solution. The bath temperature was raised to 85°C, and while oxygen was being blown into the flask at a pressure of 100 mm Hg (13.3 kPa), the water produced was removed by distillation.

When water had ceased to appear, the solution was returned to normal atmospheric pressure, diluted with 500 cc of benzene and 500 cc of n-hexane, and washed with an aqueous solution of sodium carbonate, and methacrylic acid and p-toluenesulfonic acid were removed by extraction. The solution was washed with neutral water repeatedly, and after it had been ascertained that the solution was neutral, it was dried with anhydrous sodium sulfate, 100 mg of MEHQ were added, and the solvent was removed by vacuum distillation at a temperature not exceeding 80°C. There was obtained a monoester and diester mixture weighing 242 g, and found to contain 72 mol% of monoester as a result of analysis by HLC. The mixture did not contain more than 0.1% of unreacted diol.

A solution containing 55 g (0.36 mol) of phosphorus oxychloride in 100 cc of ethyl ether was placed in a one-liter reactor, and cooled to -40°C. A solution containing 96.5 g (0.3 mol) of a separately synthesized methacrylate mixture, and 37 g (0.36 mol) of triethylamine in 100 cc of ethyl ether was placed in a 300 cc dropping funnel, and the funnel was connected to the reactor. The latter solution was dropped slowly, while the phosphorus oxychloride solution was being stirred strongly, and dry N₂ gas was being blown into the reactor. The reactant was held at -30°C for three hours, and then, its temperature was raised to 0°C. Then, 30 g of water were placed in the dropping funnel, and dropped into the reactor under stirring. A solution containing 72.9 g (0.72 mol) of triethylamine in 100 cc of ethyl ether was, then, dropped. The reactant was held at 0°C for 10 hours thereafter under slow stirring. After the precipitated triethylamine hydrochloride had been removed by a glass filter, 10 mg of MEHQ were added into the filtrate, and ethyl ether was removed by vacuum distillation at 40°C, whereupon a nonvolatile liquid was obtained as a residue. The liquid was dispersed in 200 cc of water, and while the solution was cooled with ice, and stirred strongly, 65 g (0.6 mol) of sodium carbonate were added into the solution little by little to neutralize it. When the solution had ceased to bubble, it was transferred into a separatory funnel, and washed four times by extraction with 100 cc of chloroform. Then, 6N HCI was added into the solution to acidify it, while itwas cooled with ice, and the separated oily matter was extracted three times with chloroform. After all the extracts had been combined, and dried with anhydrous sodium sulfate, 10 mg of MEHQ were added, and the solvent was removed by distillation at a temperature not exceeding 40°C, whereupon there was obtained a colorless liquid weighing 70 g.

The NMR at 90 MHz of a 10% CDCI₃ solution of the liquid was examined at-ordinary room temperature. There were found an ethylenic proton signal at 6 = 6.05 and 5.5, a methyl proton signal at 6 = 1.9, two methylenic proton signals adjacent to oxygen atoms at δ = 3.6 to 4.1, methylene and methyl proton signals on the cyclohexane ring at 5 = 0.7 to 1.9, and an acidic proton signal of phosphoric acid at δ = 9.4 to 9.8. The elementary analysis of the compound indicated 48.9% C (theoretically 49.3%),7.6% H (7.2%), 33.1 % 0 (32.9%) and 10.4% P (10.6%), and showed that it was 4-(methacryloyloxymethyl)cyclohexylmethyl dihydrogen phosphate. The compound showed a purity of 96 to 98% upon analysis by HLC employing a Unisil Q C₁₈ column.

### Example 22

A 500 cc flask was charged with 140 g (1.9 mols) of acrylic acid, 230 g (1.6 mols) of 1,4-cyclohexanedimethanol, 15 g of p-toluenesulfonic acid, and 0.6 g of 2,2'-methylenebis(4-ethyl-6-tertbutylphenol). They were heated at 80°C at a pressure of 100 to 200 mm Hg (13 to 27 kPa), while oxygen was blown into the flask through a capillary, and water was removed by distillation. The procedures of Example 21 were, then, repeated to yield 251 g of a monoester and diester mixture. The mixture showed a monoester content of 72 mol% upon analysis by HLC. The procedures of Example 21 were repeated for reacting 89 g of the mixture with 55 g (0.36 mol) of phosphorus oxychloride, and separating phosphate monoester to yield 59 g of a light yellow, transparent liquid.

The NMR at 90 MHz of a 10% CDCI₃ solution of the liquid was examined. There were found three ethylenic proton signals at 5 = 5.7, 6.1 and 6.3, two methylenic proton signals adjacent to oxygen atoms at δ = 3.6 to 4.1, methylene and methine proton signals on the cyclohexane ring at δ = 0.7 to 1.9, and a phosphoric acid proton signal at 6 = 9.1 to 9.5. The elementary analysis of the compound indicated 47.9% C (theoretically 47.5%), 7.1% H (6.9%), 34.1% O (34.5%) and 11.0% P (11.1%), and showed that it was 4-(acryloyloxymethyl)cyclohexylmethyl dihydrogen phosphate. It showed a purity of 95 to 97% upon analysis by HLC.

### Examples 23 and 24

Adhesive compositions containing the compounds obtained in Examples 21 and 22 as an adhesive monomer (Examples 23 and 24), and an adhesive composition containing 2-methacryloyloxyethyl dihydrogen phosphate (Comparative Example 1) were prepared in accordance with the following recipe, and tested for adhesive strength on metal after immersion in water.

### Components and Proportions:

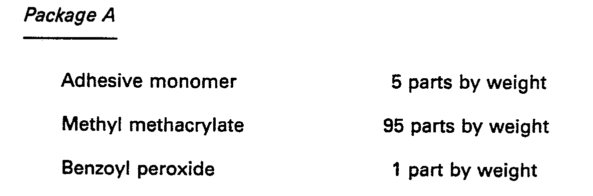

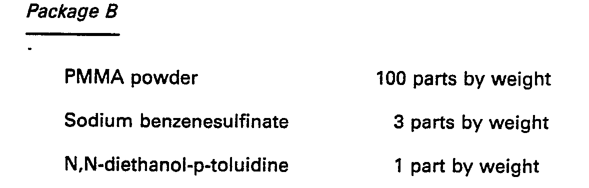

Package A consisted of a uniform solution, and Package B was a uniform dispersion of the sodium benzenesulfinate and amine powders in the PMMA powder.

A 10 mm square by 3 mm thick plate of a Ni-Cr alloy containing 92.7% of nickel was polished with #1000 abrasive paper, and washed ultrasonically with water and methyl ethyl ketone. An adhesive cellophane tape having a hole with a diameter of 5 mm was bonded to the plate so that the hole might be aligned with center of the polished plate surface. An equal number of stainless steel bars having a diameter of 7 mm and a length of 35 mm were prepared, and one end surface of each bar was polished and washed as the alloy plates had been. An adhesive composition was prepared by mixing equal weights of the components in Packages A and B, and applied by a brush to the alloy plate exposed through the hole in the cellophane tape, and the stainless steel bar. The alloy plate and the bar were bonded to each other to form a butt joint. Twenty samples, or bonded bar and plate combinations were prepared by employing each adhesive composition, and immersed in water at 37°C after an hour. Ten samples were removed from water after 24 hours, and the remaining 10 samples after 10 days. They were examined for adhesive strength by an Instron tensile tester. Table 4 shows the average obtained from the test results. The adhesive compositions developed cohesive failure mainly on the Ni-Cr alloy surface at a lower tensile strength.

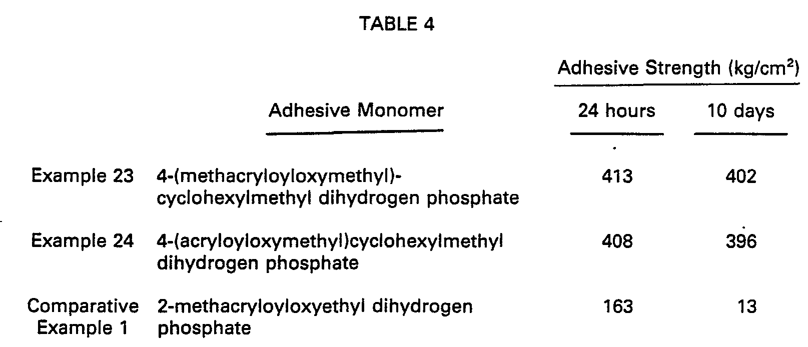

As is obvious from Table 4, the adhesive compositions of this invention showed a very high initial adhesive strength exceeding 400 kg/cm² (39.2 MPa), and did not undergo any appreciable reduction in adhesive strength after 10 days of immersion in water. On the other hand, the conventional adhesive composition containing 2-methacryloyloxyethyl dihydrogen phosphate showed a great reduction in adhesive strength as a result of immersion in water.

### Examples 25 and 26

Two-pack adhesive compositions were prepared in accordance with the following recipe by employing the adhesive monomers shown in Table 5, applied for bonding a commercially available dental composite resin to the dentin of a human tooth, and tested for adhesive strength:

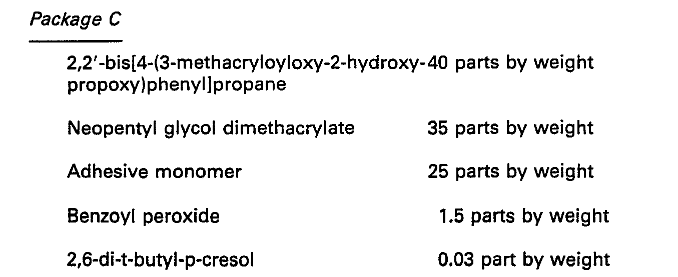

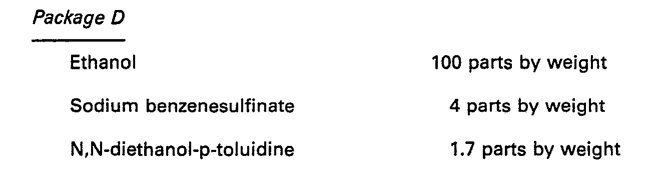

Human molars were cut in the shape of a square pillar to have an exposed adherend dentin surface and square ivory bars measuring 10 mm square by 30 mm long were also prepared. They were kept in cold water until immediately prior to use. Immediately prior to use, water was wiped off the adherend ivory surface of each molar, and the dentin surface was etched for a minute with a 40% w/w aqueous solution of orthophosphoric acid. The molar was, then, washed carefully with flowing water, and after clean air or nitrogen had been blown against the molar to volatilize water, a strip of aluminum was wound about the molar. Water was also wiped off the adherend surfaces of the ivory bars. An adhesive composition prepared by mixing equal volumes of the liquids C and D was applied to the molar and the ivory bar, and clean air or nitrogen was blown against them to dry them. A commercially available dental composite resin Clearfil F (trademark of KURARAY CO., LTD., Japan) was kneaded and it was sandwiched between the adherend surfaces of the molar and the ivory bar. The test samples thus prepared were immersed in water at 37°C after 30 minutes, and examined after one day of immersion for adhesive strength'by an Instron tensile tester at a crosshead speed of 2 mm/min. The results are shown in Table 5. Seven human molars were employed for testing each adhesive composition. Table 5 shows the average of the results obtained.

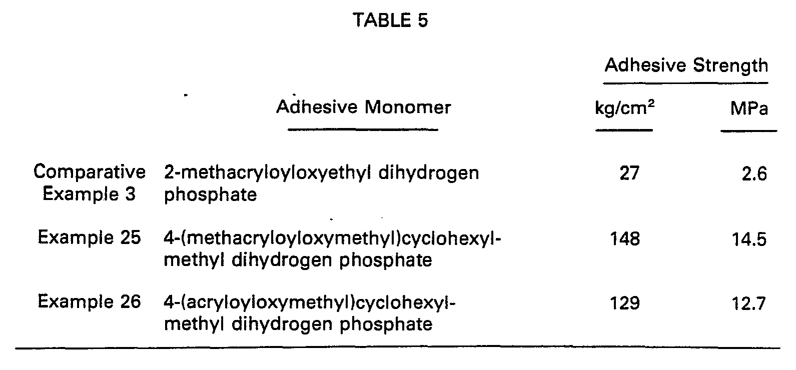

### Example 27

A one-liter three-neck flask was charged with 250 g (2.9 mols) of methacrylic acid, 480 g (2.4 mols) of 1,4-bis(2-hydroxyethoxy)benzene, 35 g of p-toluenesulfonic acid, and 0.6 g of MEHQ. They were heated to 80°C until they were dissolved to form a uniform solution. The flask pressure was reduced to a level of 20 to 100 mm Hg (0.27 to 1.3 kPa) by an aspirator, and while oxygen was being blown into the flask, the solution was heated at a temperature of 85°C to 90°C, and water was removed by distillation. When water ceased to appear after four hours, the reaction was discontinued, and while the reactant solution was hot, it was poured into 1.5 liters of toluene. The unreacted diol precipitate was removed by a #4 filter. The filtrate was washed twice with 500 cc of a 5% aqueous solution of Na₂C0₃ and three times with neutral water. After it had been dried with anhydrous sodium sulfate, 0.1 g of MEHQ was added, and toluene was removed by vacuum distillation at a temperature not exceeding 80°C. There was obtained a monoester and diester mixture weighing 440 g. The analysis of the mixture by HLC indicated a monoester content of 65% by weight, and only a trace of unreacted diol.

A solution containing 55.2 g (0.36 mol) of phosphorus oxychloride in 150 cc of tetrahydrofuran was placed in a one-liter reactor, and cooled to -50°C. A solution containing 123 g of the above synthesized monoester and diester mixture containing 0.3 mol of monoester, and 33.4 g (0.33 mol) of triethylamine in 150 cc of tetrahydrofuran was placed in a 500 cc dropping funnel, and the funnel was connected to the reactor. The monoester solution was dropped slowly, while dry N₂ gas was being blown to stir the phosphorus oxychloride solution strongly, and the internal temperature of the reactor was kept at a level of -45°C to -50°C. It was also kept at -45°C for an hour after the dropping operation had been finished, and raised then to 0°C. A solution containing 30 ml of water and 75.9 g of triethylamine in 100 cc of tetrahydrofuran was dropped slowly. The reactant solution was then cooled with ice and stirred continuously for 15 hours, whereby the P-Cl bonds were hydrolyzed. The precipitated triethylammonium chloride was removed by a #4 glass filter. After 30 mg of MEHQ had been added to the filtrate, tetrahydrofuran was removed by vacuum distillation at a temperature not exceeding 30°C. The residue thereby obtained was poured into 400 cc of a 0.3 N aqueous solution of hydrochloric acid. The solidified residue was collected by filtration, washed with 500 cc of 0.3 N aqueous solution of hydrochloric acid, and dewatered on the filter. The residue was, then, washed with toluene several times for the removal of any matter soluble in toluene, and vacuum dried at ordinary room temperature. There was obtained a white solid crude phosphate monoester weighing 74 g. The monoester was dissolved in 300 cc of tetrahydrofuran, and after the insoluble matter had been removed by filtration, it was recrystallized with a mixed solvent containing toluene and n-hexane in a ratio by volume of 1:1 to yield 69 g of crystals.

The crystals had a melting point of 108.5°C to 109.5°C. The NMR at 90 MHz of a dₑ-acetone solution of the crystals was examined at ordinary room temperature. There were found an ethylenic proton signal at 6 = 6.0₅ and 5.5₅, multifold signals of eight methylenic protons at 6 = 4.0 to 4.5, a methyl proton signal at 5 = 1.8₅, four proton signals of phenylene at δ = 6.9, and a phosphoric acid proton signal at δ = 8.3. The elemental analysis of the compound indicated 48.1% C (theoretically 48.6%) and 9.1% P (8.9%), and showed that it was a compound of the formula:

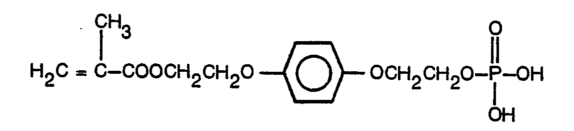

### Example 28

A diol monoester and diester mixture was prepared by repeating the procedures of Example 27, except for the use of 216 g (3.0 mols) of acrylic acid instead of methacrylic acid. After the monoester content of the mixture had been examined by HLC, that quantity of the mixture which contained 0.3 mol of monoester was subjected to phosphate esterification. The procedures of Example 27 were repeated for separating the reaction product to yield 65 g of a white solid compound. The NMR of the compound, and its elemental analysis [C: 46.5% (theoretically 47.0%), and P: 9.1% (9.3%)] indicated that it was a compound of the formula: 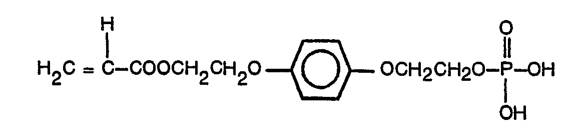 It showed a purity of at least 95% upon analysis by HLC.

### Examples 29 to 32

The procedures of Examples 27 and 28 were followed to synthesize four compounds as shown in Table 6 by employing methacrylic or acrylic acid, and 1,4-bis(3-hydroxypropoxy)benzene or 1,4-bis(4-hydroxybutoxy)benzene as diol. The structure of each compound was determined by NMR and elementary analysis, and its purity by HLC. As regards the results of elementary analysis, the values of C, H and P alone are shown in Table 6. All the compounds showed a purity of at least 95%.

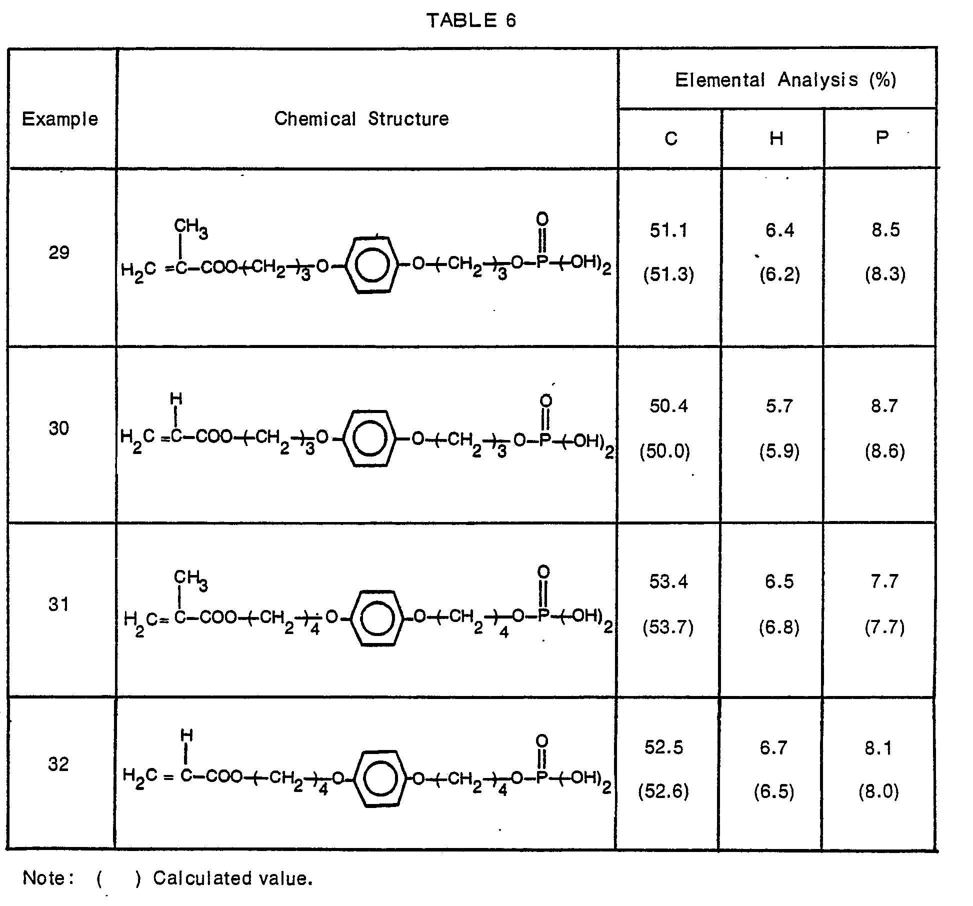

### Examples 33 and 34

Three adhesive compositions containing the compounds obtained in Examples 27 and 28 (Examples 33 and 34) and 2-methacryloyloxyethyl dihydrogen phosphate (Comparative Example 1) as the adhesive monomer were prepared in accordance with the following recipe, and tested for adhesive strength on metal after immersion in water:

### Components and Proportions:

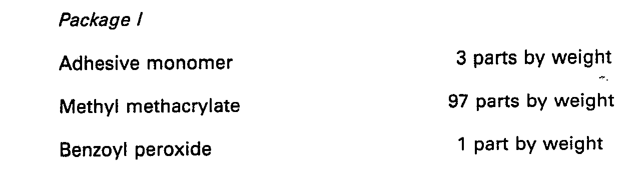

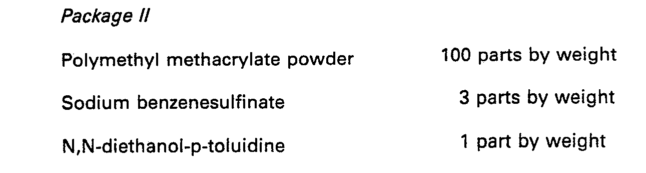

Package II was a uniform mixture of the PMMA powder in the benzenesulfinate and amine powders.

A 10 mm square by 3 mm thick plate of a Ni-Cr alloy containing 92.7% of nickel was polished with #1000 abrasive paper, and washed ultrasonically with water and methyl ethyl ketone. An adhesive cellophane tape having a hole with a diameter of 5 mm was bonded to the plate so that the hole might be aligned with the center of the polished plate surface. An equal number of stainless steel bars having a diameter of 7 mm and a length of 35 mm were prepared, and one end surface of each bar was polished and washed as the alloy plates had been. A mixture containing equal weights of the components in Packages I and II was applied by a brush to the alloy plate exposed through the hole in the cellophane tape, and the stainless steel bar, and the plate and the bar were joined to each other by forming a butt joint. Twenty samples, or bonded plate and bar combinations were prepared by employing each adhesive composition. They were immersed in water at 37°C one hour after the bar and the plate had been bonded to each other. Ten samples were removed from water after 24 hours, and the remaining 10 samples after 10 days. They were examined for adhesive strength by an Instron tensile tester. Table 7 shows the average of the results obtained. The adhesives developed cohesive failure at a tensile strength of 400 kg/cm² (39.2 MPa), or above, and adhesive failure mainly on the Ni-Cr alloy surface at a lower tensile strength.

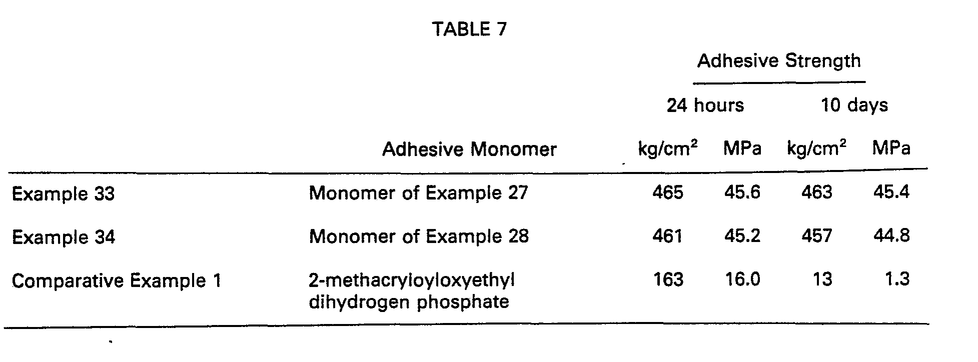

As is obvious from Table 7, the adhesive compositions of this invention showed a very high initial adhesive strength exceeding 400 kg/cm² (39.2 MPa), and did not undergo any appreciable reduction in adhesive strength after 10 days of immersion in water. On the other hand, the adhesive composition of Comparative Example 1 showed a great reduction in adhesive strength as a result of immersion in water.

### Examples 35 and 36, and Comparative Example 4

Two-pack adhesive compositions were prepared in accordance with the following recipe by employing the adhesive monomers of Examples 27 and 28 and Comparative Example 1, applied for bonding a commercially available dental composite resin to acid-etched human dentin and unetched dentin, and tested for adhesive strength:

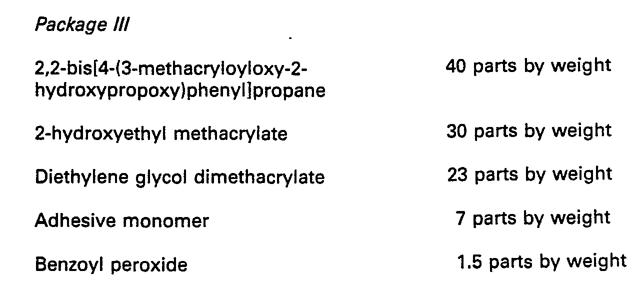

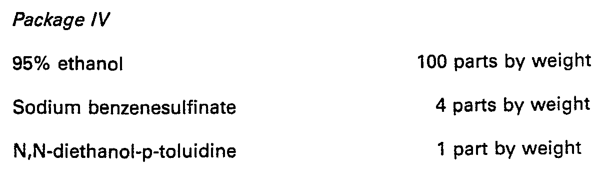

Human molars were cut in the shape of a square pillar to have an exposed adherend dentin surface and square ivory bars measuring 10 mm square by 30 mm long were also prepared. They were held in cold water until immediately prior to use. A half of the molars were subjected to acid etching, while the other molars were not. Immediately prior to use, water was wiped off the adherend dentin surface of each molar. The dentin surface of each molar to be etched was etched for a minute with a 40% aqueous solution of orthophosphoric acid. The etched surface was washed carefully with flowing water, and clean air or nitrogen was blown against the dentin surface to volatilize moisture therefrom. A strip of aluminum was wound about the molar. Water was also wiped off the adherend surfaces of the ivory bars. A mixed solution containing equal volumes of the liquids III and IV was coated on the teeth and the ivory bars, and clean air or nitrogen was blown against them to dry them. A commercially available dental composite resin Clearfil F (trademark of KURARAY CO., LTD., Japan) was kneaded. This paste was sandwiched between the adherend surfaces of the teeth and the ivory bars, and bonded therewith. The test samples were immersed in water at 37°C after 30 minutes of adhesion, and tested after one day for adhesive strength by an Instron tensile tester at a crosshead speed of 2 mm/min. The results are shown in Table 8. Seven teeth were employed for testing each adhesive composition. Table 8 shows the average of the results obtained.

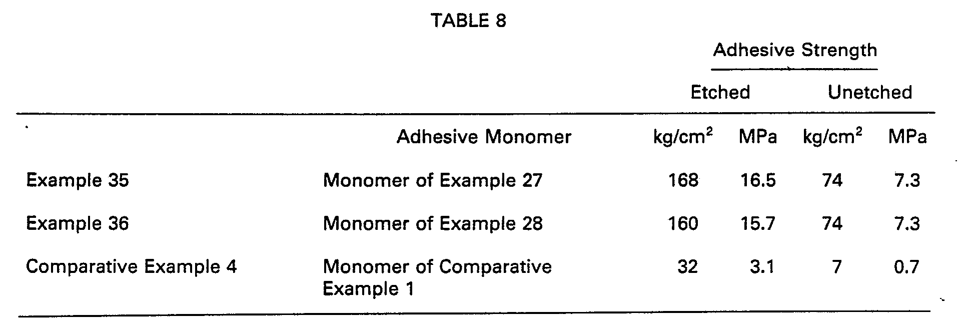

As is obvious from Table 8, the adhesive compositions of this invention showed an extremely higher adhesive strength than that of Comparative Example 4 containing a conventional phosphate monoester monomer. The adhesive composition of this invention provides a high adhesive strength as required for clinical application in dentistry, even if no acid etching is made on the dentin. This enables the simplification of dental treatment which has never been expected from the prior art.

### Example 37

A one-liter three-neck flask was charged with 250 g (2.9 mols) of methacrylic acid, 480 g (2.4 mols) of 1,3-bis(2-hydroxyethoxy)benzene, 35 g of p-toluenesulfonic acid, and 0.6 g of MEHQ. They were heated to 85°C until they were dissolved to form a uniform solution. The flask pressure was reduced to a level of 20 to 100 mm Hg (2.7 to 13 kPa) by an aspirator. While oxygen was blown into the flask, the solution was heated to a temperature of 85°C to 90°C, and water was removed by distillation. When water ceased to appear after about four hours, the reaction was discontinued. While the reactant solution was still hot, it was poured into 1.5 liters of toluene, and the precipitated unreacted diol was removed by a #4 filter. The filtrate was washed twice with a 5% aqueous solution of Na₂C0₃, and three times with neutral water, and dried with anhydrous sodium sulfate. After 0.1 g of MEHQ had been added, toluene was removed by vacuum distillation at a temperature not exceeding 80°C. There was obtained a monoester and diester mixture weighing 460 g. The analysis of the mixture by HLC indicated a monoester content of 64% by weight, and only a trace of unreacted diol.

A solution containing 55.2 g (0.36 mol) of phosphorus oxychloride in 150 cc of tetrahydrofuran was placed in a one-liter reactor, and cooled to -50°C. A solution containing 125 g of the monoester and diester mixture, which contained 0.3 mol of monoester, and 33.4 g (0.33 mol) of triethylamine in 150 cc of tetrahydrofuran was placed in a 500 cc dropping funnel, and the funnel was connected to the reactor. The monoester solution was dropped slowly, while dry N₂ gas was being blown into the reactor to stir the phosphorus oxychloride solution strongly. The internal temperature of the reactor was kept at a level of -45°C to -50°C while the dropping operation was continued, and at -45°C for an hour after its termination. The temperature was, then, raised to 0°C, and a solution containing 30 ml of water and 75.9 g of triethylamine in 100 cc of tetrahydrofuran was dropped slowly. The reactant solution was cooled with ice, and stirred continuously for 15 hours thereafter, whereby the P-CI bonds were hydrolyzed. The precipitated triethylammonium chloride was removed by a #4 glass filter. After 30 mg of MEHQ had been added into the filtrate, tetrahydrofuran was removed by vacuum distillation at a temperature not exceeding 30°C. A solution of the residue in 800 cc of ethyl acetate was put in a separatory funnel, and washed three times with 300 cc of a 2N aqueous solution of hydrochloric acid, and twice with 300 cc of neutral water. After the residue had been dehydrated overnight with anhydrous sodium sulfate, 30 mg of MEHQ were added, and ethyl acetate was removed by vacuum distillation to yield a liquid residue. It was washed with toluene repeatedly, and any volatile matter in the residue was removed by vacuum distillation. There was obtained a solid crude phosphate monoester weighing 67 g. The monoester was dissolved in 150 cc of acetone, and after the insoluble matter had been removed by filtration, the phosphate monoester in the solution was recrystallized by adding a mixed solvent containing toluene and n-hexane in a ratio by volume of 1:1 to yield 58 g of crystals.

The crystals had a melting point of 64°C to 67°C. The NMR at 90 MHz of a dₛ-acetone solution of the crystals was examined at ordinary room temperature. There were found an ethylenic proton signal at 5 = 6.0₅ and 5.5₅, multifold signals of eight methylenic protons at 5 = 4.0 to 4.5, a methyl proton signal at 6 = 1.8₅, four proton signals of phenylene at δ = 6.3 to 6.5 and 7.0 to 7.2, and a phosphoric proton signal at 6 = 9.₂. The elemental analysis of the compound indicated 49.0% C (theoretically 48.6%) and 8.6% P (8.9%), and showed that it was a compound of the formula:

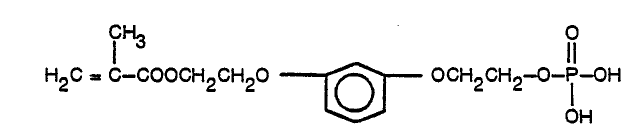

### Example 38

A diol monoester and diester mixture was prepared by repeating the procedures of Example 37, except for the use of 216 g (3.0 mols) of acrylic acid instead of methacrylic acid. After the monoester content of the mixture had been examined by HLC, that quantity of the mixture which contained 0.3 mol of monoester was subjected to phosphate esterification. The procedures of Example 37 were followed for separating the reaction product to yield a solid compound weighing 60 g. The NMR of the compound, and its elemental analysis [C: 47.5% (theoretically 47.0%) and P: 8.9% (9.3%)] indicated that it was a compound of the formula: 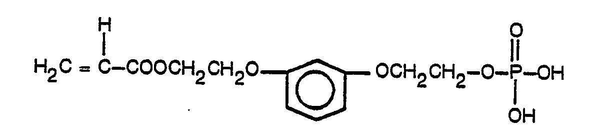 It showed a purity of at least 95% upon analysis by HLC.

### Examples 39 to 42

The procedures of Examples 37 and 38 were followed for synthesizing four compounds as shown in Table 9 by employing methacrylic or acrylic acid, and 1,3-(bis(3-hydroxypropoxy)benzene or 1,3-bis(4-hydroxybutoxy)benzene as diol. The structure of each compound was determined by NMR and elemental analysis, and its purity by HLC. As regards the results of elemental analysis, the values of C, H and P alone are shown in Table 9. All the compounds showed a purity of at least 95%.

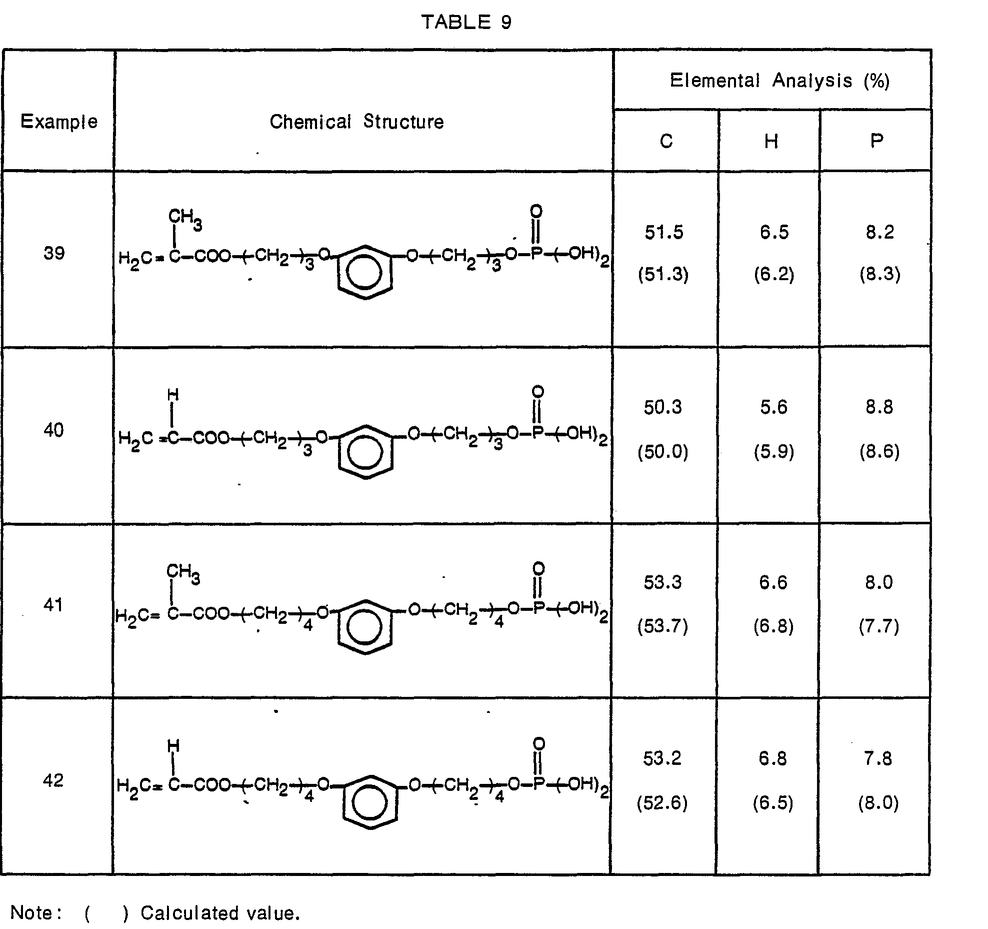

### Examples 43 and 44

Three adhesive compositions containing the compounds of Examples 37 and 38, and 2-methacryloyloxyethyl dihydrogen phosphate as the adhesive monomer were prepared in accordance with the following recipe, and tested for adhesive strength and its durability in water:

### Components and Proportions:

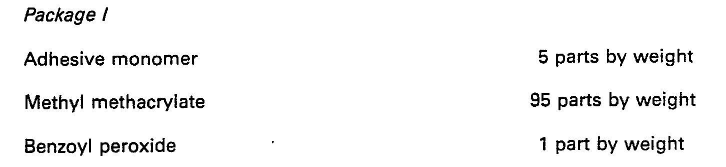

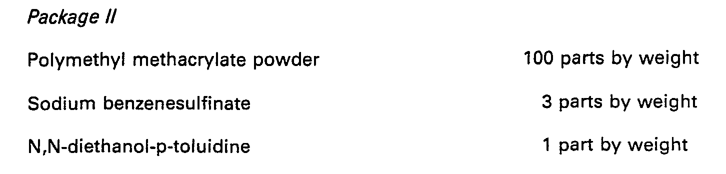

Package It was a uniform mixture of the benzenesulfinate and amine powders in the PMMA powder.

A 10 mm square by 3 mm thick plate of a Ni-Cr alloy containing 92.7% of nickel was polished with #1000 abrasive paper, and washed ultrasonically in water and methyl ethyl ketone. An adhesive cellophane tape having a hole with a diameter of 5 mm was bonded to the plate so that the hole might be aligned with the center of the polished plate surface. An equal number of stainless steel bars having a diameter of 7 mm and a length of 35 mm were prepared, and one end surface of each bar was polished and washed as the alloy plates had been. A mixture containing equal weights of the components in Packages I and II was applied by a brush to the alloy plate exposed through the hole in the cellophane tape, and the stainless steel bar, and the plate and the bar were joined to each other by forming a butt joint. Twenty samples, or bonded bar and plate combinations were prepared by employing each adhesive composition, and immersed in water at 37°C after an hour. Ten samples were removed from water after 24 hours, and the remaining 10 samples after 10 days. They were examined for adhesive strength by an Instron tensile tester. Table 10 shows the average of the results obtained. Adhesive failure was entirely found on the Ni-Cr alloy surface.

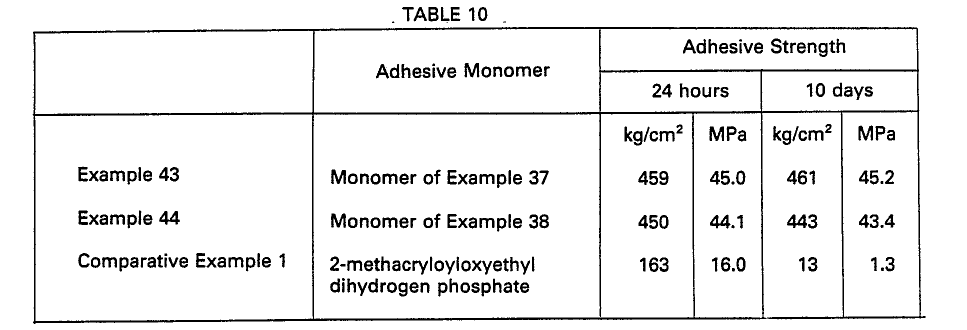

As is obvious from Table 10, the adhesive compositions of this invention showed a very high initial adhesive strength exceeding 400 mg/cm² (39.2 MPa), and did not undergo any appreciable reduction in adhesive strength after 10 days of immersion in water. On the other hand, the composition of Comparative Example 1 containing a conventional phosphate ester showed a great reduction in adhesive strength as a result of immersion in water.

### Examples 45 and 46, and Comparative Example 5

Two-pack adhesive compositions were prepared in accordance with the following recipe by employing the monomers of Examples 37 and 38, and Comparative Example 1, applied to bond a commercially available dental composite resin to acid etched human dentin and unetched dentin, and examined for adhesive strength:

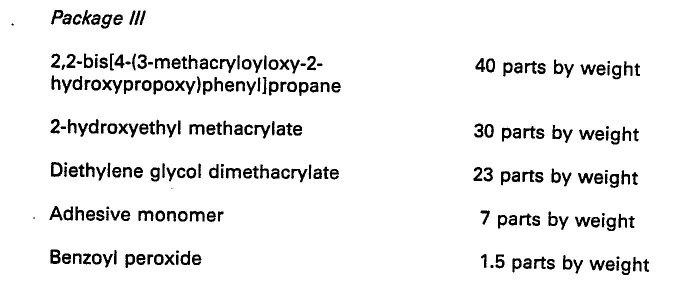

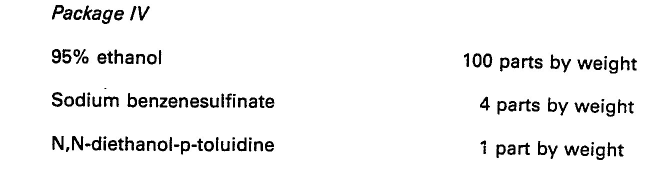

Human molars were cut in the shape of a square pillar to expose an adherend dentin surface and square ivory bars measuring 10 mm square by 30 mm long were also prepared. They were kept in cold water until immediately prior to use. A half of the molars were subjected to acid etching, while the other molars were not. Immediately prior to use, water was wiped off the adherend dentin surface of each molar. The dentin surface of each molar to be etched was subjected to etching for a minute with a 40% aqueous solution of orthophosphoric acid. The etched surface was washed carefully with flowing water, and clean air or nitrogen was blown against the etched surface to volatilize water therefrom. A strip of aluminum was wound about the molar. Water was wiped off the adherend surfaces of the ivory bars, too. A mixed solution containing equal volumes of the liquids III and IV was coated on the teeth and the ivory bars, and clean air or nitrogen was blown against them to dry them. A commercially available dental composite resin Clearfil F (trademark of KURARAY CO., LTD., Japan) was kneaded. The resulting paste was sandwiched between the adherend surfaces of the tooth and the ivory bar, and the tooth and the ivory bar were joined to each other. The test samples were immersed in water at 37°C after 30 minutes of adhesion, and examined after one day for adhesive strength by an Instron tensile tester at a crosshead speed of 2 mm/min. The results are shown in Table 11. Seven teeth were employed for testing each adhesive composition. Table 11 shows the averaqe of the results obtained.

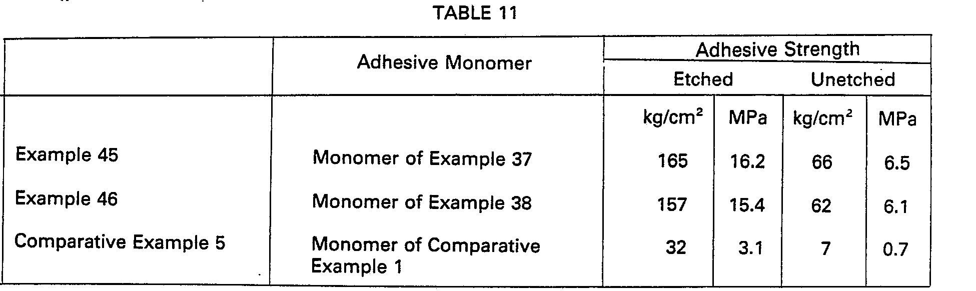

As is obvious fromTable 11, the adhesive compositions of this invention showed an extremely higher adhesive strength than the composition containing a conventional phosphate ester The adhesive composition of this invention provides a high adhesive strength as required for clinical application in dentistry, even if no acid etching is made on the dentin. This enables the simplification of dental treatment which has never been expected from the prior art.

### Example 47

Two-pack adhesive compositions were prepared in accordance with the following recipe by employing the adhesive vinyl compounds shown in Table 12, tested for bonding a commercially available dental composite resin to the dentin of a human tooth, and examined for adhesive strength:

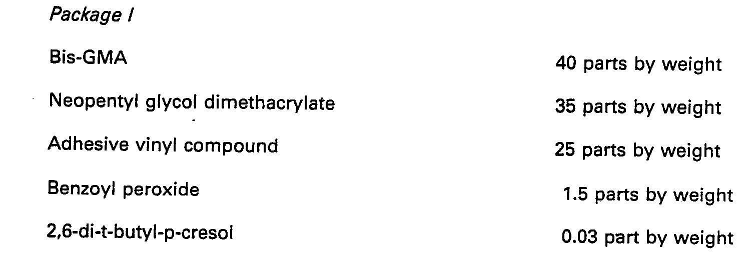

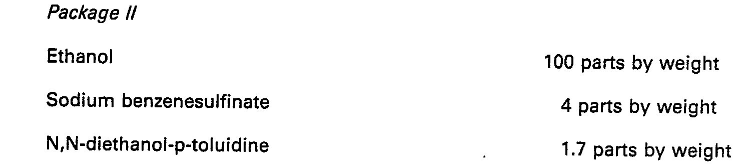

Human molars were cut in the shape of a square pillar to expose an adherend dentin surface and square ivory bars measuring 10 mm square by 30 mm long were also prepared. They were kept in cold water until immediately prior to use. Immediately prior to use, water was wiped off the adherend dentin surface of each molar, and the dentin surface was etched with a 40% aqueous solution of orthophosphoric acid for a minute. The etched surface was washed carefully with flowing water, and clean air or nitrogen was blown against it to volatilize any moisture. A strip of aluminum was wound about the molar. Water was wiped off the adherend surfaces of the ivory bars, too. A mixture containing equal volumes of the liquids I and II was coated on the adherend surfaces of the tooth and the ivory bar. Clean air or nitrogen was blown against the adherend surfaces to volatilize ethanol. A commercially available dental composite resin Clearfil F (trademark of KURARAY CO., LTD., Japan) was kneaded to form a paste. This paste was sandwiched between the adherend surfaces of the tooth and the ivory bar, and bonded thereto. The test samples were immersed in water at 37°C after 30 minutes of immersion, and examined after one day for adhesive strength by an Instron tensile tester at a crosshead speed of 2 mm/min. The results are shown in Table 12. Seven teeth were employed for testing each adhesive composition. Table 12 shows the average of the results obtained.

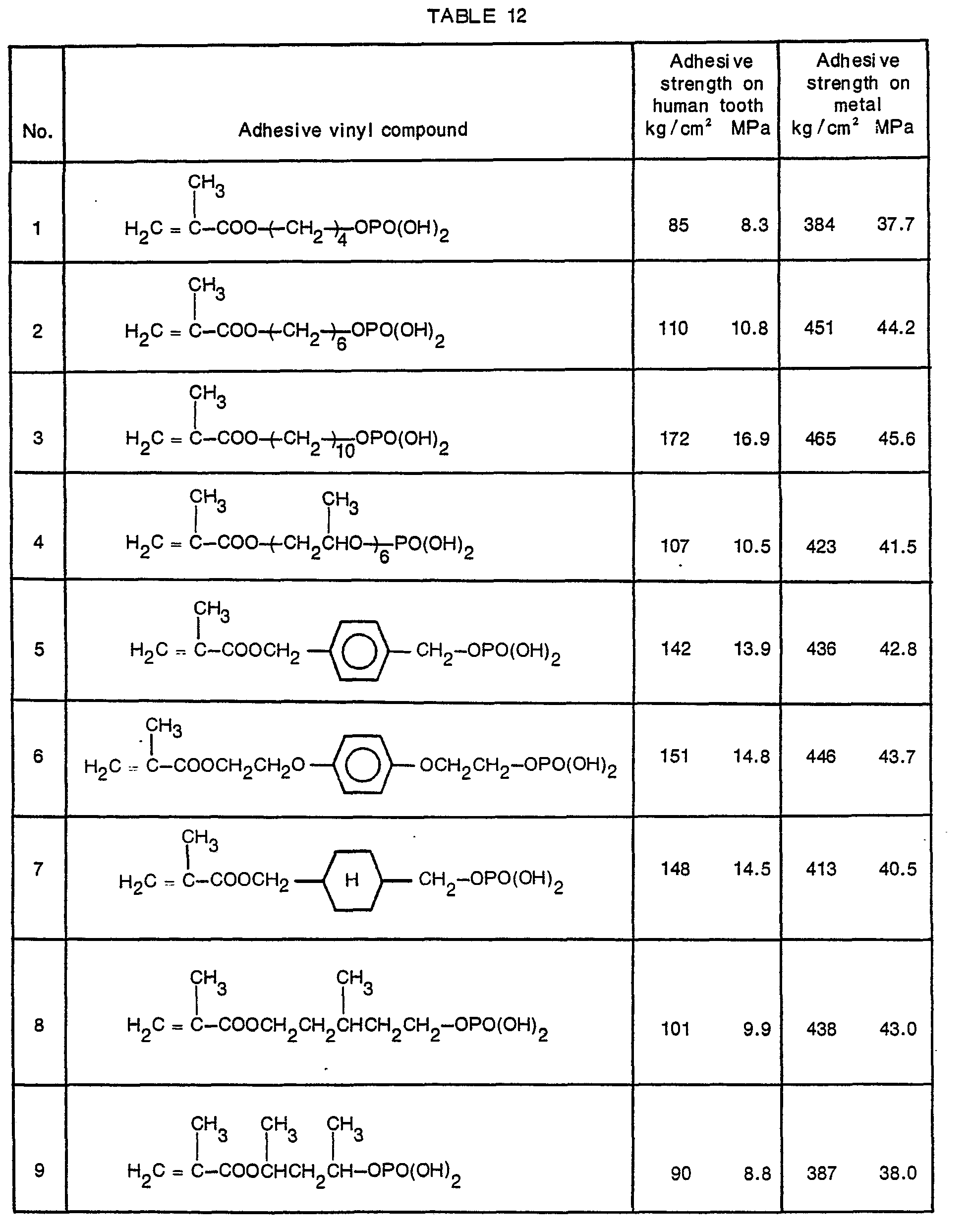

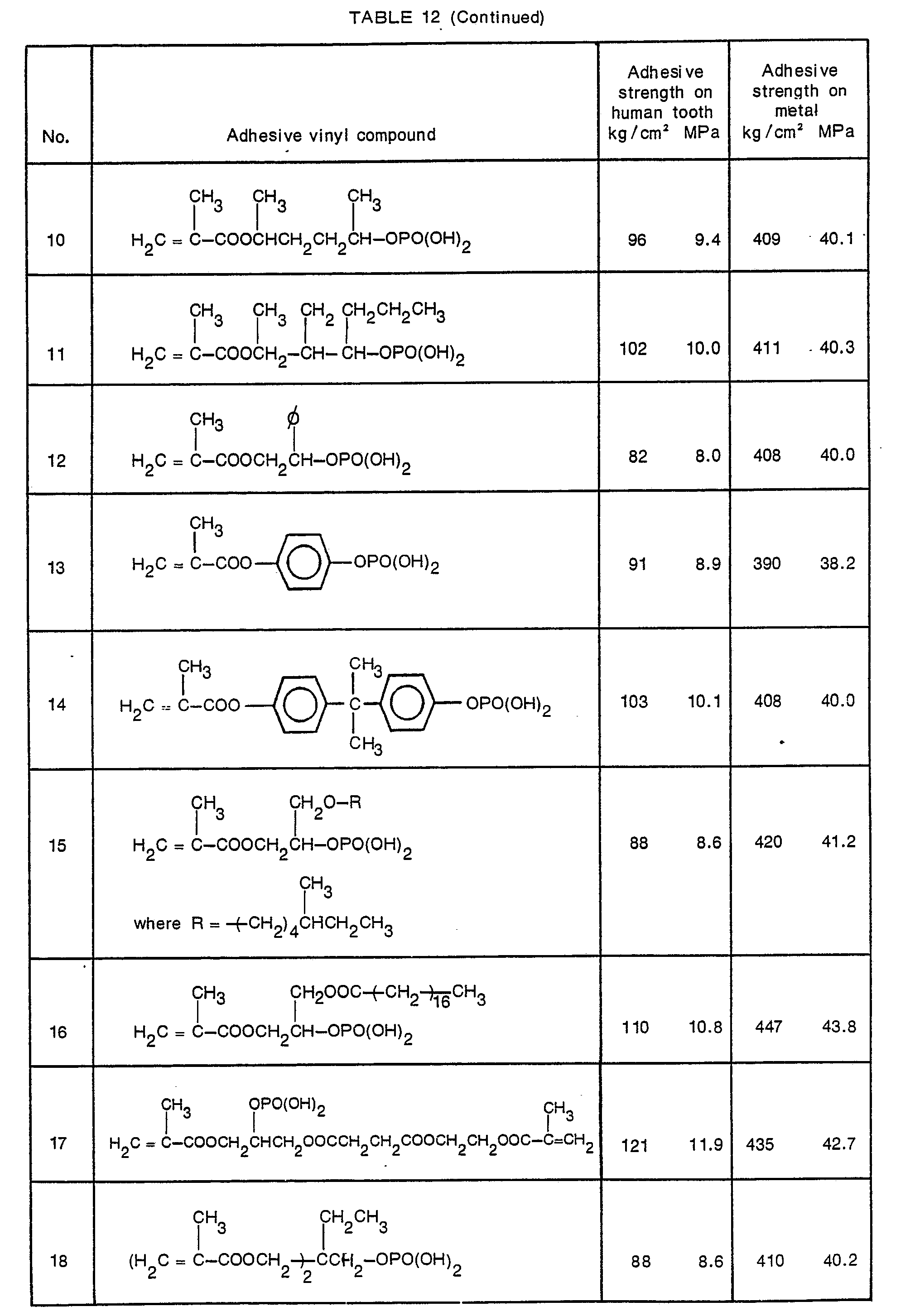

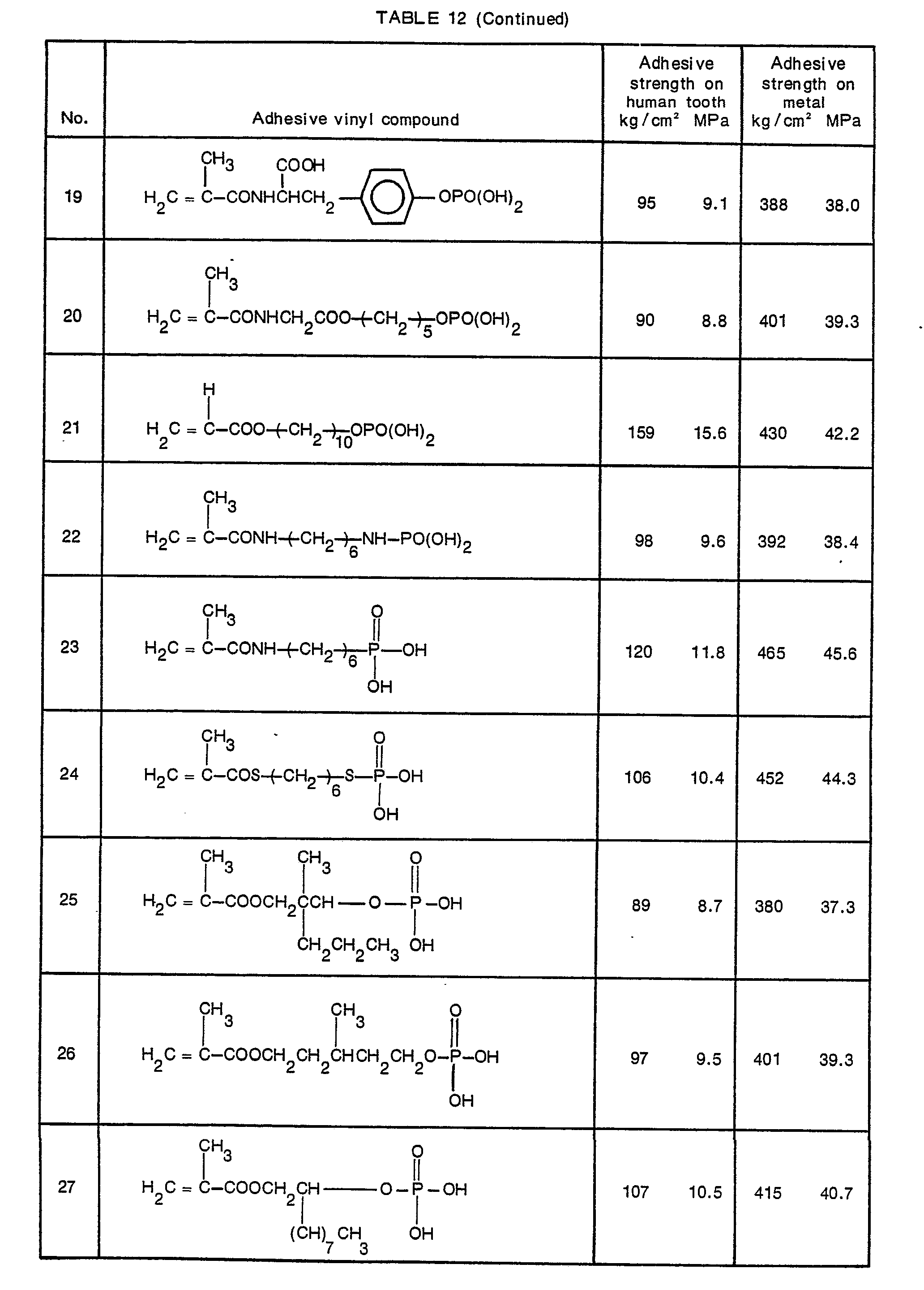

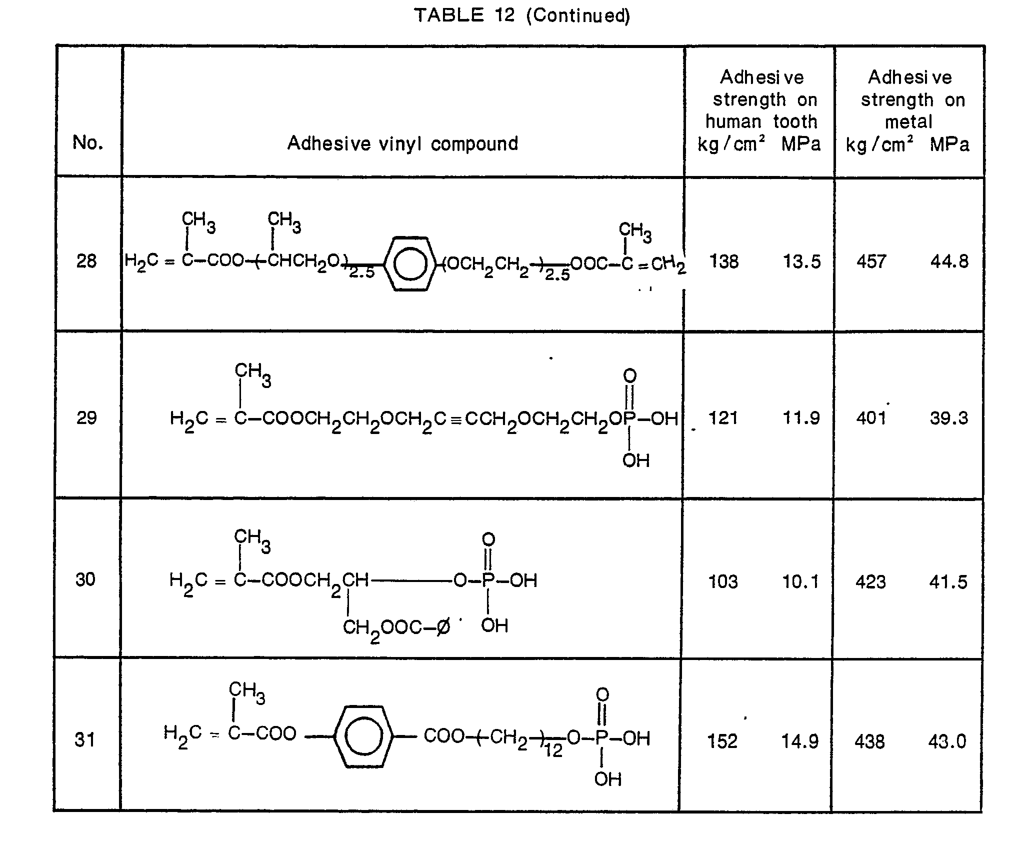

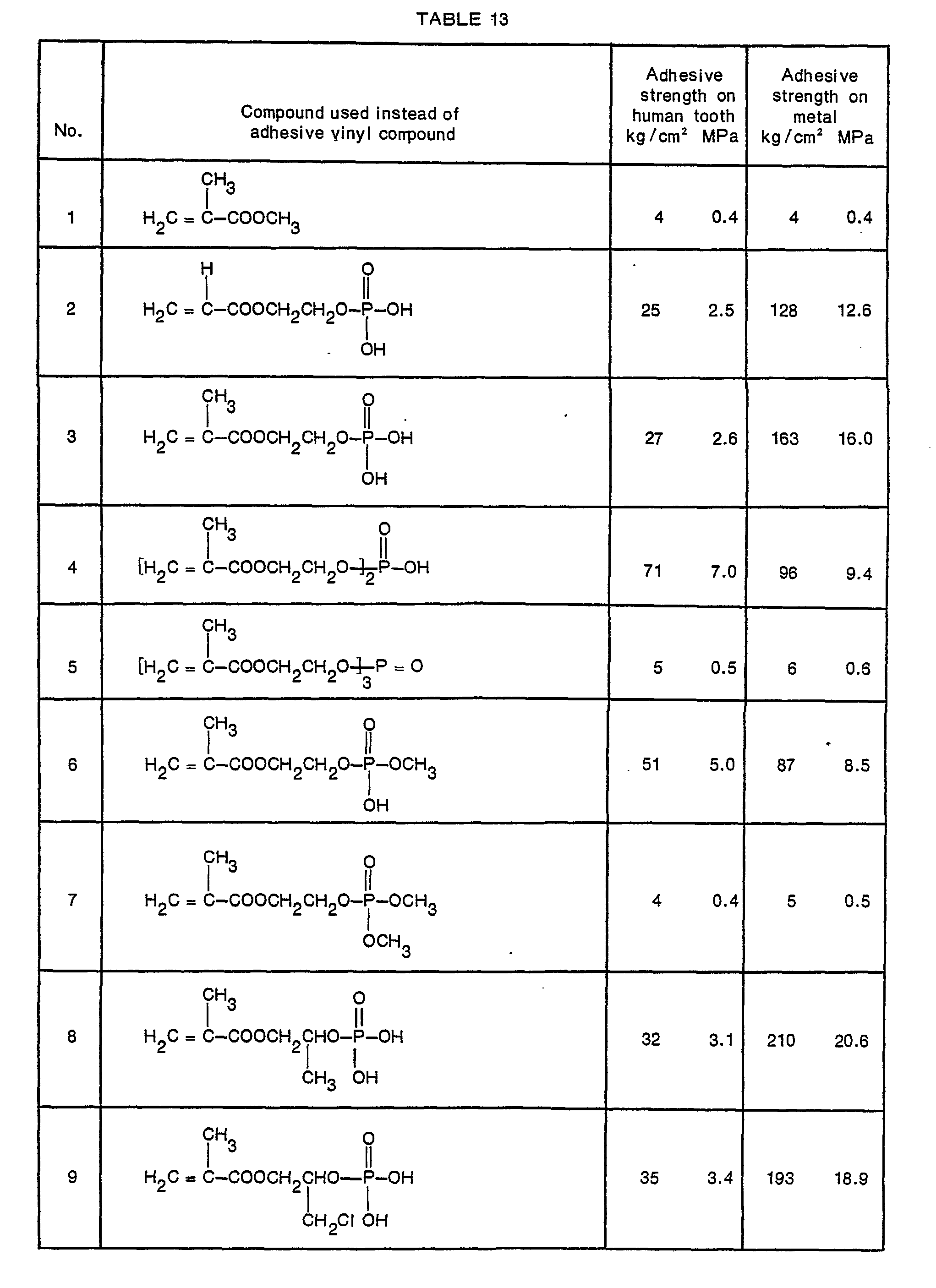

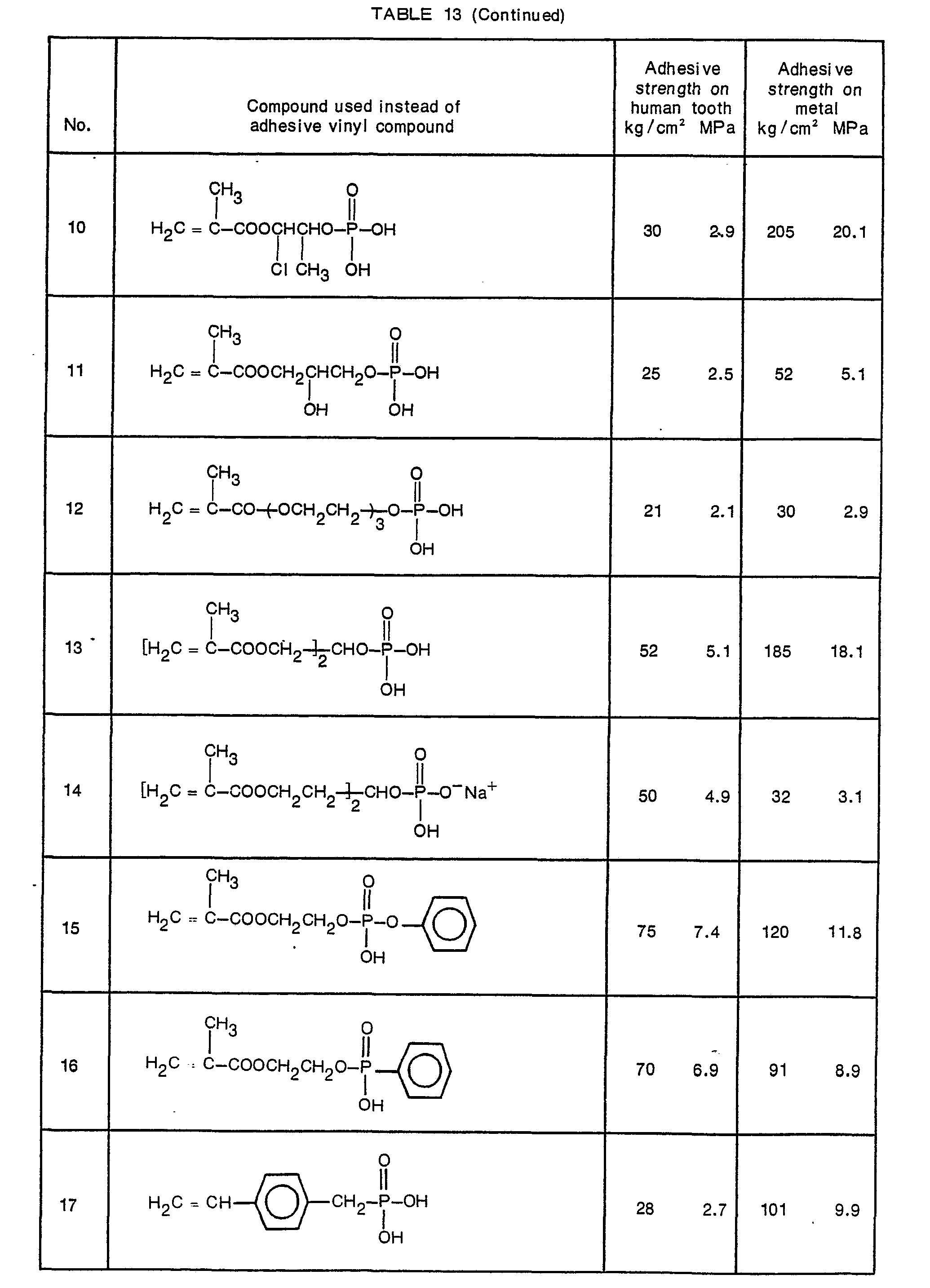

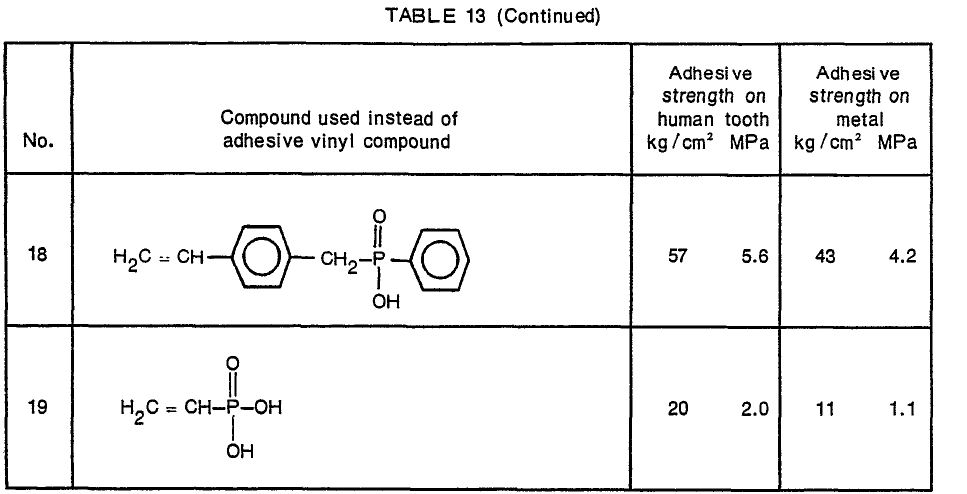

### Comparative Example 6

Adhesive compositions were prepared by employing the adhesive vinyl compounds shown in Table 13, and repeating the procedures of Example 47 in all the other respects. The results are shown in Table 13.

### Example 48

Powder-liquid type adhesive compositions were prepared in accordance with the following recipe by employing the adhesive vinyl compounds employed in Example 47, and tested for adhesive strength on metal:

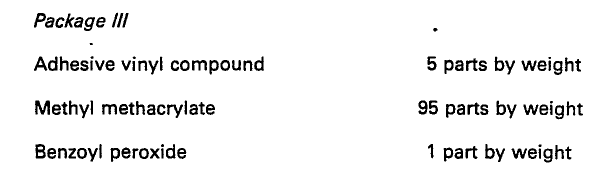

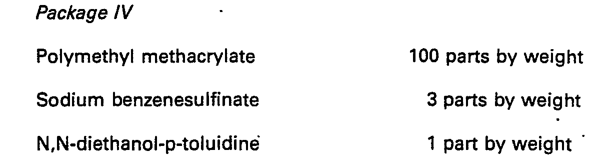

A cast plate of a dental Ni-Cr alloy Now Chrom (I) (product of TOWA GIKEN K.K., Japan, containing 92.7% Ni, and 6.0% Cr) was ground to provide a smooth surface, polished with #1000 abrasive paper, washed ultrasonically, and dried. An adhesive cellophane tape having a hole with a diameter of 5 mm was bonded to the polished plate surface. A stainless steel (SUS 304) bar having a diameter of 7 mm and a length of 35 mm was likewise polished with #1000 abrasive paper, washed ultrasonically, and dried. Equal weights of the components in Packages III and IV were placed in a Dappen dish, and mixed together for 10 to 20 seconds. The resultant viscous mixture was coated on the adherend surfaces of the alloy plate and the stainless steel bar. They were joined to each other by forming a butt joint. The bonded plate and bar combination was immersed in water at 37°C after one hour, and tested after 72 hours for adhesive strength by an Instron tensile tester at a crosshead speed of 2 mm/min. The results are shown in the right column of Table 12.

### Comparative Example 7

Adhesive compositions were prepared by employing the 19 vinyl compounds shown in Table 13 instead of the adhesive vinyl compounds in Package III employed in Example 48, and otherwise repeating the procedures of Example 48. They were tested for adhesive strength on the Ni-Cr alloy. The test results are shown in the right column of Table 13.

### Example 49 and Comparative Example 8

The procedures of Example 48 were followed for bonding a Ni-Cr alloy by employing the six adhesive vinyl compounds shown in Table 14. The test samples were immersed in water at 37°C after one hour of adhesion, and tested after 10 days for adhesive strength. The results are shown in Table 14. As is obvious from Table 14, the dental adhesive composition of this invention showed a high adhesive strength on not only the dentin, but on the metal as well. It is surprising to note that the adhesive composition of this invention shows a by far higher adhesive strength than the methacryloyloxy dihydrogen phosphate (Comparative Example) which is known for its high adhesive strength on metals.

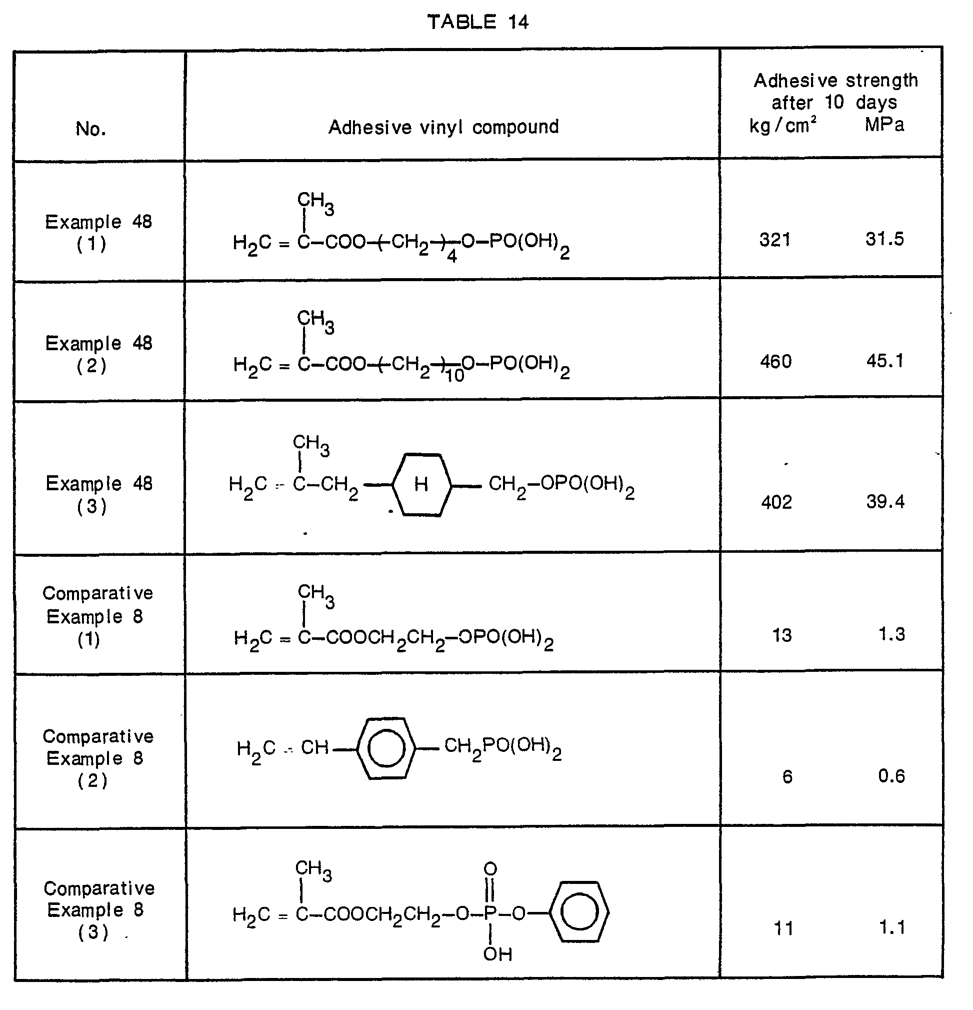

### Example 49a and Comparative Example 9

Two-pack adhesive compositions were prepared in accordance with the recipe of Example 47 by employing the phosphate esters shown in Table 15, though methacrylic acid was employed in Comparative Example 9 (3). The procedures of Example 47 were followed for adhesive strength tests on the unetched dentin of the human molar. The dentin surface was cut by a diamond point in a stream of water being poured to provide an adherend surface. The test results are shown in Table 15. The compounds employed in accordance with this invention were extremely superior in adhesive property to the conventional phosphate esters employed in Comparative Examples 9 (1) and (2). According to this invention, a clinically significant adhesive strength was obtained without the acid cleaning of the dentin surface.

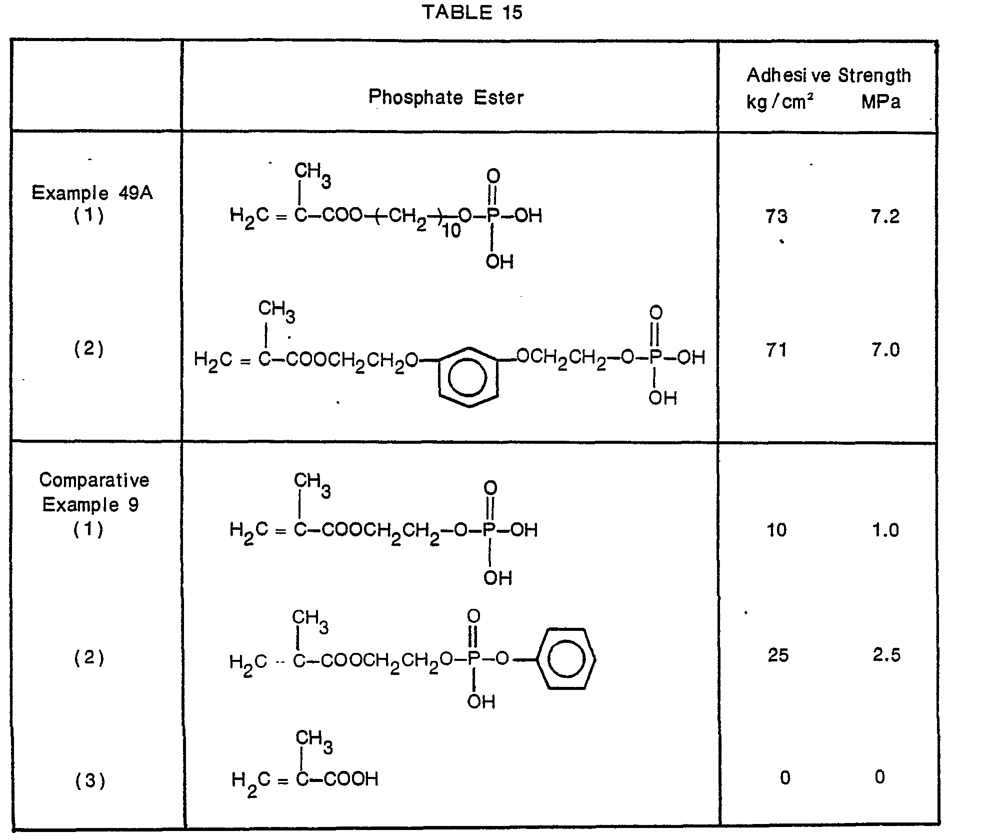

### Example 50

A dental restoration system comprising the adhesive composition of Example 49a (1) and a commercially available composite resin of the following recipe was tested for dental treatment:

### Two-Pack Composite Resin:

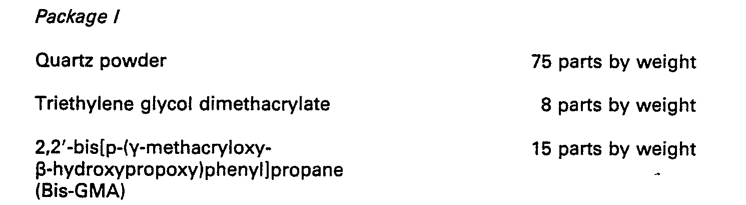

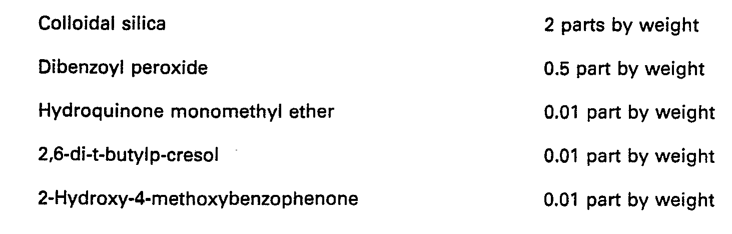

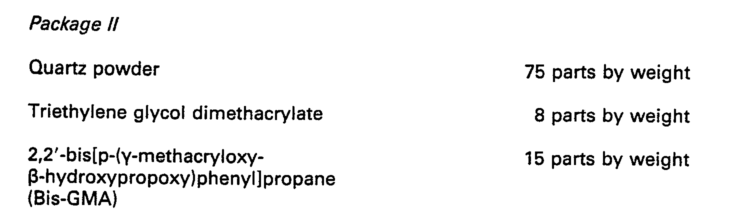

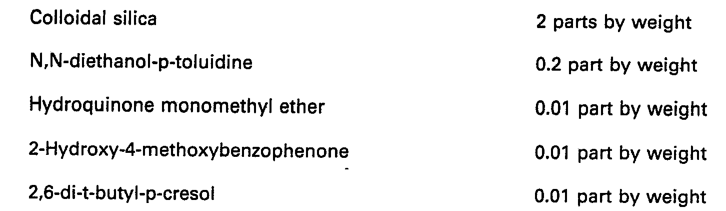

Two cylindrical cavities having a diameter of 3.5 mm and a depth of 3 mm were formed in an extracted human molar by a diamond point in a stream of water being poured. The cavities were treated with a 40% aqueous solution of phosphoric acid for a minute, washed with water, and dried fully by an air syringe. Two liquids forming the adhesive composition of Example 49 (1) were dropped into a Dappen dish, and mixed together. The resultant mixture was coated on the inner surfaces of the cavities, and air was blown thereagainst by an air syringe for several seconds to volatilize ethanol. The components of the two-pack composite resin were mixed, and the resultant mixture was filled in the cavities under pressure. The molar was immersed in water at 37°C overnight, and on the following day, the excess of the composite resin was removed to expose the margins of the cavities. The remaining portion of the tooth was sealed with an acrylic resin. The test samples were immersed alternately in two aqueous solutions of fuchsine having a temperature of 4°C and 60°C, respectively, 100 times for a minute each time for examination as to the penetration of the dye into the cavity margins (percolation test). After the percolation tests had been finished, the tooth was split, and did not indicate any penetration of fuchsine into the margins.

### Example 51

A powder-liquid type adhesive composition was prepared by employing adhesive monomer No. 3 of Example 47, and tested for adhesive strength on the dentin of human teeth, the enamel of bovine teeth, and a Ni-Cr alloy Nowchrom (I) (product of TOWA GIKEN K.K., Japan).

### (1) Components of the Adhesive Composition:

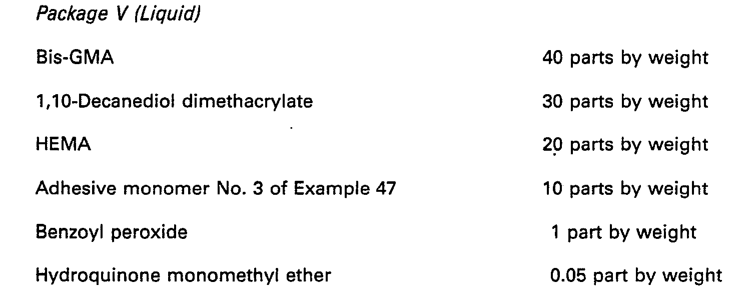

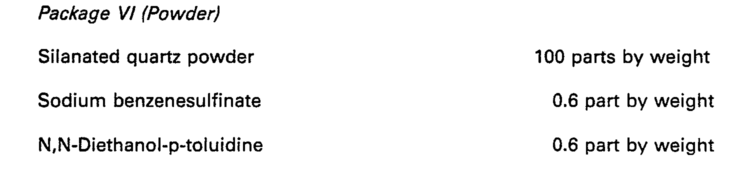

The powder VI was prepared by spraying a solution of sodium benzenesulfinate and N,N-diethanol-p-toluidine in 10 parts by weight of methanol uniformly on quartz powder, and volatilizing methanol.

### (2) Adhesion on Human Dentin:

The crown of a human molar was cut off to expose the dentin, while water was being poured on the tooth. The dentin surface was etched with a 40% aqueous solution of orthophosphoric acid for a minute, and after it had been washed with water, the water on the dentin surface was blown away by an air syringe. A double coated adhesive tape was bonded to the dentin surface. The tape had a hole with a diameter of 5 mm. The tooth was supported so that it might have a horizontal cross section. A plastics ring having an inside diameter of 6 mm and a thickness of 5 mm was placed on the tape so that the ring might be aligned with the center of the hole in the tape. The liquid V and the powder VI were mixed in a ratio by weight of 1:2, and kneaded for about one minute to form a paste. This paste was filled in the ring, and a hook for a tensile test was anchored in the paste and left at rest for 30 minutes. This test sample was immersed in water at 37°C. Five teeth were employed for testing each adhesive composition.

### (3) Adhesion on Bovine Tooth Enamel:

The labial surface of a bovine incisor was ground in a stream of water to provide a smooth surface. The procedures described at (2) above were followed for the acid etching of the labial surface, and the preparation of a test sample.

### (4) Adhesion on Ni-Cr Alloy:

A 10 mm square by 3 mm thick plate and a round bar having a diameter of 7 mm and a length of 20 mm were cast from a Ni-Cr alloy Nowchrom (I) containing 92% Ni (product of TOWA GIKEN K.K., Japan). The plate and the bar were polished with #1000 abrasive paper, washed ultrasonically with water, and dried by an air syringe. An adhesive backed tape Transpore (trademark) having a hole with a diameter of 5 mm was bonded to the alloy plate. The liquid V and the powder VI were mixed in a ratio by weight of 1:2, and kneaded for about one minute to form a paste. A thick layer of the paste was applied onto one end of the bar, and pressed against the plate to bond the bar to the plate. The test sample thus prepared was left at rest for 30 minutes, and immersed in water at 37°C. Six samples were prepared by employing each adhesive composition.

### (5) Adhesive Strength Test:

After one week of immersion in water at 37°C, each sample was examined for adhesive strength by an Instron tensile tester at a crosshead speed of 2 mm/min. Table 16 shows the average of the results obtained on the several samples in each case.

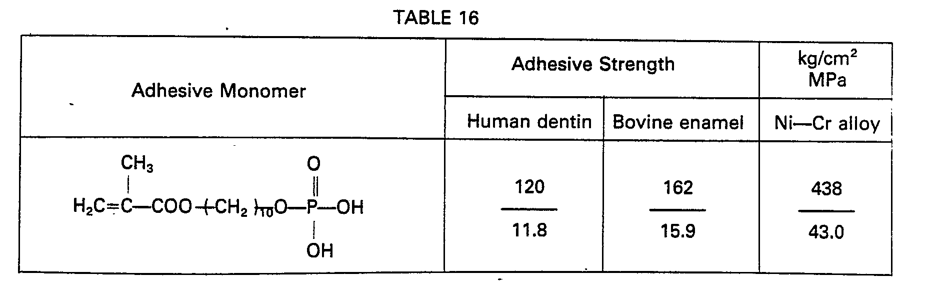

### Example 52

A cylindrical cavity having a diameter of 4 mm and depth of 3 mm was formed in the occlusal surface of an extracted human molar by a diamond point while water was being poured on the molar. An inlay fitting the cavity was cast from a 14-carat gold alloy, and its adherend surface was sandblasted with alumina particles having a particle size of 30 microns. The cavity was not etched with an acid, but was only washed with water, and dried fully by an air syringe. The liquid and the powder for the powder-liquid type adhesive composition (resin cement) employed in Example 51 were mixed in a ratio of 0.1 to 0.4 g, and kneaded for a minute to form a soft paste. This paste was coated on the cavity surface, and the inlay was immediately fitted in the cavity. The bonded molar and inlay assembly was immersed in water at 37°C after 10 minutes, and subjected to a percolation test after 24 hours in accordance with the method employed in Example 50. The test results indicated that the penetration of fuchsine had stopped in the enamel in the cavity, and that there was no penetration of fuchsine in the dentin.

### Example 53

A visible light curing dental composite resin was prepared from 15 parts by weight of Bis-GMA, 4 parts by weight of 1,2-bis(3-methacryloyloxy-2-hydroxypropoxy)ethane, 4 parts by weight of neopentyl glycol dimethacrylate, 3 parts by weight of 10-methacryloyloxydecyl dihydrogen phosphate, 0.1 part by weight of camphorquinone, 0.3 part by weight of allylthiourea, 73 parts by weight of silanated quartz powder, and 1.5 parts by weight of colloidal silica.

A cavity having a diameter of 3 mm and a depth of 2 mm was formed in the labial surface of an extracted human incisor by a diamond point. The cavity was etched, washed with water, and dried in accordance with the procedures of Example 50. The cavity was filled with the composite resin, and the resin was exposed for three minutes to the light of a 500 W tungsten-halogen lamp. The tooth was, then, immersed in water at 37°C. After it had been removed from water, it was cut, and the adhesion of the resin to the cavity wall was examined through a microscope. This examination indicated complete adhesion of the resin to the cavity wall, and did not reveal any clearance therebetween.

### Example 54 and Comparative Example 10

Powder-liquid type adhesive compositions were prepared in accordance with the following recipe by employing the phosphate esters shown in Table 17, tested for adhesion on nickel and a gold alloy, and examined for the durability of their adhesive strength in water.

### (1) Recipe:

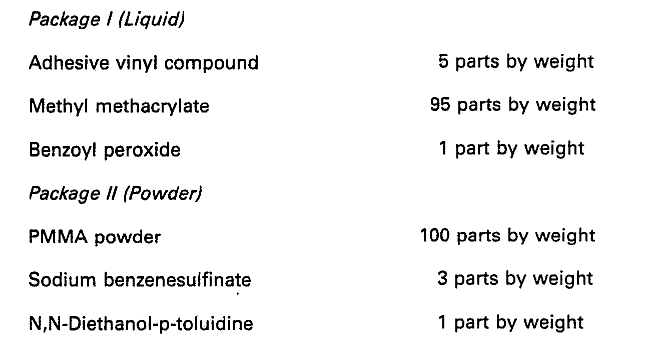

The powder II was prepared by grinding sodium benzenesulfinate and N,N-diethanol-p-toluidine into fine particles, and dispersing them in the PMMA powder.

### (2) Adhesion on Nickel:

One end surface of a round nickel bar having a diameter of 7 mm and a length of 30 mm was polished with #1000 silicon carbide abrasive paper, washed ultrasonically in water for 10 minutes, and dried. Equal weights of the liquid I and the powder II were mixed to form a viscous mixture. The viscous mixture was coated on the end surfaces of a pair of nickel bars forming one test sample, and the bars were joined together by forming a butt joint. Fourteen samples were prepared for testing each adhesive composition. The samples were left at rest in a temperature controlled bath having a constant temperature of 30°C for 24 hours, and seven samples were immediately removed from the bath and examined for adhesive strength by a tensile tester. The other seven samples were tested for adhesive strength after they had been immersed in water at 30°C for five days.

### (3) Adhesion on Gold Alloy:

An 8 mm square by 1.5 mm thick plate of a gold alloy containing 55% Au, 29% Ag and 8.9% Pd, and one end of a round stainless steel (SUS 304) bar having a diameter of 5 mm and a length of 30 mm were polished and washed, as the nickel bars had been. ATranspore (trademark) surgical tape having a hole with a diameter of 4 mm was bonded to the gold alloy plate to serve as a spacer. The adhesive composition was applied in a somewhat thick layer to the gold alloy plate exposed through the hole in the tape. The polished end of the stainless steel bar was pressed against the adhesive layer, and bonded to the gold alloy plate. The test samples thus prepared were kept and examined for adhesive strength in accordance with the procedures described at (2) above for the nickel samples. The results are shown in Table 17.

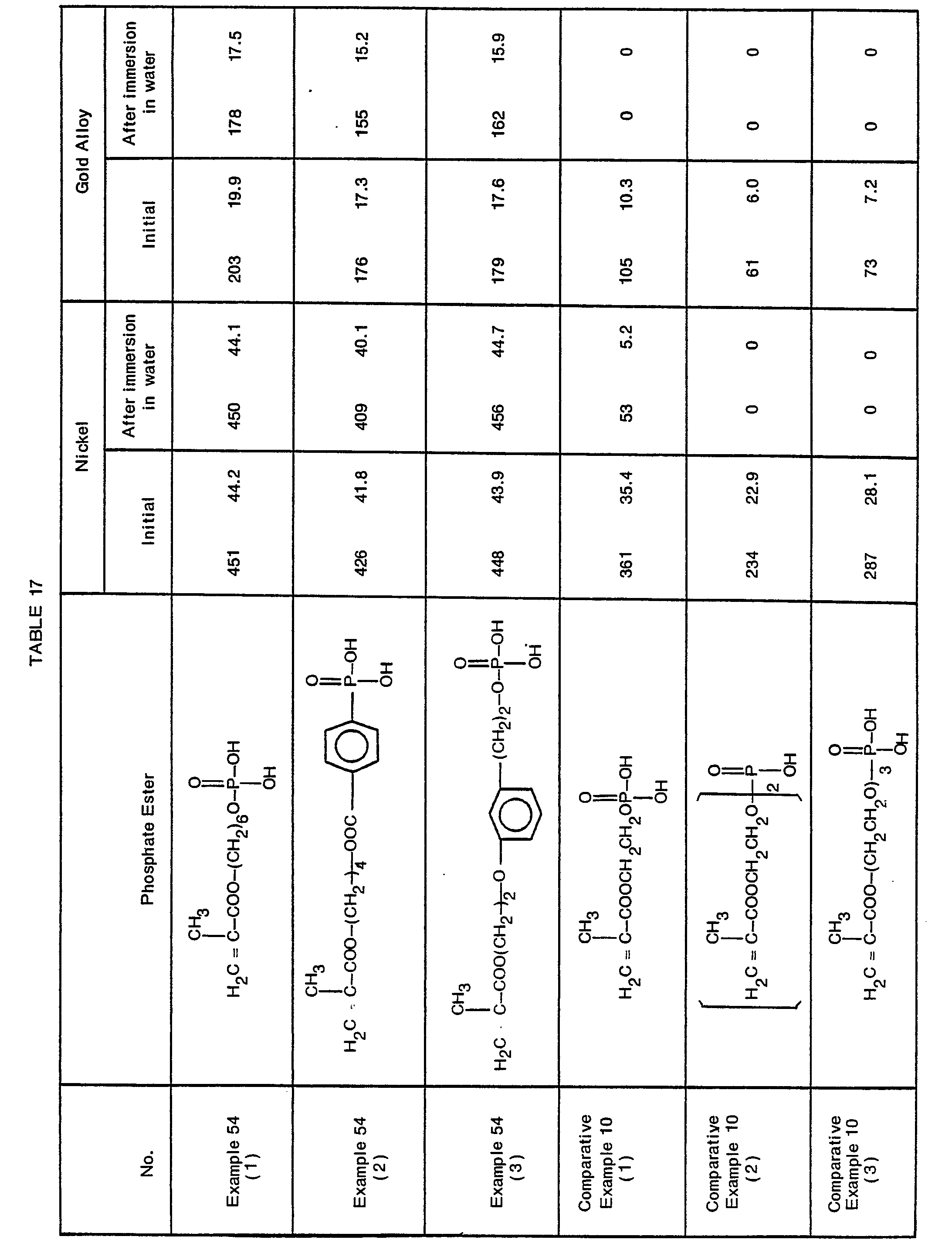

### Example 55

The procedures of Example 54 were followed for preparing test samples each consisting of a pair of round bars having a diameter of 7 mm and a length of 30 mm, and joined end to end by employing the adhesive composition containing the phosphate ester shown at Example 54 (1) in Table 17. The samples were prepared from bars of stainless steel (SUS 304), ordinary steel, aluminum, hard glass, PMMA and polycarbonate. They were left at rest in a dry temperature controlled bath having a constant temperature of 30°C for 24 hours, and examined for adhesive strength by a tensile tester. The results were as follows:

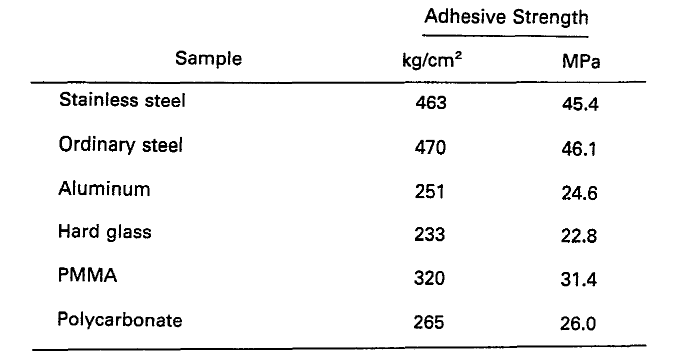

### Example 56 and Comparative Example 11

Powder-liquid adhesive compositions were prepared in accordance with the following recipe by employing the phosphate esters shown in Table 18:

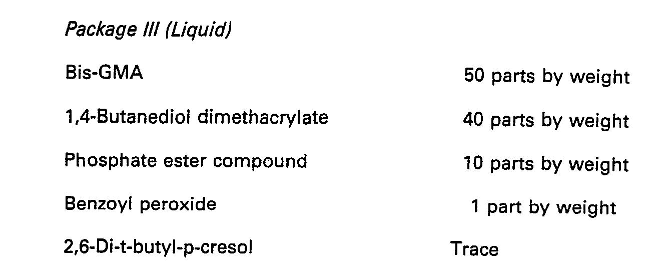

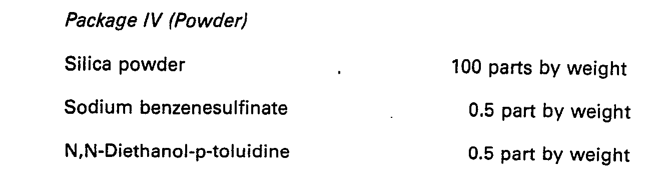

Test samples were prepared from stainless steel (SUS 304) bars having a diameter of 7 mm and a length of 30 mm. One end surface of each bar was polished with silicon carbide abrasive paper. The bars were immersed in water at 25°C. The liquid III and the powder IV were mixed in a ratio by weight of 1:4, and kneaded by a spatula for a minute to form an adhesive paste. A pair of bars were joined to each other by the paste while they were in water. The samples were left at rest in water at 25°C., and examined after one day for adhesive strength by a tensile tester. Five samples were prepared for testing each adhesive composition. Table 18 shows the average of the results obtained on those samples.

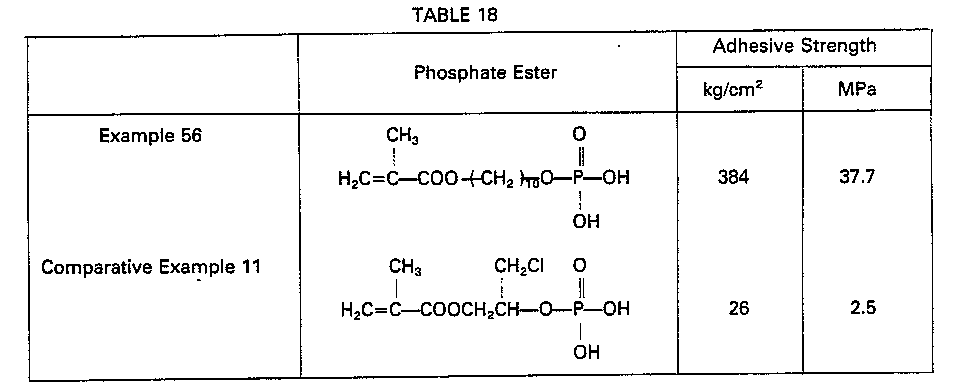

### Example 57 and Comparative Example 12

Light curable paint compositions were prepared in accordance with the following recipe by employing the phosphate esters shown in Table 19.

### Recipe

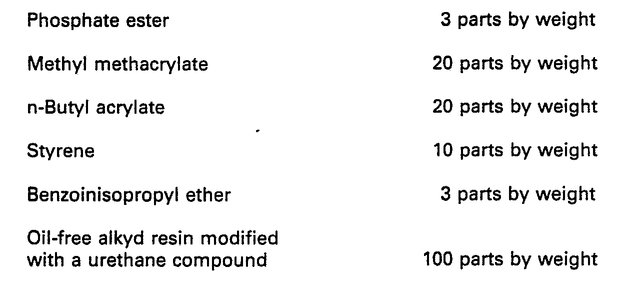

A film of each paint composition having a thickness of 40 microns was formed on a steel plate polished with #800 abrasive paper. The composition was cured by exposure for 20 seconds to the light of a 2 kW high-pressure mercury lamp positioned at a distance of 30 cm from the plate. The plate was, then, immersed in water at 70°C for 20 days, and the coated film was examined. The results are shown in Table 19.

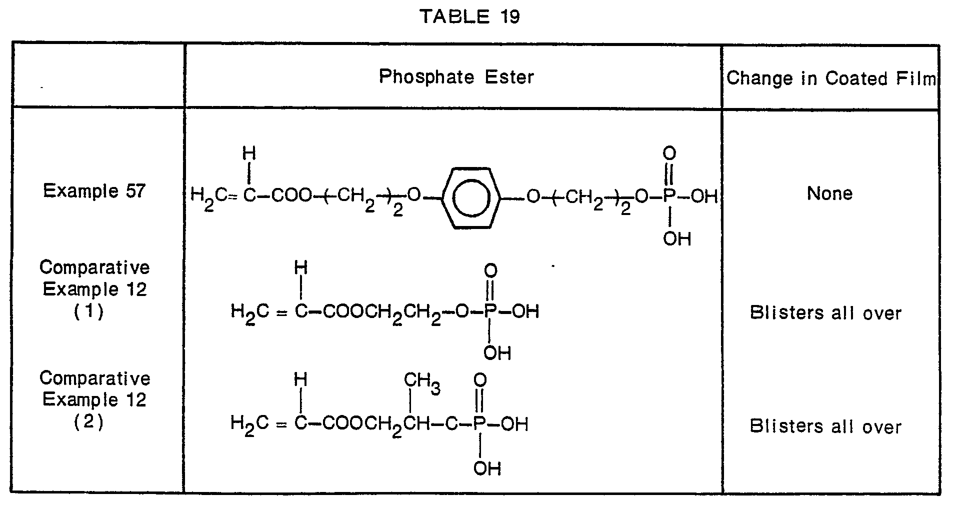

### Example 58 and Comparative Example 13

Powder-liquid type adhesive compositions were prepared in accordance with the following recipe by employing the phosphate esters shown in Table 20, tested for adhesion on nickel and a gold alloy.

### (1) Recipe:

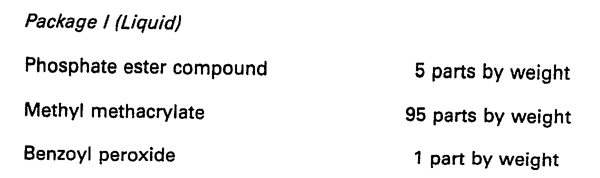

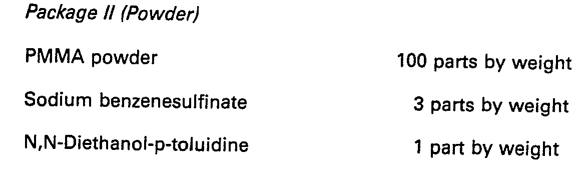

The powder was prepared by grinding sodium benzenesulfinate and N,N-diethanol-p-toluidine into fine particles, and dispersing them uniformly in the PMMA powder.

### (2) Adhesion on Nickel:

Round nickel bars having a diameter of 7 mm and a length of 30 mm had one end surface polished with #1000 silicon carbide abrasive paper, and were washed ultrasonically in water for 10 minutes, and dried. Equal weights of the liquid I and the powder II were mixed to form a viscous mixture. It was coated on the polished ends of the bars, and each pair of bars were joined end to end. Fourteen test samples were prepared for testing each adhesive composition. They were left at rest in a temperature controlled bath having constant temperature of 30°C for 24 hours. Seven of them were, then, removed from the bath immediately, and examined for adhesive strength by a tensile tester. The other seven samples were examined for adhesive strength after they had been immersed in water at 30°C for five days.

### (3) Adhesion on Gold Alloy:

An 8 mm square by 1.5 mm thick plate of a gold alloy containing 55% Au, 29% Ag and 8.9% Pd, and one end surface of a round stainless steel (SUS 304) bar having a diameter of 5 mm and a length of 30 mm were polished and washed as the nickel bars had been. A Transpore (trademark) surgical tape having a hole with a diameter of 4 mm was bonded to the gold alloy plate to serve as a spacer. The adhesive composition was applied in a somewhat thick layer to the gold alloy plate exposed through the hole in the tape. The polished end of the stainless steel bar was pressed against the adhesive layer, and bonded to the gold alloy plate. The test samples were kept and examined for adhesive strength in accordance with the procedures as hereinbefore described with reference to the nickel samples. The results are shown in Table 20.

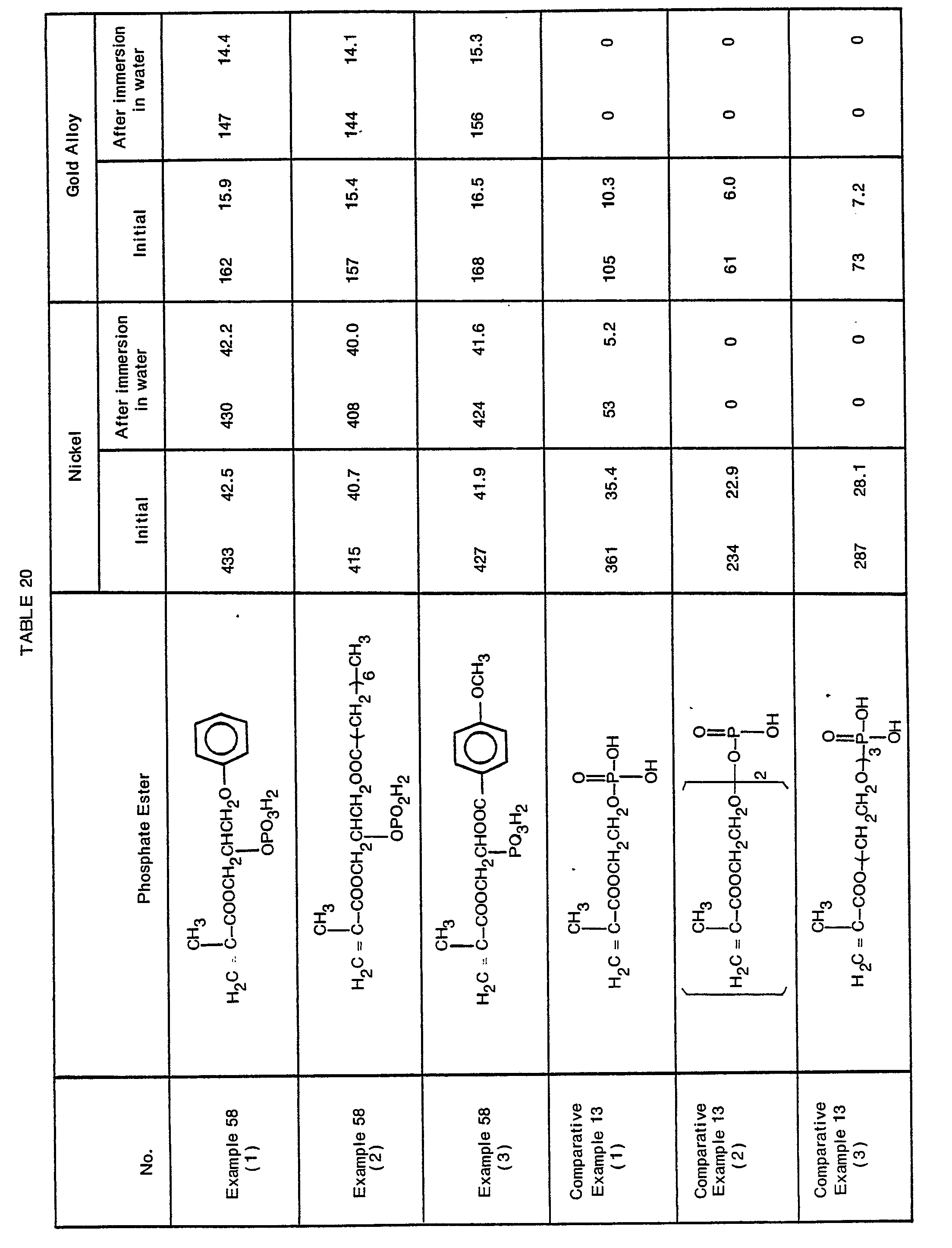

### Example 59

The procedures of Example 58 were followed for preparing test samples each consisting of a pair of round bars having a diameter of 7 mm and a length of 30 mm, and joined end to end by employing the adhesive composition containing the phosphate ester shown at Example 58 (1) in Table 20. The samples were prepared from bars of stainless steel (SUS 304), ordinary steel, aluminum, hard glass, PMMA and polycarbonate. They were left at rest in a dry temperature controlled bath having a constant temperature of 30°C for 24 hours, and examined for adhesive strength by a tensile tester. The results were as follows:

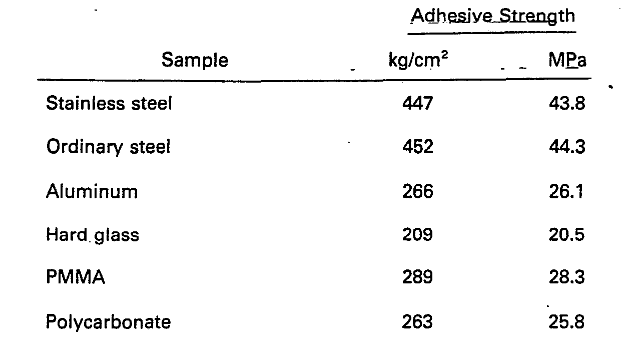

### Example 60 and Comparative Example 14

Powder-liquid type adhesive compositions were prepared in accordance with the following recipe by employing the phosphate esters shown in Table 21:

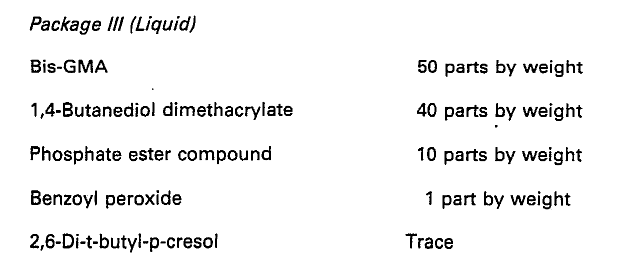

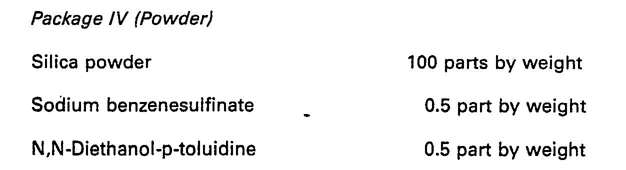

Test samples were prepared from stainless steel (SUS 304) bars having a diameter of 7 mm and a length of 30 mm. One end surface of each bar was polished with silicon carbide abrasive paper. The bars were immersed in water at 25°C. The liquid III and the powder IV were mixed in a ratio by weight of 1:4, and kneaded by a spatula for a minute to form an adhesive paste. A pair of bars were joined to each other by the paste while they were in water. The samples were left at rest in water at 25°C, and examined after one day for adhesive strength by a tensile tester. Five samples were prepared for testing each adhesive composition. Table 21 shows the average of the results obtained on those samples.

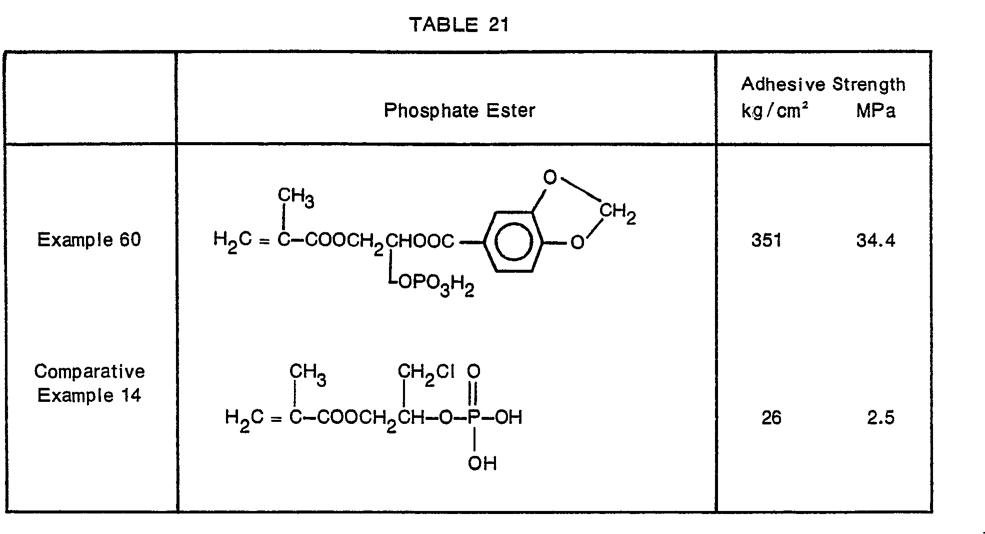

### Example 61 and Comparative Example 15

Light curable paint compositions were prepared in accordance with the following recipe by employing the phosphate esters shown in Table 22:

### Recipe

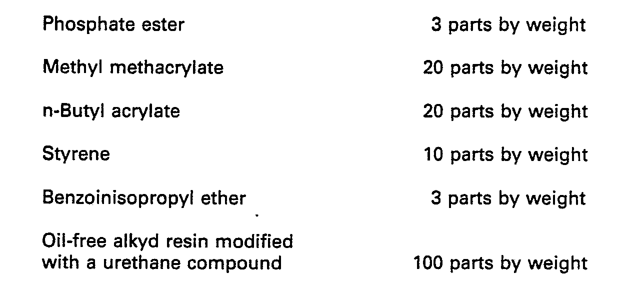

A film of each paint composition having a thickness of 40 µm was formed on a steel plate polished with #800 abrasive paper. The composition was cured by exposure for 20 seconds to the light of a 2 kW high-pressure mercury lamp positioned at a distance of 30 cm from the plate. The plate was, then, immersed in water at 70°C for 20 days, and the coated film was examined. The results are shown in Table 22.

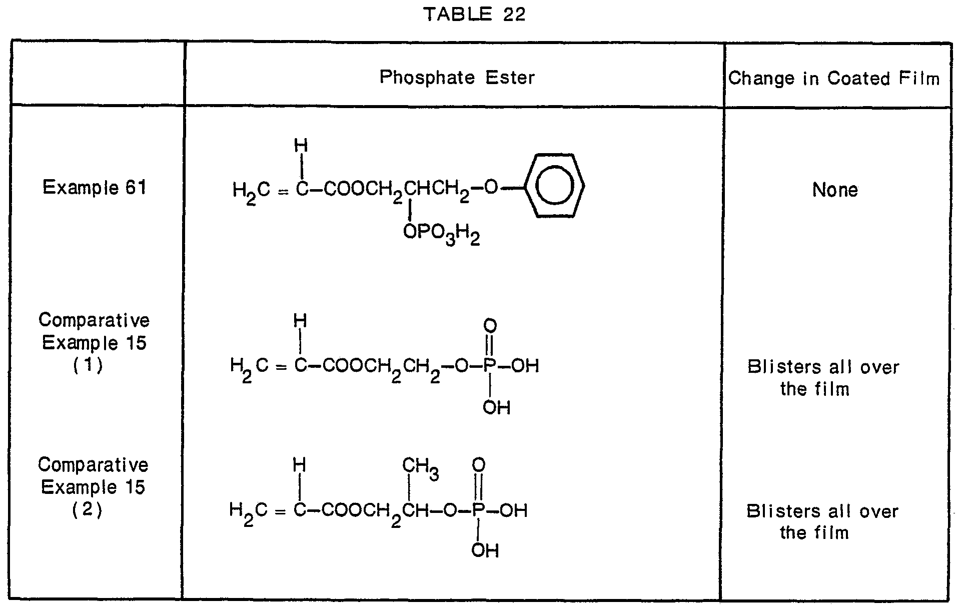

## Claims

1. An adhesive composition comprising 100 parts by weight of a polymerizable monomer comprising
(a) 0.5 to 50 parts by weight of a compound represented by one of formulae I and II: 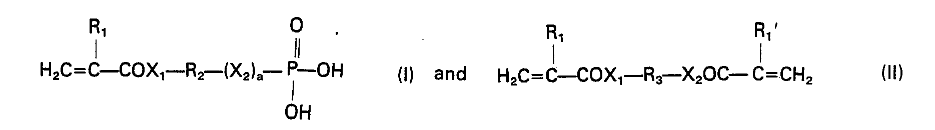 where
each of R₁ and R₁' is hydrogen or methyl;
R₂ is (i) a bivalent hydrocarbon group having 5 to 30 carbon atoms optionally having halogen, hydroxy, amino and/or carboxy substituent(s), or (ii) a bivalent organic group having 5 to 30 carbon atoms composed of two to seven hydrocarbon residues bonded to one another by one or more ether, thioether, ester, thioester, thiocarbonyl, amide, urethane, carbonyl and/or sulfonyl linkages, each of the hydrocarbon residues having 1 to 29 carbon atoms and at least one of them having four or more carbon atoms, and optionally having halogen, hydroxy, amino or carboxy substituents;
each of X₁ and X₂ is ―O―, ―S― or ―NH―;
a is 0 or 1, and
R₃ is a group of the formula 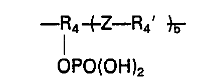 having 6 to 40 carbon atoms, where each of R₄ and R₄' is a hydrocarbon group having 1 to 29 carbon atoms optionally having as substituent(s) halogen, hydroxy, amino or carboxy, b is 0, 1, 2 or 3, the R₄'s, if more than one are present, being the same or different, and at lest one of R₄ and R₄' having at lest three carbon atoms, and Z is -0-, -COO- or -NH-; and
(b) 50 to 99.5 parts by weight of a vinyl monomer copolymerizable with the said compound; and
0.01 to 20 parts by weight of a curing agent.

2. A composition as claimed in Claim 1, in which the said compound is of formula I.

3. A composition as claimed in Claim 1, in which the said compound is of formula II.

4. A composition as claimed in Claim 1 or 2, in which R₂ is represented by the formuta―R₅(̵Y―R₅')̵_{c} where each of R₅ and R₅' is a hydrocarbon group having 1 to 29 carbon atoms, optionally having halogen, hydroxy, amino and/or carboxy substituent(s), at least one of R₅ and Rₛ' having at least four carbon atoms, 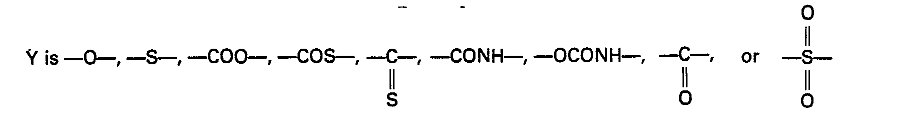 and c is an integer of 1 to 6, the Rs's, when two or more are present, being the same or different.

5. A composition as claimed in Claim 2, in which R₂ is represented by the formula 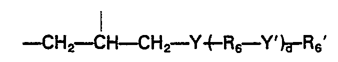 where each of Y and Y', which are the same or different, are as defined for Y in Claim 4, d is 1, 2 or 3, and each of R₆ and Rₛ' is a hydrocarbon group having 4 to 27 carbon atoms, and optionally having halogen, hydroxy, amino and/or carboxy substituent(s), at least one of R₆ and R₆' having at least four carbon atoms.

6. A composition as claimed in Claim 1 or 2, in which X₁ and X₂ stand for ―O― and a is 1.

7. A composition as claimed in Claim 4, in which X₁ and X₂ both stand for ―O― and a is 1.

8. A composition as claimed in Claim 5, in which X₁ and X₂ both stand for ―O― and a is 1.

9. A composition as claimed in Claim 6, in which R₂ is represented by the formula 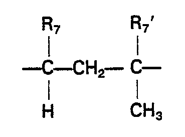 is a hydrogen atom or hydrocarbon or halogenated hydrocarbon group having 1 to 4 carbon atoms.

10. A composition as claimed in Claim 6, in which R₂ is represented by the formula 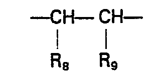 where R₈ stands for hydrogen or methyl and Rg stands for a hydrocarbon or halogenated hydrocarbon group having 3 to 28 carbon atoms.

11. A composition as claimed in Claim 6, in which R₂ is represented by the formula 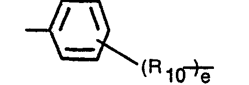 where Rₗₒ stands for a hydrocarbon or halogenated hydrocarbon group having 1 to 12 carbon atoms and e is 0 or 1.

12. A composition as claimed in Claim 6, in which R₂ is represented by the formula (̵CH₂)̵_{f} where fis an integer of from 5 to 20.

13. A composition as claimed in Claim 6, in which R₂ is represented by the formula 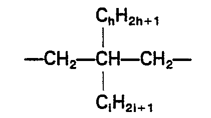 where h is an integer of 1 to 5, and i is 0, 1, 2 or 3, the sum of h nd i being at least 2.

14. A composition as claimed in Claim 6, in which R₂ is represented by the formula

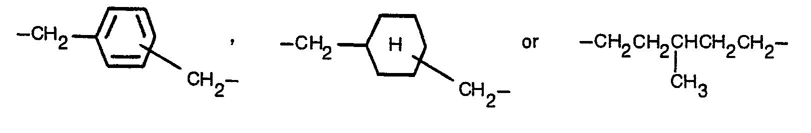

15. A composition as claimed in Claim 6, in which R₂ is represented by the formula 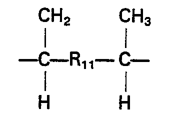 or halogenated hydrocarbon group having 2 to 6 carbon atoms.

16. A composition as claimed in Claim 6, in which R₂ is represented by the formula

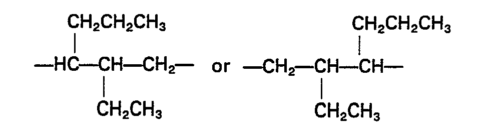

17. A composition as claimed in Claim 10, in which R₂ is represented by the formula 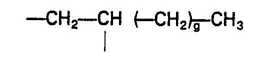 where g is an integer of 2 to 20, or by the formula

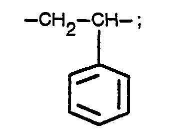

18. A composition as claimed in Claim 12, in which R₂ is represented by the formula (̵CH₂)̵ⱼ where; is an integer of 5 to 12.

19. A composition as claimed in Claim 14, in which R₂ is represented by the formula

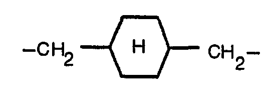

20. A composition as claimed in any one of Claims 9 to 19, in which R₁ is a methyl group.

21. A composition as claimed in Claim 7, in which R₂ is represented by the formula 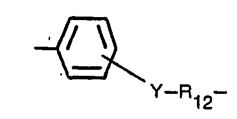 where R₁₂ is a hydrocarbon or halogenated hydrocarbon group having 1 to 12 carbon atoms.

22. A composition as claimed in Claim 21, in which Y stands for―O― or―COO―.

23. A composition as claimed in Claim 7, in which R₂ is represented by the formula 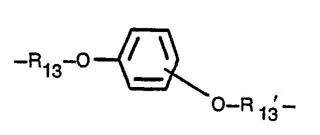 where each of R,₃ and R,₃', which are the same or different, is a hydrocarbon or halogenated hydrocarbon group having 1 to 6 carbon atoms.

24. A composition as claimed in Claim 7, in which R₂ is represented by the formula 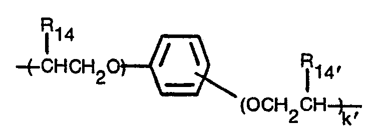 where each of R₁₄ and R₁₄' is hydrogen or methyl, and each of k and k' is 2 or 3.

25. A composition as claimed in Claim 7, in which R₂ is represented by the formula

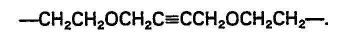

26. A composition as claimed in Claim 23, in which R₂ is represented by the formula

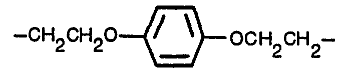

27. A composition as claimed in Claim 23, in which R₂ is represented by the formula

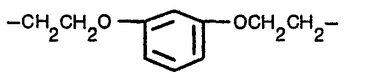

28. A composition as claimed in Claim 23, in which R₂ is represented by the formula 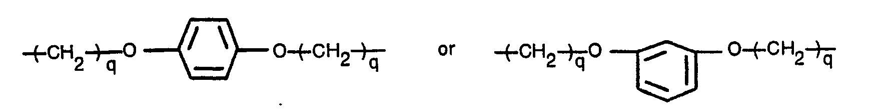 where q is 3 or 4.

29. A composition as claimed in any one of Claims 21 to 28, in which R, stands for a methyl group.

30. A composition as claimed in Claim 8, in which R₂ is represented by the formula 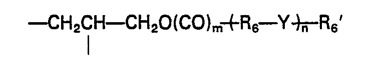 where R₆ and R₆' are as defined in Claim 5, m is 0 or 1, and n is 0, 1, 2 or 3.

31. A composition as claimed in Claim 30, in which at least one of R₆ and Rₛ' is an aromatic hydrocarbon group.

32. A composition as claimed in Claim 30 or 31, in which R₁ is a methyl group.

33. A composition as claimed in Claim 1, in which the said compound is a compound represented by formula II.

34. A composition as claimed in Claim 33, in which R₄ is represented by the formula -CH₂CHCH₂-.

35. A composition as claimed in any one of Claims 1 to 34 in which the vinyl monomer is a (meth)acrylate monomer, styrene monomer or vinyl acetate.

36. A composition as claimed in Claim 35, in which the monomer is a mono- or tetra-functional (meth)acrylate represented by the formula 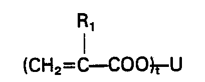 in which R₁ stands for H or CH₃, U stands for an organic residue having 1 to 50 carbon atoms, and t is an integer of 1 to 4.

37. A composition as claimed in Claim 35, in which the monomer is represented by the formula: 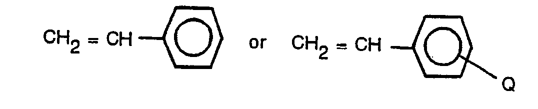 where Q stands for a halogen atom or a hydrocarbon group having 1 to 6 carbon atoms.

38. A composition as claimed in any one of Claims 1 to 37 further comprising a volatile organic solvent having a boiling point up to 150°C at 760 Torr (10.1 kPa), the amount of solvent by weight being not more than 300 times that of the polymerizable monomer.

39. A composition as claimed in any one of Claims 1 to 38, in which the curing agent is an agent of the redox type.

40. A composition as claimed in Claim 39, in which the curing agent is a ternary agent composed of (a) a salt of an organic sulfinic acid, (b) an amine or amine salt, and (c) a peroxide.

41. A composition as claimed in any one of Claims 1 to 40, further comprising 20 to 500 parts by weight of a filler.

42. A composition as claimed in Claim 41, in which the filler is a silanated inorganic filler or an organic-inorganic composite filler.

43. For use in applying to the wall of a tooth cavity before filling the cavity with a dental composite resin, a dental adhesive composition comprising:
100 parts by weight of a polymerizable monomer comprising (a) 0.5 to 50 parts by weight of a compound represented by general formula I or II set forth in claim 1, where R₁, R₁', R₂, X₁, X₂ and R₃ are as defined in claim 1, and (b) 50 to 99.5 parts by weight of a vinyl monomer copolymerizable with the said compound; and 0.01 to 20 parts by weight of a curing agent.

44. For use in applying to the wall of a tooth cavity before filling the cavity with a dental composite resin, a composition as set forth in claim 43, in which the compound is of formula I and R₂ stands for (a) a hydrocarbon group having 5 to 30 carbon atoms optionally having halogen, hydroxy, amino or carboxy substituent(s), or (b) a group having 5 to 30 carbon atoms and composed of two to seven hydrocarbon residues bonded to one another by one or more ether, thioether, ester, thioester, thiocarbonyl, amide, urethane, carbonyl and/or sulfonyl linkages, each of the said hydrocarbon residues having 1 to 29 carbon atoms and at least one of them having four or more carbon atoms, and optionally having halogen, hydroxy, amino and/or carboxy substituent(s).

45. For use in applying to the wall of a tooth cavity before filling the cavity with a dental composite resin, a composition as set forth in claim 44, in which R₂ is represented by the formula ―R₅(̵Y―R₅')̵ where each of Rₛ and Rₛ' is a hydrocarbon group having 1 to 29 carbon atoms, optionally having halogen, hydroxy, amino and/or carboxy substituent(s), at least one of R₅ and R₅' having at least four carbon atoms, and Y and c are as defined in claim 4, the R₃'s when two or more are present being the same or different.

46. For use in applying to the wall of a tooth cavity before filling the cavity with a dental composite resin, a composition as set forth in Claim 44, in which R₂ is represented by the formula 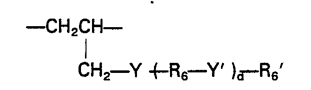 where d, Y and Y' are as in Claim 5 and each of R₆ and R₆' is a hydrocarbon group having 4 to 27 carbon atoms, optionally having halogen, hydroxy, amino or carboxy substituent(s).

47. For use in applying to the wall of a tooth cavity before filling the cavity with a dental composite resin, a composition as set forth in Claim 44, 45 or 46, in which X, and X₂ both stand for -0- and a is 1.

48. For use in applying to the wall of a tooth cavity before filling the cavity with a dental composite resin, a composition as set forth in Claim 47, in which R₂ is represented by the formula (̵CH₂)̵_{f} where fis an integer of 5 to 20.

49. For use in applying to the wall of a tooth cavity before filling the cavity with a dental composite resin, a composition as set forth in Claim 47, in which R₂ is represented by the formula 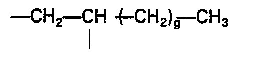 where g is an integer of 2 to 20, or the formula

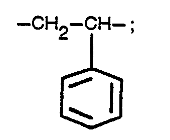

50. For use in applying to the wall of a tooth cavity before filling the cavity with a dental composite resin, a composition as claimed in any of Claims 7 to 11, 13 to 16 and 18 to 40.

51. For use in bonding either a tooth and a dental restorative material or bonding dental restorative materials to each other, a dental adhesive composition comprising 100 parts by weight of a polymerizable monomer as claimed in any one of Claims 43 to 50, 20 to 500 parts by weight of a filler and 0.01 to 20 parts by weight of a curing agent.

52. For use in bonding either a tooth and a dental restorative material or bonding dental restorative materials to each other, a dental adhesive composition as claimed in Claim 51, in which the filler is a silanated inorganic filler, or an organic-inorganic composite filler.

53. A phosphate monoester of the general formula 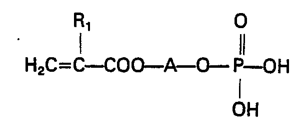 where R₁ stands for hydrogen, or a methyl group, and A stands for one of formulae 3 to 6: 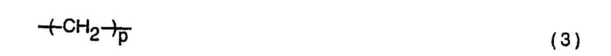 where p is an integer of 5 to 12; 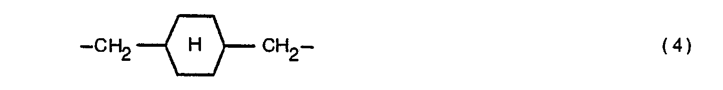 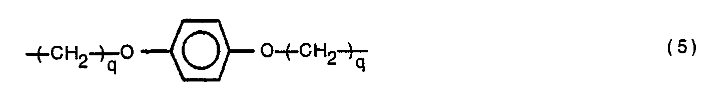 where q is an integer of 2 to 4; 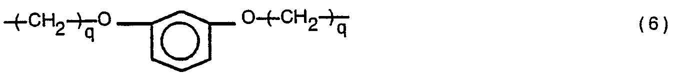 where q is an integer of 2 to 4.

54. For use in coating a tooth surface for protecting teeth against dental caries, a pit and fissure sealant composition comprising 100 parts by weight of a polymerizable monomer as claimed in any one of Claims 43 to 50, and 0.01 to 20 parts by weight of a curing agent.

## Patentansprüche

1. Klebmittel-Zusammensetzung, enthaltend 100 Gew.-Teile eines polymerisierbaren Monomeren, welches
(a) 0,5 bis 50 Gew.-Teile einer Verbindung einer der Formeln I und II 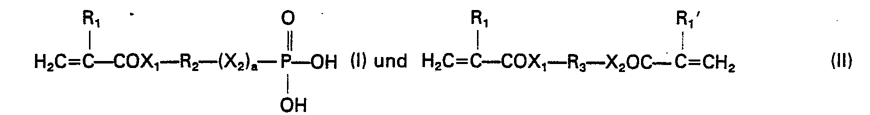 worin
jeder der Reste R₁, und R₁' für Wasserstoff oder Methyl steht;
R₂ (i) eine zweiwertige Kohlenwasserstoffgruppe mit 5 bis 30 Kohlenstoffatomen, die gegebenenfalls einen oder mehr Halogen-, Hydroxy-, Amino- und/oder Carboxy-Substituenten aufweist, oder (ii) eine zweiwertige organische Gruppe mit 5 bis 30 Kohlenstoffatomen, bestehend aus 2 bis 7 Kohlenwasserstoffresten, die über eine oder mehr Ether-, Thioether-, Ester-, Thioester-, Thiocarbonyl-, Amid-, Urethan-, Carbonyl- und/oder Sulfonyl-Verknüpfungen aneinander gebunden sind, wobei jeder der Kohlenwasserstoffreste 1 bis 29 Kohlenstoffatome enthält und mindestens einer davon 4 oder mehr Kohlenstoffatome aufweist, die gegebenfalls Halogen-, Hydroxy-, Amino- oder Carboxysubstituenten trägt, bedeutet;
jeder der Reste X₁ und X₂ für ―O―, ―S― oder ―NH― steht;
a für 0 oder 1 steht, und
R₃ eine Gruppe der Formel 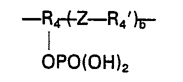 bedeutet, die 6 bis 40 Kohlenstoffatome aufweist, wobei jeder der Reste R₄ und R₄' eine Kohlenwasserstoffgruppe mit 1 bis 29 Kohlenstoffatomen bedeutet, die gegebenenfalls einen oder mehr Substituenten aus der Gruppe Halogen, Hydroxy, Amino oder Carboxy aufweist, b für 0, 1, 2 oder 3 steht, bei Vorliegen von mehr als einem Rest R₄ diese Reste gleich oder verschieden sind und mindestens einer der Reste R₄ und R₄' mindestens drei Kohlenstoffatomen aufweist und Z für -0-, -COO- oder -NH- steht; und
(b) 50 bis 99,5 Gew.-Teile eines mit dieser Verbindung copolymerisierbaren Vinylmonomeren umfaßt, und
0,01 bis 20 Gew.-Teile eines Härtungsmittels.

2. Zusammensetzung nach Anspruch 1, in der die Verbindung der Formel genügt.

3. Zusammensetzung nach Anspruch 1, in der die Verbindung der Formel II genügt.

4. Zusammensetzung nach Anspruch 1 oder 2, in der R₂ durch die Formel ―R₅(̵Y―R₅')̵_{c} dargestellt wird, worin jeder der Reste Rₛ und Rₛ' eine Kohlenwasserstoffgruppe mit 1 bis 29 Kohlenstoffatomen, die gegebenenfalls einen oder mehr Halogen-, Hydroxy-, Amino- und/oder Carboxysubstituenten aufweist, bedeutet, mindestens einer der Reste R₅ und R₅' mindestens 4 Kohlenstoffatome aufweist, Y für ―O―, 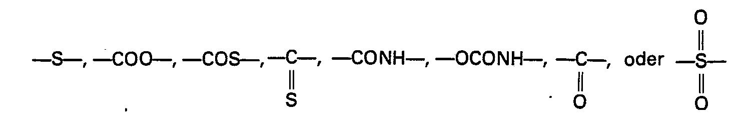 und c eine ganze Zahl von 1 bis 6 ist, wobei bei Anwesenheit von zwei oder mehr Resten R₅ diese gleich oder verschieden sind.

5. Zusammensetzung nach Anspruch 2, in der R₂ durch die Formel 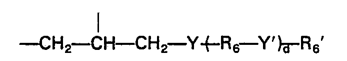 dargestellt wird, worin Y und Y' gleich oder verschieden sind und die für 0, 1, 2 oder 3 steht und jeder der Reste R₆ und Rₛ' eine Kohlenwasserstoffgruppe mit 4 bis 27 Kohlenstoffatomen bedeutet, und gegebenenfalls einen oder mehr Halogen-, Hydroxy-, Amino- und/oder Carboxysubstituenten aufweist, wobei mindestens einer der Reste R₆ und R₆' mindestens 4 Kohlenstoffatome umfaßt.

6. Zusammensetzung nach Anspruch 1 oder 2, worin beide Reste X₁ und X₂ für ―O― stehen und a 1 bedeutet.

7. Zusammensetzung nach Anspruch 4, in der beide Reste X₁ und X₂ für―O― stehen und a für 1 steht.

8. Zusammensetzung nach Anspruch 5, in der beide Reste X₁ und X₂ für―O―stehen und a für 1 steht.

9. Zusammensetzung nach Anspruch 6, in der R₂ durch die Formel 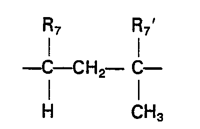 dargestellt wird,
worin jeder der Reste R₇ und R₇' ein Wasserstoffatom, eine Kohlenwasserstoff- oder halogenierte Kohlenwasserstoffgruppe mit 1 bis 4 Kohlenstoffatomen bedeutet.

10. Zusammensetzung nach Anspruch 6, in der R₂ durch die Formel 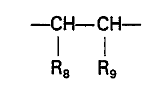 dargestellt wird, in der R₈ für Wasserstoff oder Methyl und R₉ für eine Kohlenwasserstoffgruppe oder halogenierte Kohlenwasserstoffgruppe mit 3 bis 28 Kohlenstoffatomen steht.

11. Zusammensetzung nach Anspruch 6, in der R₂ durch die Formel 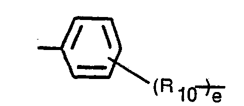 dargestellt wird, in der R₁₀für eine Kohlenwasserstoff- oder halogenierte Kohlenwasserstoffgruppe mit 1 bis 12 Kohlenstoffatomen steht und e für 0 oder 1 steht.

12. Zusammensetzung nach Anspruch 6, in der R₂ durch die Formel (̵CH₂)̵ dargestellt wird, in der f eine ganze Zahl von 5 bis 20 bedeutet.

13. Zusammensetzung nach Anspruch 6, in der R₂ durch die Formel 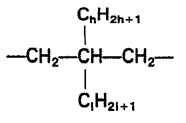 dargestellt wird, in der h eine ganze Zahl von 1 bis 5 und i 0, 1, 2 oder 3 bedeuten, wobei die Summe von h und i mindestens 2 ist.

14. Zusammensetzung nach Anspruch 6, in der R₂ durch die Formel 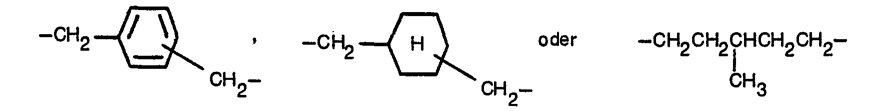 dargestellt wird.

15. Zusammensetzung nach Anspruch 6, in der R₂ durch die Formel 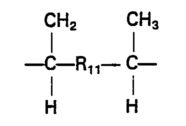 dargestellt wird, in der R₁₁ eine Kohlenwasserstoffgruppe oder halogenierte Kohlenwasserstoffgruppe mit 2 bis 6 Kohlenstoffatomen bedeutet.

16. Zusammensetzung nach Anspruch 6, in der R₂ durch die Formel 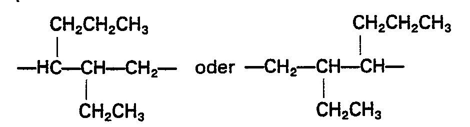 dargestellt wird.

17. Zusammensetzung nach Anspruch 10, in der R₂ durch die Formel 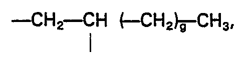 in der g eine ganze Zahl von 2 bis 20 bedeutet, oder durch die Formel 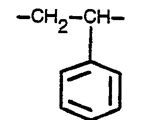 dargestellt wird.

18. Zusammensetzung nach Anspruch 12, in der R₂ durch die Formel (̵CH₂)̵ⱼ dargestellt wird, in der j eine ganze Zahl von 5 bis 12 bedeutet.

19. Zusammensetzung nach Anspruch 14, in der R₂ durch die Formel 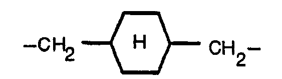 dargestellt wird.

20. Zusammensetzung nach einem der Ansprüche 9 bis 19, in der R, eine Methylgruppe ist.

21. Zusammensetzung nach Anspruch 7, in der R₂ durch die Formel 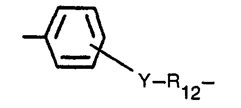 dargestellt wird, in der R₁₂ eine Kohlenwasserstoffgruppe oder halogenierte Kohlenwasserstoffgruppe mit 1 bis 12 Kohlenstoffatomen bedeutet.

22. Zusammensetzung nach Anspruch 21, in der Y für ―O― oder ―COO― steht.

23. Zusammensetzung nach Anspruch 7, in der R₂ durch die Formel 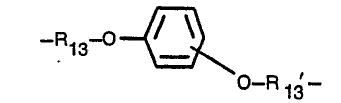 dargestellt wird, in der jeder der Reste R₁₃ und R₁₃', die gleich oder verschieden sind, eine Kohlenwasserstoffgruppe oder halogenierte Kohlenwasserstoffgruppe mit 1 bis 6 Kohlenstoffatomen bedeuten.

24. Zusammensetzung nach Anspruch 7, in der R₂ durch die Formel 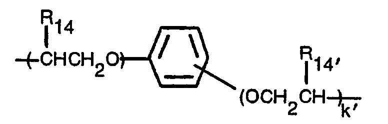 dargestellt wird, in der jeder der Reste R₁₄ und R₁₄' Wasserstoff oder Methyl bedeutet und k und k' jeweils 2 oder 3 bedeuten.

25. Zusammensetzung nach Anspruch 7, in der R₂ durch die Formel 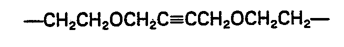 dargestellt wird.

26. Zusammensetzung nach Anspruch 23, in der R₂ durch die Formel 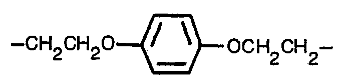 dargestellt wird.

27. Zusammensetzung nach Anspruch 23, in der R₂ durch die Formel 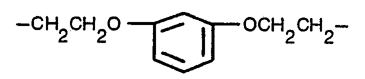 dargestellt wird.

28. Zusammensetzung nach Anspruch 23, in der R₂ durch die Formel 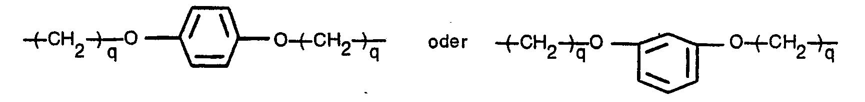 dargestellt wird, worin q für 3 oder 4 steht.

29. Zusammensetzung nach einem der Ansprüche 21 bis 28, in der R₁ für eine Methylgruppe steht.

30. Zusammensetzung nach Anspruch 8, in der R₂ durch die Formel 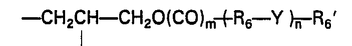 dargestellt wird, worin R₆ und R₆' die in Anspruch 5 gegebene Definition haben, m für oder 1 und n für 0, 1, 2 oder 3 steht.

31. Zusammensetzung nach Anspruch 30, in der mindestens einer der Reste R₆ und Rₛ' eine aromatische Kohlenwasserstoffgruppe bedeutet.

32. Zusammensetzung nach Anspruch 30 oder 31, in der R₁ eine Methylgruppe ist.

33. Zusammensetzung nach Anspruch 1, in der die Verbindung eine Verbindung der Formel II ist.

34. Zusammensetzung nach Anspruch 33, in der R₄ durch die Formel 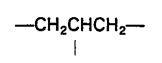 dargestellt wird.

35. Zusammensetzung nach einem der Ansprüche 1 bis 34, in der das Vinylmonomere ein (Meth)acrylat-Monomeres, Styrol-Monomeres oder Vinylacetat ist.

36. Zusammensetzung nach Anspruch 35, in der das Monomere ein mono- oder tetrafunktionelles (Meth)acrylat der Formel 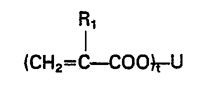 ist, in der R, für H oder CH₃ steht, U für einen organischen Rest mit 1 bis 50 Kohlenstoffatomen steht und t eine ganze Zahl von 1 bis 4 ist.

37. Zusammensetzung nach Anspruch 35, in der das Monomere durch eine der Formeln 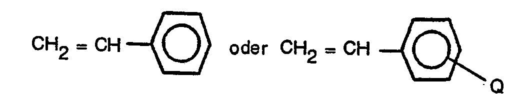 dargestellt wird, worin Q für ein Halogenatom oder eine Kohlenwasserstoffgruppe mit 1 bis 6 Kohlenstoffatomen steht.

38. Zusammensetzung nach einem der Ansprüche 1 bis 37, die außerdem ein flüchtiges organisches Lösungsmittel mit einem Siedepunkt bis 150°C bei 760 Torr (10,1 kPa) enthält, wobei die Gewichtsmenge des Lösungsmittels nicht mehr als das 300-fache der des polymerisierbaren Monomeren ist.

39. Zusammensetzung nach einem der Ansprüche 1 bis 38, in der das Härtungsmittel ein Mittel des Redox-Typs ist.

40. Zusammensetzung nach Anspruch 39, in der das Härtungsmittel ein ternäres Mittel ist, das aus (a) einem Salz einer organischen Sulfinsäure, (b) einem Amin oder einem Aminsalz und (c) einem Peroxid besteht.

41. Zusammensetzung nach einem der Ansprüche 1 bis 40, die zusätzlich 20 bis 500 Gew.-Teile eines Füllstoffes enthält.

42. Zusammensetzung nach Anspruch 41, in der der Füllstoff ein silanierter anorganischer Füllstoff oder ein organisch-anorganischer Verbundfüllstoff ist.

43. Dentale Klebmittel-Zusammensetzung zur Verwendung zum Auftragen auf die Wand einer Zahnkavität vor dem Füllen der Kavität mit einem dentalen Verbundharz, enthaltend:
100 Gew.-Teile eines polymerisierbaren Monomeren, welches (a) 0,5 bis 50 Gew.-Teile einer Verbindung der allgemeinen Formel I oder 11 gemäß Anspruch 1, in der R₁, R₁', R₂, X₁, X₂ und X₃ die in Anspruch 1 gegebene Definition haben, und (b) 50 bis 99,5 Gew.-Teile eines mit dieser Verbindung copolymerisierbaren Vinylmonomeren umfaßt; und 0,01 bis 20 Gew.-Teile eines Härtungsmittels.

44. Zusammensetzung nach Anspruch 43 zur Verwendung zum Auftragen auf die Wand einer Zahnkavität vor dem Füllen der Kavität mit einem dentalen Verbundharz, wobei die Verbindung der Formel I entspricht und R₂ für (a) eine Kohlenwasserstoffgruppe mit 5 bis 30 Kohlenstoffatomen, die gegebenenfalls einen oder mehr Halogen-, Hydroxy-, Amino- oder Carboxy-Substituenten aufweist, oder (b) eine Gruppe mit 5 bis 30 Kohlenstoffatomen steht, die aus 2 bis 7 Kohlenwasserstoffresten, die über eine oder mehr Ether-, Thioether-, Ester-, Thioester-, Thiocarbonyl-, Amid-, Urethan-, Carbonyl- und/oder Sulfonyl-Verknüpfungen aneinander gebunden sind, wobei jeder dieser Kohlenwasserstoffreste 1 bis 29 Kohlenstoffatome aufweist und mindestens einer davon 4 oder mehr Kohlenstoffatome enthält, und gegebenenfalls einen oder mehr Halogen-, Hydroxy-, Amino- und/oder Carboxy-Substituenten aufweist.

45. Zusammensetzung nach Anspruch 44 zur Verwendung zum Auftragen auf die Wand einer Zahnkavität vor dem Füllen der Kavität mit einem dentalen Verbundharz, in der R₂ durch die Formel ―R₅(̵Y―R5')̵_{c} dargestellt wird, in der jeder der Reste R₅ und R₅' eine Kohlenwasserstoffgruppe mit 1 bis 29 Kohlenstoffatomen ist, die gegebenenfalls einen oder mehr Halogen-, Hydroxy-, Amino- und/oder Carboxy-Substituenten aufweist, mindestens einer der Reste R₅ und Rₛ' mindestens 4 Kohlenstoffatome enthält und Y und c die in Anspruch 4 gegebene Definition haben, wobei dann, wenn zwei oder mehr der Reste R₅ vorliegen, diese gleich oder verschieden sind.

46. Zusammensetzung nach Anspruch 44 zur Verwendung zum Auftragen auf die Wand einer Zahnkavität vor dem Füllen der Kavität mit einem dentalen Verbundharz, in der R₂ durch die Formel 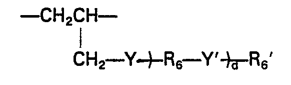 dargestellt wird, in der d, Y und Y' wie in Anspruch 5 definiert sind und jeder der Reste R₆ und R₆' eine Kohlenwasserstoffgruppe mit 4 bis 27 Kohlenstoffatomen bedeutet, die gegebenenfalls einen oder mehr Halogen-, Hydroxy-, Amino- oder Carboxy-Substituenten aufweist.

47. Zusammensetzung nach Anspruch 44, 45 oder 46 zur Verwendung zum Auftragen auf die Wand einer Zahnkavität vor dem Füllen der Kavität mit einem dentalen Verbundharz, in der beide Reste X, und . X₂ für -0- stehen und a für 1 steht.

48. Zusammensetzung nach Anspruch 47 zur Verwendung zum Auftragen auf die Wand einer Zahnkavität vor dem Füllen der Kavität mit einem dentalen Verbundharz, in der R₂ durch die formel (̵CH₂)̵_{f} dargestellt wird, worin f eine ganze Zahl von 5 bis 20 ist.

49. Zusammensetzung nach Anspruch 47 zur Verwendung zum Auftragen auf die Wand einer Zahnkavität vor dem Füllen der Kavität mit einem dentalen Verbundharz, in der R₂ durch die Formel 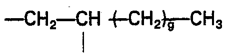 worin g eine ganze Zahl von 2 bis 20 ist, oder durch die Formel 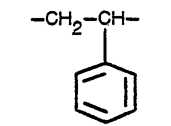 dargestellt wird.

50. Zusammensetzung nach einem der Ansprüche 7 bis 11, 13 bis 16 und 18 bis 40 zur Verwendung zum Auftragen auf die Wand einer Zahnkavität vor dem Füllen der Kavität mit-einem dentalen Verbundharz.

51. Dentale Klebmittel-Zusammensetzung, enthaltend 100 Gew.-Teile eines polymerisierbaren Monomeren gemäß einem der Ansprüche 43 bis 50, 20 bis 500 Gew.-Teile eines Füllstoffes und 0,01 bis 20 Gew.-Teile eines Härtungsmittels, zur Verwendung zum Verbinden eines Zahns mit einem dentalen reparaturmaterial oder zum gegenseitigen Verbinden von dentalen Reparaturmaterialien.

52. Dentale Klebmittel-Zusammensetzung nach Anspruch 51, in der der Füllstoff ein silanierter anorganischer Füllstoff oder ein organisch-anorganischer Verbundfüllstoff ist, zur Verwendung zum Verbinden eines Zahns mit einem dentalen Reparaturmaterial oder zum gegenseitigen Verbinden von dentalen Reparaturmaterialien.

53. Phosphorsäure-monoester der allgemeinen Formel 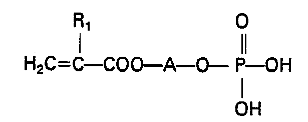 in der R₁ für Wasserstoff oder eine Methylgruppe steht, und A für eine der Formeln (3) bis (6) steht: 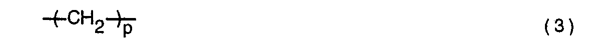 in der p eine ganze Zahl von 5 bis 12 ist; 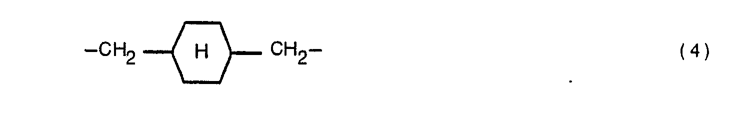 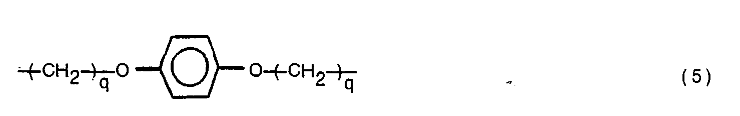 in der q eine ganze Zahl von 2 bis 4 ist; 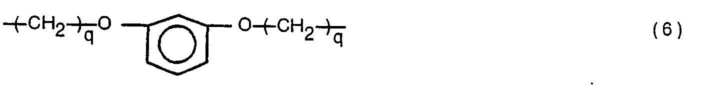 in der q eine ganze Zahl von 2 bis 4 ist.

54. Zusammensetzung zum Abdichten von Kavitäten und Spalten zur Verwendung zum Überziehen einer Zahnoberfläche zum Schutz der Zähne gegen Zahnkaries, die 100 Gew.-Teile eines polymerisierbaren Monomeren gemäß einem der Ansprüche 43 bis 50 und 0,01 bis 20 Gew.-Teile eines Härtungsmittels enthält.

## Revendications

1. Une composition adhésive comprenant 100 parties en poids d'un monomère polymérisable comprenant
(a) 0,5 à 50 parties en poids d'un composé représenté par l'une des formules 1 et Il: 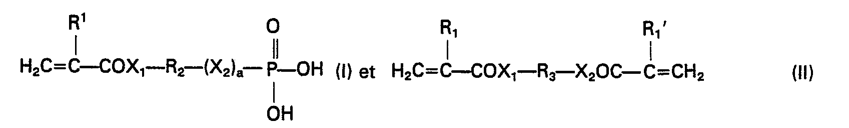 dans lesquelles chaque R₁ et R₁' est un hydrogène ou un méthyle;
R₂ est (i) un groupe hydrocarboné bivalent ayant 5 à 30 atomes de carbone et ayant éventuellement un ou plusieurs susbtituants halogène, hydroxy, amino et/ou carboxy ou (ii) un groupe organique bivalent ayant 5 à 30 atomes de carbone composé de 2 à 7 restes hydrocarbonés unis entre eux par une ou plusieurs liaisons éther, thioéther, ester, thioester, thiocarbonyle, amide, uréthane, carbonyle et/ou sulfonyle, chacun des restes hydrocarbonés ayant 1 à 29 atomes de carbone et au moins un d'entre eux ayant 4 atomes de carbone ou plus, et portant éventuellement des substituants halogène, hydroxy, amino ou carboxy; chacun de X₁ et X₂ est ―O―, ―S― ou ―NH―;
a est 0 ou 1, et
R₃ est un groupe de formule 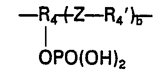 ayant 6 à 40 atomes de carbone et dans lequel chacun de R₄ et R₄' est un groupe hydrocarboné ayant 1 à 29 atomes de carbone et ayant éventuellement un ou plusieurs substituants halogène, hydroxy, amino ou carboxy, b est 0,1,2 ou 3, les R₄' s'ils sont plusieurs étant semblables ou différents et au moins un de R₄ et R₄' ayant au moins 3 atomes de carbone, et Z est -O-, -COO- ou -NH-; et
(b) 50 à 99,5 parties en poids d'un monomère vinylique copolymérisable avec ledit composé; et
0,01 à 20 parties en poids d'un agent de durcissement.

2. Une composition comme revendiqué dans la revendication 1 dans laquelle ledit composé répond à la formule I.

3. Une composition comme revendiqué dans la revendication 1 dans laquelle ledit composé répond à la formule Il.

4. Une composition comme revendiqué dans la revendication 1 ou 2 dans laquelle R₂ est représenté par la formule ―R5(̵Y―R₅')̵_{c} dans laquelle chacun de R₅ et Rₛ' est un groupe hydrocarboné ayant 1 à 29 atomes de carbone, ayant éventuellement un ou plusieurs substituants halogène, hydroxy, amino et/ou carboxy, au moins un de R₅ und R₅' ayant au moins 4 atomes de carbone, Y est ―O―, ―S―,―COO―, 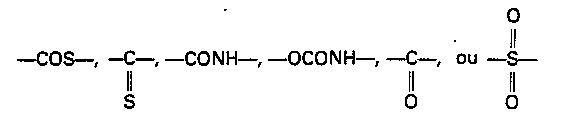 et c est un entier de 1 à 6, les Rₛ' lorsque deux ou plus d'entre eux sont présents étant semblables ou différents.

5. Une composition comme revendiqué dans la revendication 2 dans laquelle R₂ est représenté par la formule: 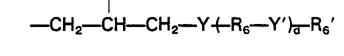 dans laquelle chacun de Y et Y', qui sont semblables ou différents, sont comme défini pour Y dans la revendication 4, d est 0, 1, ou 3 et chacun de R₆ et R₆' est un groupe hydrocarboné ayant 4 à 27 atomes de carbone et ayant éventuellement un ou plusieurs substituants halogène, hydroxy, amino et/ou carboxy, au moins un de R₆ et R₆' ayant au moins 4 atomes de carbone.

6. Une composition comme revendiqué dans la revendication 1 ou 2 dans laquelle X₁ et X₂ représentent tous deux ―O― et a est 1.

7. Une composition comme revendiqué dans la revendication 4 dans laquelle X₁ et X₂ représentent tous deux -0- et a est 1.

8. Une composition comme revendiqué dans la revendication 5 dans laquelle X₁ et X₂ représentent tous deux ―O― et a est 1.

9. Une composition comme revendiqué dans la revendication 6 dans laquelle R₂ est représenté par la formule 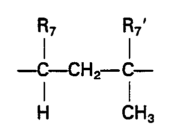 dans laquelle chacun de R₇ et R₇' est un atome d'hydrogène ou un groupe hydrocarboné ou hydrocarboné halogéné et ayant 1 à 4 atomes de carbone.

10. Une composition comme revendiqué dans la revendication 6 dans laquelle R₂ est représenté par la formule 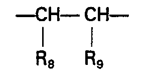 dans laquelle R₈ représente un hydrogène ou un méthyle et Rg représente un groupe hydrocarboné ou hydrocarboné halogéné ayant 3 à 28 atomes de carbone.

11. Une composition comme revendiqué dans la revendication 6 dans laquelle R₂ est représenté par la formule: 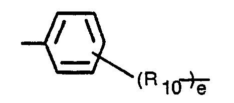 dans laquelle R₁₀ représente un groupe hydrocarboné ou un groupe hydrocarboné halogéné ayant à 12 atomes de carbone et e est 0 ou 1.

12. Une composition comme revendiqué dans la revendication 6 dans laquelle R₂ est représenté par la formule (̵CH₂)̵_{f} dans laquelle f est un entier de 5 à 20.

13. Une composition comme revendiqué dans la revendication 6 dans laquelle R₂ est représenté par la formule 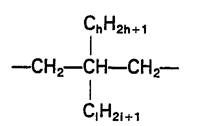 dans laquelle h est un entier de 1 à 5 et i est 0, 1, 2 ou 3, la somme de h et i étant d'au moins 2.

14. Une composition comme revendiqué dans la revendication 6 dans laquelle R₂ est représenté par la formule

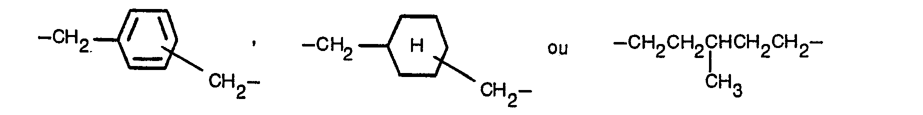

15. Une composition comme revendiqué dans la revendication 6 dans laquelle R₂ est représenté par la formule 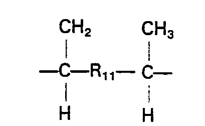 dans laquelle R₁₁ est un groupe hydrocarboné ou hydrocarboné halogéné ayant 2 à 6 atomes de carbone.

16. Une composition comme revendiqué dans la revendication 6 dans laquelle R₂ est représenté par la formule

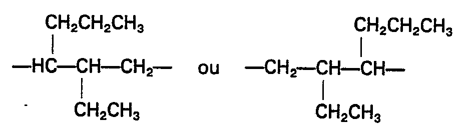

17. Une composition comme revendiqué dans la revendication 10 dans laquelle R₂ est représenté par la formule 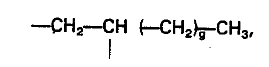 dans laquelle g est un entier de 1 à 20 ou par la formule

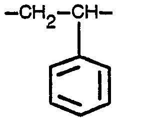

18. Une composition comme revendiqué dans la revendication 12 dans laquelle R₂ est représenté par la formule (̵CH₂)̵ⱼ dans laquelle j est un entier de 5 à 12.

19. Une composition comme revendiqué dans la revendication 14 dans laquelle R₂ est représenté par la formule

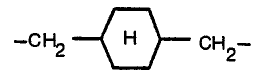

20. Une composition comme revendiqué dans l'une quelconque des revendications 9 à 19 dans laquelle R₁ est un groupe méthyle.

21. Une composition comme revendiqué dans la revendication 7 dans laquelle R₂ est représenté par la formule 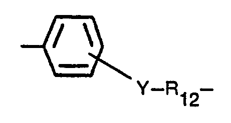 dans laquelle R₁₂ est un groupe hydrocarboné ou hydrocarboné halogéné ayant 1 à 12 atomes de carbone.

22. Une composition comme revendiqué dans la revendication 21 dans laquelle Y représente ―O― ou -COO-.

23. Une composition comme revendiqué dans la revendication 7 dans laquelle R₂ est représenté par la formule 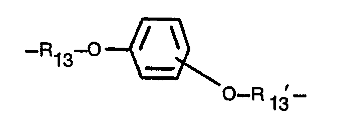 dans laquelle chacun de R₁₃ et R,₃', qui sont semblables ou différents, est un groupe hydrocarboné ou hydrocarboné halogéné ayant 1 à 6 atomes de carbone.

24. Une composition comme revendiqué dans la revendication 7 dans laquelle R₂ est représenté par la formule: 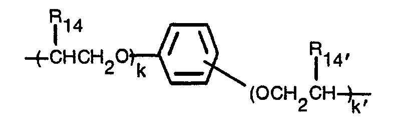 dans laquelle chacun de R₁₄ et R₁₄' est un hydrogène ou un méthyle et chacun de k et k' est 2 ou 3.

25. Une composition comme revendiqué dans la revendication 7 dans laquelle R₂ est représenté par la formule:

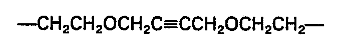

26. Une composition comme revendiqué dans la revendication 23 dans laquelle R₂ est représenté par la formule:

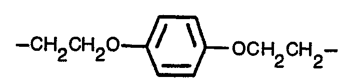

27. Une composition comme revendiqué dans la revendication 23 dans laquelle R₂ est représenté par la formule:

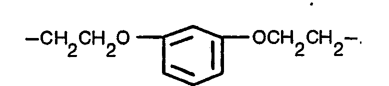

28. Une composition comme revendiqué dans la revendication 23 dans laquelle R₂ est représenté par la formule: 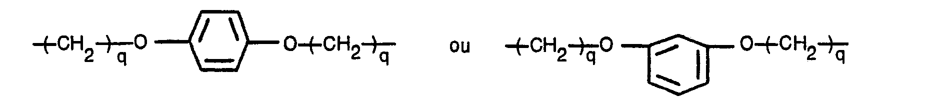 dans laquelle q est 3 ou 4.

29. Une composition comme revendiqué dans l'une quelconque des revendications 21 à 28 dans laquelle R₁ représente un groupe méthyle.

30. Une composition comme revendiqué dans la revendication 8 dans laquelle R₂ est représenté par la formule: 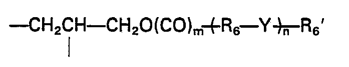 dans laquelle R₆ et R₆' sont comme défini dans la revendication 5, m est 0 ou 1 et n est 0, 1, 2 ou 3.

31. Une composition comme revendiqué dans la revendication 30 dans laquelle au moins un de R₆ et R₆' est un groupe hydrocarboné aromatique.

32. Une composition comme revendiqué dans la revendication 30 ou 31 dans laquelle R₁ est un groupe méthyle.

33. Une composition comme revendiqué dans la revendication 1 dans laquelle ledit composé est un composé représenté par la formule Il.

34. Une composition comme revendiqué dans la revendication 33 dans laquelle R₄ est représenté par la formule

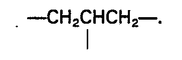

35. Une composition comme revendiqué dans l'une quelconque des revendications 1 à 34 dans laquelle le monomère vinylique est un (méth)acrylate monomère, le styrène monomère ou l'acétate de vinyle.

36. Une composition comme revendiqué dans la revendication 35 dans laquelle le monomère est un (méth)acrylate mono- ou tétra-fonctionnel représenté par la formule 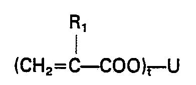 dans laquelle R, représente H ou CH₃, U représente un reste organique ayant 1 à 50 atomes de carbone et t est un entier de 1 à 4.

37. Une composition comme revendiqué dans la revendication 35 dans laquelle le monomère est représenté par la formule: 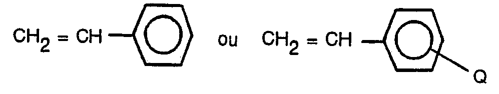 dans laquelle Q représente un atome d'halogène ou un groupe hydrocarboné ayant 1 à 6 atomes de carbone.

38. Une composition comme revendiqué dans l'une quelconque des revendications 1 à 37 comprenant de plus un solvant organique volatil ayant un point d'ébullition d'au plus 150°C à 760 torrs (10,1 kPa) la quantité de solvant exprimée en poids ne dépassant pas 300 fois celle du monomère polymérisable.

39. Une composition comme revendiqué dans l'une quelconque des revendications 1 à 38 dans laquelle l'agent de durcissement est un agent du type redox.

40. Une composition comme revendiqué dans la revendication 39 dans laquelle l'agent de durcissement est un agent ternaire composé de (a) un sel d'un acide sulfinique organique, (b) une amine ou un sel d'amine et (c) un peroxyde.

41. Une composition comme revendiqué dans l'une quelconque des revendications 1 à 40 comprenant de plus 20 à 500 parties en poids d'une charge.

42. Une composition comme revendiqué dans la revendication 41 dans laquelle la charge est une charge organique silanisée ou une charge composite organique-minérale.

43. Pour l'emploi dans l'application à la paroi d'une cavité dentaire avant obturation de la cavité avec une résine composite dentaire, une composition adhésive dentaire comprenant:
100 parties en poids d'un monomère polymérisable comprenant (a) 0,5 à 50 parties en poids d'un composé représenté par les formules générales 1 ou Il indiquées dans la revendication 1, dans lesquelles R₁, R₁', R₂, X₁, X₂ et R₃ sont comme défini dans la revendication 1 et (b) 50 a 99,5 parties en poids d'un monomère vinylique copolymérisable avec ledit composé; et 0,01 à 20 parties en poids d'un agent de durcissement.

44. Pour l'emploi dans l'application à la paroi d'une cavité dentaire avant obturation de la cavité avec une résine composite dentaire, une composition comme indiqué dans la revendication 43 dans laquelle le composé répond à la formule et R₂ représente (a) un groupe hydrocarboné ayant 5 à 30 atomes de carbone ayant éventuellement un ou plusieurs substituants halogène, hydroxy, amino ou carboxy ou (b) un groupe ayant 5 à 30 atomes de carbone et composé de 2 à 7 restes hydrocarbonés unis l'un à l'autre par une ou plusieurs liaisons éther, thioéther, ester, thioester, thiocarbonyle, amide, uréthane, carbonyle et/ou sulfonyle, chacun desdits restes hydrocarbonés ayant 1 à 29 atomes de carbone et au moins un d'entre eux ayant 4 atomes de carbone ou plus et ayant éventuellement un ou plusieurs substituants halogène, hydroxy, amino et/ou carboxy.

45. Pour l'emploi dans l'application à la paroi d'une cavité dentaire avant obturation de la cavité avec une résine composite dentaire, une composition comme indiqué dans la revendication 44 dans laquelle R₂ est représenté par la formule―_{R}(̵Y―_{R}')̵_{c} dans laquelle chacun de R₅ et R₅' est un groupe hydrocarboné ayant 1 à 29 atomes de carbone et ayant éventuellement un ou plusieurs substituants halogène, hydroxy, amino et/ou carboxy, au moins un de Rₛ et Rₛ' ayant au moins 4 atomes de carbone et Y et c sont comme défini dans la revendication 4, les Rₛ', lorsque deux ou plus sont présents, étant semblables ou différents.

46. Pour l'emploi dans l'application à la paroi d'une cavité dentaire avant obturation de la cavité avec une résine composite dentaire, une composition comme indiqué dans la revendication 44 dans laquelle R₂ est représenté par la formule 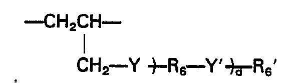 dans laquelle d, Y et Y' sont comme dans la revendication 5 et chacun de R₆ et R₆' est un groupe hydrocarboné ayant 4 à 27 atomes de carbone, ayant éventuellement un ou plusieurs substituants halogène, hydroxy, amino ou carboxy.

47. Pour l'emploi dans l'application à la paroi d'une cavité dentaire avant obturation de la cavité avec une résine composite dentaire, une composition comme indiqué dans les revendications 44,45 ou 46, dans laquelle X, et X₂ représentent tous deux -0- et a est 1.

48. Pour l'emploi dans l'application à la paroi d'une cavité dentaire avant obturation de la cavité avec une résine composite dentaire, une composition comme indiqué dans la revendication 47 dans laquelle R₂ est représenté par la formule (̵CH₂)̵_{f} dans laquelle f est un entier de 5 à 20.

49. Pour l'emploi dans l'application à la paroi d'une cavité dentaire avant obturation de la cavité avec une résine composite dentaire, une composition comme indiqué dans la revendication 47 dans laquelle R₂ est représenté par la formule 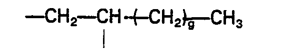 dans laquelle g est un entier de 2 à 20 ou par la formule

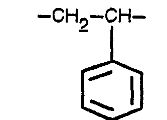

50. Pour l'emploi dans l'application à la paroi d'une cavité dentaire avant obturation de la cavité avec une résine composite dentaire, une composition comme revendiqué dans l'une quelconque des revendications 7 à 11, 13 à 16 et 18 à 40.

51. Pour l'emploi soit dans l'union d'une dent et d'un matériau de restauration dentaire, soit dans l'union des matériaux de restauration dentaire entre eux, une composition adhésive dentaire comprenant 100 parties en poids d'un monomère polymérisable comme revendiqué dans l'une quelconque des revendications 43 à 50, 20 à 500 parties en poids d'une charge et 0,01 à 20 parties en poids d'un agent de durcissement.

52. Pour l'emploi dans l'union d'une dent et d'un matériau de restauration dentaire ou de matériaux de restauration dentaire entre eux, une composition adhésive comme revendiqué dans la revendication 51 dans laquelle la charge est une charge minérale silanisée ou une charge composite organique-minérale.

53. Un monoester phosphorique de formule générale: 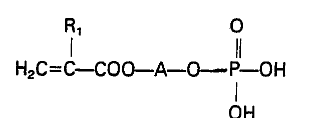 dans laquelle R, représente un hydrogène ou un groupe méthyle et A représente une des formules 3 à 6: 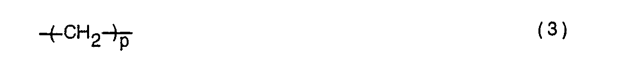 dans laquelle p est un entier de 5 à 12; 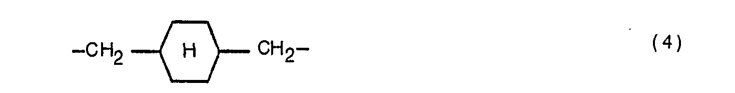 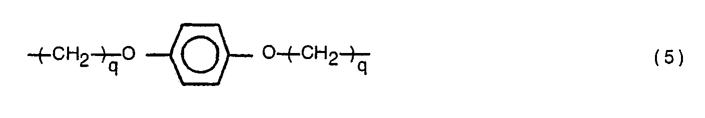 dans laquelle q est un entier de 2 à 4; 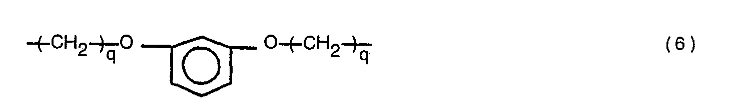 dans laquelle q est un entier de 2 à 4.

54. Pour l'emploi dans le revêtement d'une surface dentaire pour protéger les dents contre les caries dentaires, une composition d'obturation des trous et des fissures comprenant 100 parties en poids d'un monomère polymérisable comme revendiqué dens l'une quelconque des revendications 43 à 50 et 0,01 à 20 parties en poids d'un agent de durcissement.
